(19) Europäisches Patentamt
European Patent Office
Office européen des brevets

(11) EP 4 202 009 A1

(12) EUROPEAN PATENT APPLICATION
published in accordance with Art. 153(4) EPC

(43) Date of publication:
**28.06.2023 Bulletin 2023/26**

(21) Application number: **21858410.0**

(22) Date of filing: **20.08.2021**

(51) International Patent Classification (IPC):
***C09K 11/06*** *(2006.01)* ***C07F 7/02*** *(2006.01)*
***C07F 7/08*** *(2006.01)* ***G02B 5/20*** *(2006.01)*

(52) Cooperative Patent Classification (CPC):
**C07F 7/02; C07F 7/08; C09K 11/06; G02B 5/20**

(86) International application number:
**PCT/JP2021/030649**

(87) International publication number:
**WO 2022/039267 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2020 JP 2020140396**

(71) Applicant: **Kyushu University, National University Corporation**
**Nishi-ku**
**Fukuoka-shi**
**Fukuoka 819-0395 (JP)**

(72) Inventors:
- **YANAI, Nobuhiro**
  **Fukuoka-shi, Fukuoka 819-0395 (JP)**
- **KIMIZUKA, Nobuo**
  **Fukuoka-shi, Fukuoka 819-0395 (JP)**
- **HARADA, Naoyuki**
  **Fukuoka-shi, Fukuoka 819-0395 (JP)**
- **SASAKI, Yoichi**
  **Fukuoka-shi, Fukuoka 819-0395 (JP)**
- **WATANABE, Yuya**
  **Fukuoka-shi, Fukuoka 819-0395 (JP)**
- **MAI, Bac Lam**
  **Fukuoka-shi, Fukuoka 819-0395 (JP)**

(74) Representative: **Zimmermann & Partner Patentanwälte mbB**
**Postfach 330 920**
**80069 München (DE)**

# (54) PHOTON UPCONVERSION COMPOSITION, FILM, METHOD FOR CONVERTING VISIBLE LIGHT INTO ULTRAVIOLET LIGHT, AND COMPOUND

(57) A photon upconversion composition containing a naphthalene compound substituted with a substituent containing at least one selected from the group consisting of an alkynyl group, a substituted silyl group, a benzene ring, an heteroaromatic ring, a cyano group and a halogen atom can efficiently convert an excitation light into a UV light at a low excitation light intensity.

FIG. 1

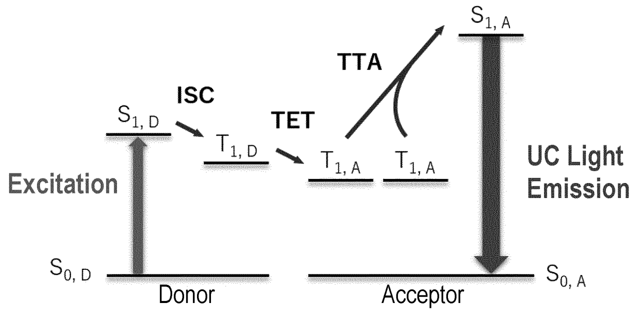


EP 4 202 009 A1

**Description**

Technical Field

The present invention relates to a photon upconversion composition useful as a creation source for UV light, a compound useful as an acceptor for the photon upconversion composition, as well as to a film and a method for converting visible light to UV light using the photon upconversion composition.

Background Art

Photon upconversion is a technique for converting low-energy light to high-energy light, and attention is paid thereto as an energy creation technology that can improve the efficiency of solar light-using devices including solar cells and photocatalysts.

As a material system in which such photon upconversion works, there is known a photon upconversion composition that contains, as combined, a donor functioning as a sensitizer and an acceptor functioning as a light emitter. In the composition, when the donor is excited to an excited singlet state by irradiation with excitation light, and is then converted into an excited triplet state through intersystem crossing, and the triplet energy transfers to the acceptor. In the acceptor thus having been in an excited triplet state through energy acceptance, triplets between two molecules meet to cause triplet-triplet annihilation, and one molecule among them transitions into an excited singlet state having a higher energy than the excited triplet state to bring about light emission (photon upconversion emission). Owing to the triplet-triplet annihilation-based photon upconversion mechanism, the composition can convert an irradiation light into a light in a higher energy level (light having a shorter wavelength).

A light conversion mode with a photon upconversion composition can include a mode of conversing a visible light into a visible light having a shorter wavelength and a mode of converting a visible light into a UV light. Among these, the mode of converting a visible light to a UV light can, for example, efficiently activate a photocatalyst by utilizing the resultant UV light, and can be therefore effectively used as a creation source for a UV light in various scenes of utilizing a UV light. However, most of photon upconversion compositions reported so far convert a visible light into a visible light having a shorter wavelength, and there are extremely few research examples of compositions converting a visible light into a UV light (for example, see NPLs 1 to 3). Moreover, the photon upconversion compositions of the study examples require an impossibly large excitation light intensity or could provide a low quantum yield, and in fact, these are far from practical use.

Citation List

Non-Patent Literature

NPL 1: Chem. Sci., 2017, 8, 5488-5496

NPL 2: J. Phys. Chem. Lett. 2019, 10, 5036-5040

NPL 3: ChemistryOpen 2020, 9, 14-17


Summary of Invention

Technical Problem

Given the situation and for the purpose of solving the problems of the prior art as above, the present inventors have promoted assiduous studies for providing a photon upconversion composition capable of efficiently converting an excitation light to a UV light at a lower excitation light intensity.

Solution to Problem

As a result of assiduous studies made for the purpose of solving the problems as above, the present inventors have found that, by using a naphthalene compound substituted with a specific substituent as an acceptor, a photon upconversion composition capable of efficiently converting an excitation light to a UV light at a lower excitation light intensity can be realized.

The present invention has been proposed based on such findings, and specifically has the following constitution.

[1] A photon upconversion composition containing a naphthalene compound substituted with a substituent containing at least one selected from the group consisting of an alkynyl group, a substituted silyl group, a benzene ring, an heteroaromatic ring, a cyano group and a halogen atom.
[2] The photon upconversion composition according to [1], in which the naphthalene compound has a substituted silyl group.
[3] The photon upconversion composition according to [1] or [2], in which the naphthalene compound has an alkynyl group.
[4] The photon upconversion composition according to any one of [1] to [3], in which the naphthalene compound has a substituted or unsubstituted phenyl group.
[5] The photon upconversion composition according to any one of [1] to [4], in which the naphthalene compound has two to four above-mentioned substituents.
[6] The photon upconversion composition according to any one of [1] to [5], in which the naphthalene compound is a 1,4-di-substituted naphthalene.
[7] The photon upconversion composition according to [1], in which the naphthalene compound has a structure represented by the following general formula (1).

General Formula (1)

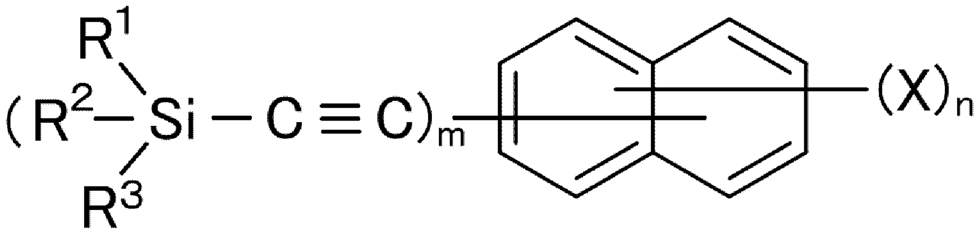

In the general formula (1), $R^1$, $R^2$ and $R^3$ each independently represent a substituted or unsubstituted alkyl group, X represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom, m represents an integer of any of 1 to 8, n represents an integer of any of 0 to 7, and m+n is an integer of any of 1 to 8.
[8] The photon upconversion composition according to [7], in which n in the general formula (1) is 1 or more.
[9] The photon upconversion composition according to [1], in which the naphthalene compound has a structure represented by the following general formula (2).

General Formula (2)

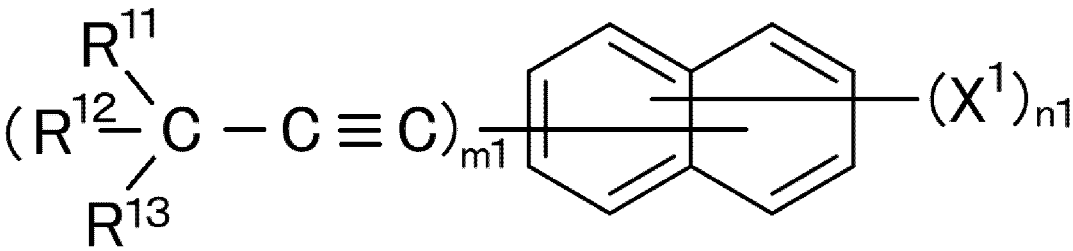

In the general formula (2), $R^{11}$, $R^{12}$ and $R^{13}$ each independently represent a hydrogen atom or a substituted or unsubstituted alkyl group, at least one of $R^{11}$, $R^{12}$ and $R^{13}$ is a substituted or unsubstituted alkyl group, $X^1$ represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom, m1 represents an integer of any of 1 to 8, n1 represents an integer of any of 0 to 7, and m1+n1 is an integer of any of 1 to 8.
[10] The photon upconversion composition according to [9], in which n1 in the general formula (2) is 1 or more.
[11] A film containing the photon upconversion composition of any one of [1] to [10].
[12] A myopia-suppressing transparent product containing the photon upconversion composition of any one of [1] to [10] or the film of [11].

[13] A method for converting a visible light into a UV light by irradiating the photon upconversion composition of any one of [1] to [10] or the film of [11] with a visible light.

[14] The method according to [13], in which emission of the UV light is recognized in a range of 360 to 400 nm.

[15] A compound represented by the following general formula (1').

General Formula (1')

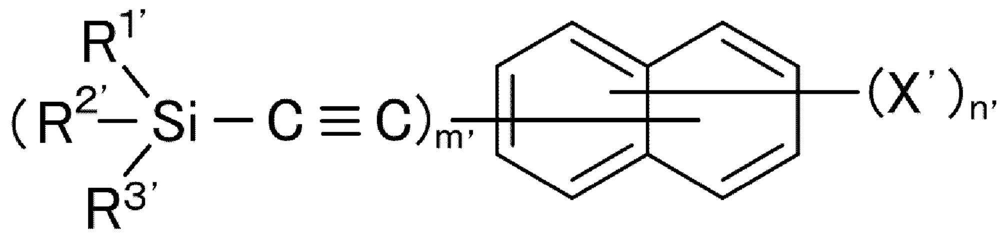

In the general formula (1'), $R^{1'}$, $R^{2'}$ and $R^{3'}$ each independently represent a substituted or unsubstituted alkyl group, the total carbon number of $R^{1'}$, $R^{2'}$ and $R^{3'}$ is 6 or more, X' represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom, m' represents an integer of any of 1 to 8, n' represents an integer of any of 0 to 7, and m'+n' is an integer of any of 1 to 8.

Advantageous Effects of Invention

According to the present invention, there can be realized a photo upconversion composition capable of efficiently converting an excitation light to a UV light at a lower excitation light intensity. Using the photo upconversion composition as a subject targeted for photoirradiation in a method of irradiating the subject with a visible light to convert the visible light into a UV light, a UV light can be efficiently created even with a weak irradiation light such as sunlight or room light.

Brief Description of Drawings

[Fig. 1] This is a view for explaining a UC light emission mechanism of a photon upconversion composition of the present invention.

[Fig. 2] This shows UC light emission spectra of a tetrahydrofuran solution of Compound A1 and Compound D1 (Composition 1) with a 445-nm excitation light.

[Fig. 3] This is a double logarithmic plot showing excitation light intensity dependence of UC light emission intensity via a tetrahydrofuran solution of Compound A1 and Compound D1 (Composition 1 and Composition 2).

[Fig. 4] This shows UC light emission spectra of a tetrahydrofuran solution of both Compound A1 and Compound D1 (Composition 1), and a tetrahydrofuran solution of Compound A1 alone (solution of Compound A1) with a simulated solar light.

[Fig. 5] This shows UC light emission spectra of a tetrahydrofuran solution of both Compound A1 and Compound D1 (Composition 1), and a tetrahydrofuran solution of Compound A1 alone (solution of Compound A1) with an LED light.

[Fig. 6] This is a UC light emission spectrum of Film 1 containing Compound A1, Compound D1 and polystyrene.

[Fig. 7] This is a UC light emission spectrum of Film 2 containing Compound A1, Compound D1 and poly(methyl methacrylate).

[Fig. 8] This is a UC light emission spectrum of Film 3 containing Compound A1, Compound D1 and poly(N-isopropylacrylamide).

[Fig. 9] This is a UC light emission spectrum of Film 4 containing Compound A1, Compound D1 and poly(butyl acrylate).

[Fig. 10] This shows UC light emission spectra of Film 5 containing Compound A1 and Compound D1.

[Fig. 11] This is a UC light emission spectrum of Film 6 containing Compound A1, Compound D1, polyvinyl alcohol and Pluronic F127.

[Fig. 12] This is a UC light emission spectrum of Film 7 containing Compound A1, Compound D1, polyvinyl alcohol and TX-100.

[Fig. 13] This is a UC light emission spectrum of a microporous film impregnated with a solution of Compound A1

and Compound D1 (Film 8).

[Fig. 14] This shows UC light emission spectra of a microporous film impregnated with a liquid material of Compound D1 having dissolved in a melt of Compound A1 (Film 9).

[Fig. 15] This shows UC light emission spectra of a tetrahydrofuran solution of Compound A3 and Compound D1 (Composition 4).

[Fig. 16] This is a graph showing time dependence of UC light emission of Composition 4 after stopping irradiation with an excitation light.

[Fig. 17] This shows UC light emission spectra of a tetrahydrofuran solution of Compound A4 and Compound D1 (Composition 5).

[Fig. 18] This shows UC light emission spectra of a tetrahydrofuran solution of Compound A5 and Compound D1 (Composition 6).

[Fig. 19] This shows UC light emission spectra of a tetrahydrofuran solution of Compound A6 and Compound D1 (Composition 7).

Description of Embodiments

Hereinunder the present invention is described in detail. The constitutional elements may be described below with reference to representative embodiments and specific examples of the invention, but the invention is not limited to the embodiments and the examples. In the description herein, a numerical range expressed as "to" means a range that includes the numerical values described before and after "to" as the lower limit and the upper limit. In the description, the hydrogen atom that is present in the molecule of the compound used in the invention is not particularly limited in isotope species, and for example, all the hydrogen atoms in the molecule may be $^{1}H$, and all or a part of them may be $^{2}H$ (deuterium D).

<Photon Upconversion Composition>

The photon upconversion composition of the present invention contains a naphthalene compound substituted with a substituent containing at least one selected from the group consisting of an alkynyl group, a substituted silyl group, a benzene ring, a heteroaromatic ring, a cyano group and a halogen atom.

The "photon upconversion composition" in the present invention means a composition exhibiting a performance of converting a light (irradiation light) applied to the composition into a light having a shorter wavelength. The light of a conversion source to be converted into "a light having a shorter wavelength" is a light falling in a longer wavelength region than a UV region and capable of exciting at least any component of the photon upconversion composition, and is preferably a visible light. Here, "a light capable of exciting a component" can be selected from a light of which the wavelength region of an emission peak overlaps with the wavelength region where the component exhibits photoabsorption, and in the following description, the light is referred to as "an excitation light" for the component. The "light having a shorter wavelength" to which the light of a conversion source is to be converted is preferably a UV light. Namely, a preferred embodiment of the "photon upconversion composition" of the present invention is a composition that exhibits a performance of converting a visible light into a UV light. Here, the "visible light" in the present description means a light of which the wavelength falls within a range of more than 400 nm to 800 nm, and the "UV light" means a light of which the wavelength falls within a range of 200 nm or more and 400 nm or less. The light of a conversion source, and the UV light that contains the converted light can be a single light or can also be a composite light containing plural lights differing in the emission maximum wavelength. The light of a conversion source can contain a visible light and a light except a visible light, and the converted light can contain a UV light and a light except a UV light.

In the following description, the phenomenon that the photon upconversion composition emits an irradiation light to convert the light into a light having a shorter wavelength can be referred to as "photon upconversion emission" or "UC light emission", and the light emitted by photon upconversion emission (a light having a shorter wavelength than the irradiation light) can be referred to as "a UC light", and the "photon upconversion composition of the present invention" can be referred to merely as the "composition of the present invention".

UC light emission of the photon upconversion composition of the present invention is derived from the emission of the naphthalene compound contained in the composition. The naphthalene compound used in the present invention has a high lowest exited singlet energy level and therefore can emit a UV light along with deactivation from the excited singlet state. Also the naphthalene compound used in the present invention is characterized in that the compound is substituted with a substituent containing at least one selected from the group consisting of an alkynyl group, a substituted silyl group, a benzene ring, a heteroaromatic ring, a cyano group and a halogen atom. As substituted with the substituent of the type, the naphthalene compound has a high formation probability f for the excited singlet state by triplet-triplet annihilation and a high fluorescence quantum yield $\Phi_{FA}$, and therefore can efficiently convert a low energy to a high energy to exhibit photon upconversion emission. The emission obtained from the composition is a photon upconversion

emission by triplet-triplet annihilation, which can be confirmed by the fact that the lifetime of the photon upconversion emission is delayed fluorescent on a level of a several seconds or so and the fact that, in the double logarithmic plot of excitation light intensity dependence of the photon upconversion emission intensity, the inclination converts from 2 to 1.

In the following description, "an alkynyl group, a substituted silyl group, a benzene ring, a heteroaromatic ring, a cyano group and a halogen atom" can be referred to as "a specific group", and "a substituent containing at least one selected from the group consisting of an alkynyl group, a substituted silyl group, a benzene ring, a heteroaromatic ring, a cyano group and a halogen atom" can be referred to as "a specific group-containing substituent" .

In the following, the structure of the naphthalene compound used in the present invention is described in detail.

[Naphthalene Compound]

The naphthalene compound in the present invention is a compound containing a naphthalene skeleton. The naphthalene skeleton referred to herein means a naphthalene ring not condensed with any other ring. Consequently, a tricyclic or more polycyclic skeleton of a naphthalene ring condensed with some other ring is not included in the naphthalene skeleton referred to herein.

First, the specific group that the substituent of the naphthalene compound includes is described.

The alkynyl group of the substituent can be any of linear, branched or cyclic ones. Preferably, the carbon number of the alkynyl group is 2 to 20, more preferably 2 to 10, even more preferably 2 to 6. Examples of the alkynyl group include an ethynyl group, a propynyl group and a butynyl group.

The substituted silyl group can be any of a mono-substituted silyl group, a di-substituted silyl group or a tri-substituted silyl group, but is preferably a tri-substituted silyl group. In the di-substituted silyl group and the tri-substituted silyl group, the plural substituents can be the same as or different from each other. The substituent of the substituted silyl group includes an alkyl group, and an aromatic ring group. The alkyl group can be any of linear, branched or cyclic ones, but is preferably linear or branched. The carbon number of the alkyl group can be 1 or more, or can be 2 or more, but is preferably 2 or more, more preferably 3 or more. In the case where the substituted silyl group is a tri-alkylsilyl group, the total carbon number of the three alkyl groups is preferably 6 or more. The upper limit of the carbon number is not specifically limited, but is preferably 20 or less. Regarding the description, the preferred range and the specific examples of the aromatic ring group, reference can be made to the description relating to the aromatic ring group as the second substituent mentioned below. Specific examples of the substituted silyl group include a trimethylsilyl group, a triethylsilyl group, a tri-n-propylsilyl group, a triisopropylsilyl group, and a triphenylsilyl group.

The benzene ring can be a single ring contained in the substituent, or can constitute at least a part of a condensed polycyclic structure contained in the substituent. Namely, at least one side of the benzene ring can be condensed with a cyclic structure. The cyclic structure to be condensed with the benzene ring can be any of an aromatic ring (aromatic hydrocarbon ring), an aromatic heteroring, an alicyclic hydrocarbon ring, or an alicyclic heteroring. Preferably, the benzene ring constitutes an aromatic ring group (aryl group) as a single ring, or forms a condensed polycyclic structure with any other aromatic ring to constitute an aromatic ring group (aryl group). The carbon number of the aromatic ring in the aromatic ring group is preferably 6 to 22, more preferably 6 to 18, even more preferably 6 to 14, further more preferably 6 to 10. Specific examples of the aromatic ring group include a phenyl group, a naphthalenyl group and a biphenyl group.

The heteroaromatic ring can be a single ring, or can also be a condensed ring of one or more heterorings condensed with one or more aromatic rings or heterorings. The heteroring in the condensed ring can be a heteroaromatic ring or an alicyclic heteroring, so far as the condensed ring has aromaticity as a whole. The carbon number of the heteroaromatic ring is preferably 3 to 40, more preferably 5 to 22, even more preferably 5 to 18, further more preferably 5 to 14, particularly preferably 5 to 10. The heteroatom to constitute the heteroring includes a nitrogen atom, an oxygen atom, and a sulfur atom. Specific examples of the heteroaromatic ring include a pyridine ring, a pyridazine ring, a pyrimidine ring, a triazole ring, and a benzotriazole ring.

Specific examples of the halogen atom include a fluorine atom, a chlorine atom, a bromine atom, and an iodine atom.

The alkynyl group in the substituent, the substituent in the substituted silyl group, the benzene ring and the heteroaromatic ring can be substituted with a substituent (hereinafter referred to as the "second substituent"). The second substituent includes an alkenyl group, an alkyl group, an aromatic ring group, a heteroaromatic ring group, an alkoxy group, a carboxy group and a pinacolatoboryl group, in addition to an alkynyl group, a substituted silyl group, a cyano group and a halogen atom. The second substituent can be further substituted with a third substituent. Regarding the description, the preferred range and the specific examples of the alkynyl group, the substituted silyl group and the halogen atom, reference can be made to the description relating to the alkynyl group, the substituted silyl group and the halogen atom of the substituent mentioned above.

The alkenyl group as the second substituent can be any of linear, branched or cyclic ones. Preferably, the carbon number of the group is 2 to 20, more preferably 2 to 10, even more preferably 2 to 6. Examples of the group include an ethenyl group, a propenyl group, and a butenyl group.

The alkyl group can be any of linear, branched or cyclic ones. Preferably, the carbon number of the group is 1 to 20, more preferably 1 to 10, even more preferably 1 to 6. Examples of the group include a methyl group, an ethyl group, an n-propyl group, and an isopropyl group.

The alkyl group bonding to the oxy group of the alkoxy group can be any of linear, branched or cyclic ones. Preferably, the carbon number of the group is 1 to 20, more preferably 1 to 10, even more preferably 1 to 6. Specific examples of the alkoxy group include a methoxy group, an ethoxy group, an n-propoxy group and an isopropoxy group.

Regarding the description, the preferred range and the specific examples of the aromatic ring group, reference can be made to the description relating to the aromatic ring group constituted by a benzene ring in the above-mentioned substituent, and regarding the description, the preferred range and the specific examples of the heteroaromatic ring to constitute the heteroaromatic ring group, reference can be made to the description relating to the heteroaromatic ring in the above-mentioned substituent.

The naphthalene compound for use in the present invention is substituted with a substituent (a specific group-containing substituent) containing at least one selected from the group consisting of an alkynyl group, a substituted silyl group, a benzene ring, a heteroaromatic ring, a cyano group and a halogen atom as mentioned above (that is, a specific group). The specific group-containing substituent can be a substituent having a structure in which the specific group bond to a naphthalene ring via a single bond, or can be a substituent having a structure in which the specific group bond to a naphthalene ring via a linking group. Also, the specific group-containing substituent can contain only one of the group of the specific groups, or can contain two or more thereof. Embodiments of a substituent that contains two or more specific groups include a substituent having a structure where a specific group bonds to a different specific group via a single bond (structure with a specific group is substituted with a different specific group), and a substituent having a structure where a specific group bonds to a different specific group via a linking group. The specific group-containing substituent can be formed of specific groups alone, or can contain any other group than specific groups. Embodiments of a substituent containing a specific group and any other group than a specific group include a substituent having a structure where a specific group bonds to a naphthalene ring via a linking group and where the linking group is a group except a specific group, and a substituent having a structure where a specific group is substituted with a substituent and where the substituent is any other group than the specific group.

The other groups than the specific group include an alkenyl group, an alkyl group, an alkoxy group, and a carboxy group. Regarding the description, the preferred range and the specific examples of the alkenyl group, the alkyl group and the alkoxy group, reference can be made to the description relating to the alkenyl group, the alkyl group and the alkoxy group as the second substituent.

In the naphthalene compound for use in the present invention, the substitution number of the specific group-containing substituents in the naphthalene ring can be one, or can be two or more. The substitution number of the specific group-containing substituents in the naphthalene ring is preferably 2 to 4. When the substitution number of the specific group-containing substituents is 2 or more, the plural specific group-containing substituents can be the same as or different from each other. The substitution position of the specific group-containing substituent in the naphthalene ring is, though not specifically limited, preferably at least 1-position and 4-position, more preferably only 1-position and 4-position. Namely, the naphthalene compound for use in the present invention is more preferably a 1,4-disubstituted naphthalene. Also, the substitution position of the specific group-containing substituent in the naphthalen ring can be 1-position and 5-position.

When the substitution number of the specific group-containing substituents in the naphthalene ring is 7 or less, the substitutable position (hydrogen atom of methylene group) in the naphthalene ring not substituted with a specific group-containing substituent can be substituted with a substituent except specific group-containing substituents. Preferred examples of the substituent except specific group-containing substituents include an alkenyl group, an alkyl group, an alkoxy group, and a carboxy group. Regarding the description, the preferred range and the specific examples of the alkenyl group, the alkyl group and the alkoxy group, reference can be made to the description relating to the alkenyl group, the alkyl group and the alkoxy group as the second substituent.

The naphthalene compound used in the present invention preferably has at least one of a substituted silyl group, an alkynyl group and a substituted or unsubstituted phenyl group. Among these, in particular, when an alkynyl group is introduced thereinto, the naphthalene compound can have a rigid structure. With that, the compound can suppress nonradiative deactivation of a triplet exciton and can therefore be advantageous for improving the formation probability f for a singlet excited state by triplet-triplet annihilation. The alkynyl group can bond to the naphthalene ring via a single bond, but can bond to the naphthalene ring via a linking group such as a phenylene group. Namely, the alkynyl group can constitute, for example, an alkynylphenyl group.

Preferably, the naphthalene compound used in the present invention has a structure represented by the following general formula (1).

General Formula (1)

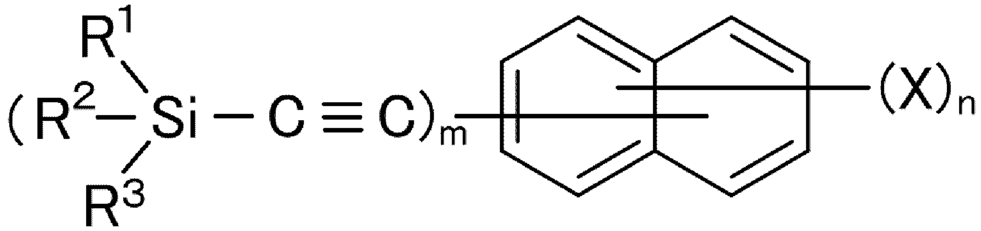

In the general formula (1), $R^1$, $R^2$ and $R^3$ each independently represent a substituted or unsubstituted alkyl group. $R^1$, $R^2$ and $R^3$ can be the same as or different from each other. The alkyl group of $R^1$, $R^2$ and $R^3$ can be any of linear, branched or cyclic ones, but is preferably linear or branched. The carbon number of the alkyl group can be 1 or more, or can be 2 or more, but is preferably 2 or more, more preferably 3 or more. Also preferably, the total carbon number of $R^1$, $R^2$ and $R^3$ is 6 or more. The upper limit of the carbon number is not specifically limited, but the carbon number of each alkyl group is preferably 20 or less. Specific examples of the alkyl group include a methyl group, an ethyl group, an n-propyl group, and an isopropyl group. When the alkyl group is a substituted alkyl group, the substituent thereof includes an alkynyl group, an alkenyl group, an aromatic ring group, a heteroaromatic ring group, an alkoxy group, a carboxy group, a cyano group and a halogen atom. Regarding the description, the preferred range and the specific examples of these, reference can be made to the description relating to the corresponding group of those that X represents.

X represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom. The group that X represents can be composed of one group alone selected from the group, or can be a group of a combination of two or more groups selected from the group. The group of a combination of two or more groups (plural groups) selected from the group is, for example, a group having a structure where any one group of the plural groups is substituted with the remaining at least one group. The number of the groups substituting for any one group of the plural groups can be 1, or can be 2 or more. Here, when X includes an alkynyl group or an alkenyl group, preferably, the alkynyl group or the alkenyl group does not bond directly to an alkoxy group, a cyano group or a halogen atom. Also preferably, X represents a group of a combination of one or more selected from the group consisting of an alkynyl group, an alkyl group, an aromatic ring group and a heteroaromatic ring group. More preferably, X is a group of a combination of one or more selected from the group consisting of an alkynyl group, an alkyl group and an aromatic ring group. X is preferably a substituted or unsubstituted alkylalkynyl group, a substituted or unsubstituted arylalkynyl group, a substituted or unsubstituted alkyl group, or a substituted or unsubstituted aryl group, and the substituent referred to herein is preferably an alkylalkynyl group, an arylalkynyl group, an alky group, an aryl group, a heteroaryl group, a cyano group or a halogen atom, more preferably an alkylalkynyl group, an arylalkynyl group, an alkyl group or an aryl group. Two or more X's do not bond to each other to form a cyclic structure.

Regarding the description, the preferred range and the specific examples of the alkynyl group, reference can be made to the description relating to the alkynyl group in the above-mentioned substituent, and regarding the description, the preferred range and the specific examples of the alkenyl group, the alkyl group, the aromatic ring group, the heteroaromatic ring group, and the alkoxy group, reference can be made to the description of the alkenyl group, the alkyl group, the aromatic ring group, the heteroaromatic ring group and the alkoxy group as the above-mentioned second substituent.

m represents a substitution number on the naphthalene ring of the tri-substituted silylethynyl groups parenthesized by m, and is an integer of any of 1 to 8. Preferably, m is an integer of any of 1 to 6, more preferably an integer of any of 1 to 4, and can be selected from, for example, a range of 1 to 2, or can be selected from a range of 2 to 4. When m is an integer of 2 or more, the tri-substituted silylethynyl groups parenthesized by m can be the same as or different from each other. The substitution position on the naphthalene ring of the tri-substituted silylethynyl groups parenthesized by m are preferably at least at 1-position and 4-position, more preferably at 1-position and 4-position alone. The substitution position on the naphthalene ring of the tri-substituted silylethynyl groups parenthesized by m can be at 1-position and 5-position.

n represents a substitution number of X on the naphthalene ring, and is an integer of any of 0 to 7. But m+n is an integer of any of 1 to 8. When n is an integer of 2 or more, plural X's can be the same as or different from each other. Preferably, n is an integer of any of 0 to 6, more preferably an integer of any of 0 to 4, and can be selected, for example, from a range of 0 to 2, or can be 0 or 1. Also preferably, n is 0. Also preferably, n is 1 or more. The compound of the general formula (1) where n is 1 or more, namely, the compound in which the naphthalene ring is substituted with the substituent X can keep a suitable clearance between the compound molecules even in a high concentration condition owing to the

steric hindrance of the substituent and can attain a high fluorescence quantum yield. The compound of the type is advantageous in using it, for example, by mixing with an epoxy resin or a resin of polystyrene or polymethyl methacrylate at a high concentration. In addition, from this viewpoint, X is preferably a bulky substituent such as a branched alkyl group. Preferred examples of the branched alkyl group include a branched alkyl group having 3 to 6 carbon atoms, such as an isopropyl group, an isobutyl group, a sec-butyl group, a tert-butyl group, an isopentyl group, a neopentyl group, a tert-pentyl group, and an isohexyl group, and more preferred is a tert-butyl group.

Also preferably, the naphthalene compound used in the present invention has a structure represented by the following general formula (2).

General Formula (2)

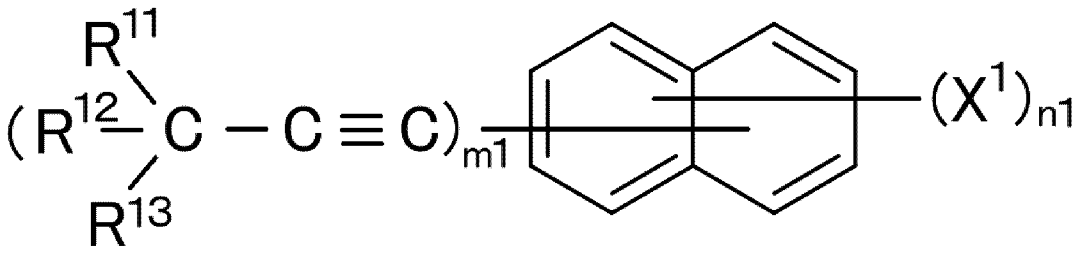

In the general formula (2), $R^{11}$, $R^{12}$ and $R^{13}$ each independently represent a hydrogen atom or a substituted or unsubstituted alkyl group, at least one of $R^{11}$, $R^{12}$ and $R^{13}$ is a substituted or unsubstituted alkyl group. Of $R^{11}$, $R^{12}$ and $R^{13}$, one or two can be a substituted or unsubstituted alkyl group, or all $R^{11}$, $R^{12}$ and $R^{13}$ can be a substituted or unsubstituted alkyl group. When two or more of $R^{11}$, $R^{12}$ and $R^{13}$ each are a substituted or unsubstituted alkyl group, the plural substituted or unsubstituted alkyl groups can be the same as or different from each other. Regarding the description, the preferred range and the specific examples of the substituted or unsubstituted alkyl group of $R^{11}$, $R^{12}$ and $R^{13}$, reference can be made to the description, the preferred range and the specific examples of the substituted or unsubstituted alkyl group that $R^1$, $R^2$ and $R^3$ in the general formula (1) represent.

In the general formula (2), $X^1$ represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom, m 1 represents an integer of any of 1 to 8, n1 represents an integer of any of 0 to 7, and m1+n1 is an integer of any of 1 to 8. Regarding the description of $X^1$, reference can be made to the description of X in the general formula (1); and regarding the description of m1 and n1, reference can be made to the description of m and n, respectively, in the general formula (1).

The compound represented by the general formula (2) is characterized in that it is excellent in long-term stability and has a high effect sustainability, and is, from this viewpoint, therefore more excellent than the compound represented by the general formula (1).

Specific examples of the naphthalene compound are shown below, but the naphthalene compound usable in the present invention should not be limitatively interpreted by these specific examples.

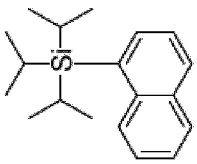

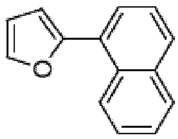

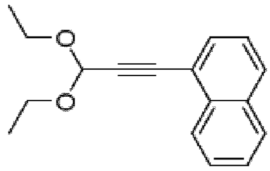

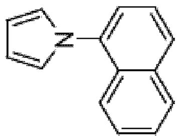

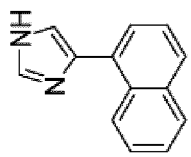

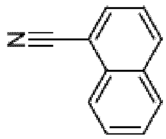

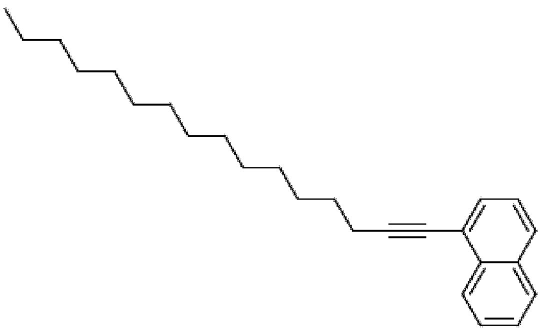

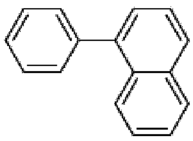

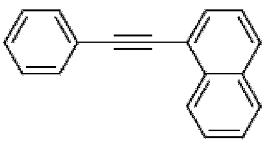

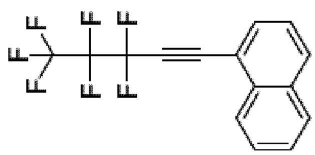

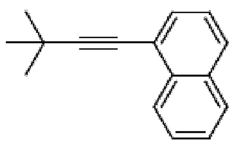

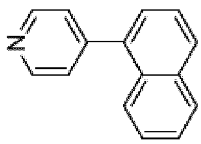

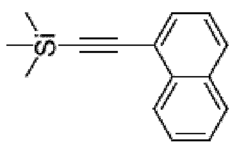

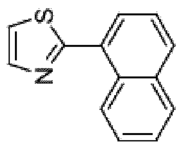

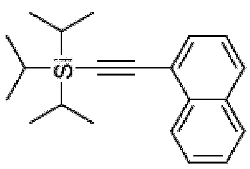

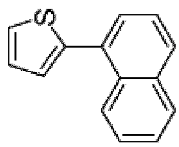

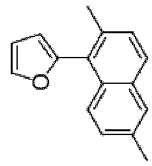
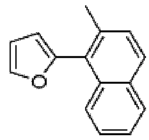
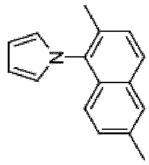
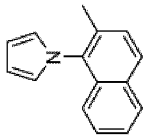
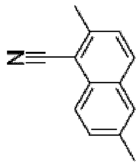
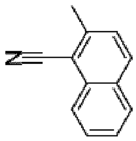
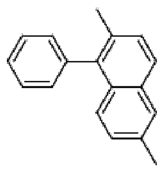
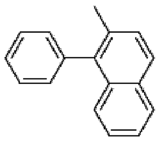
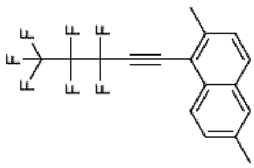
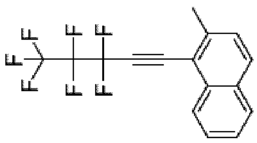
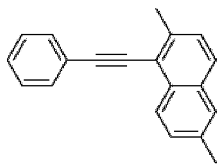
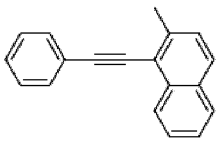
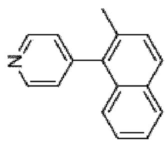
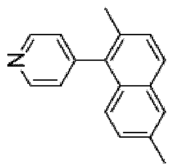
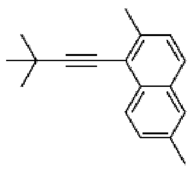
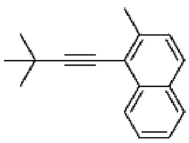
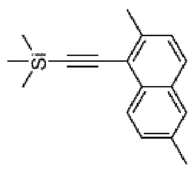
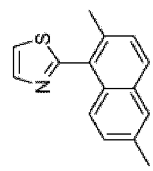
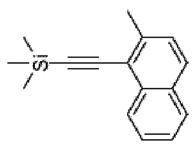
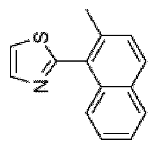
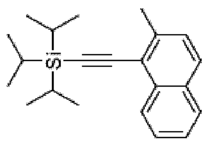
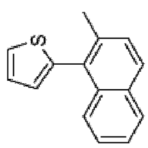
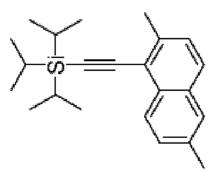
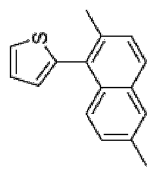

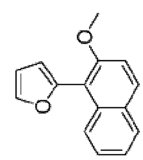
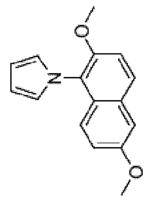
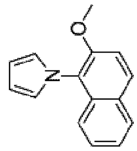
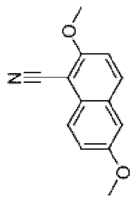
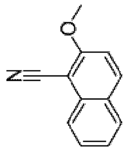
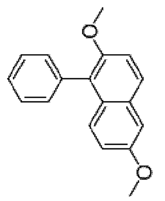
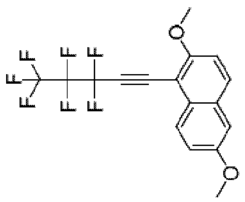
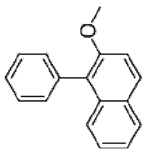
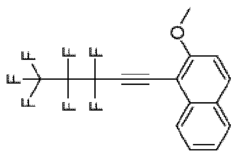
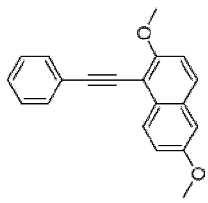
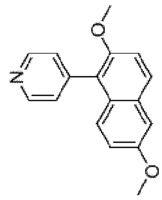
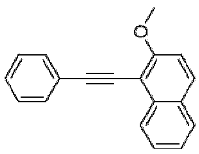
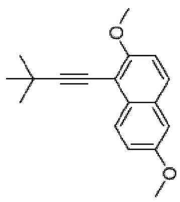
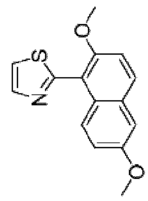
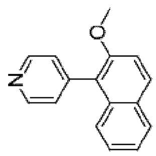
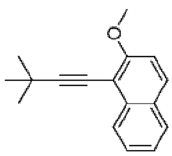
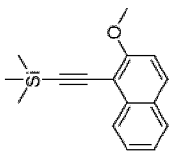
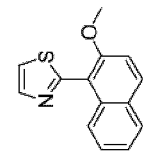
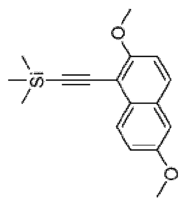
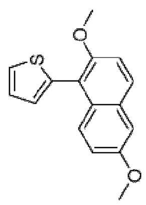

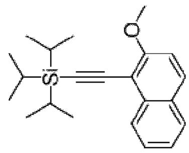
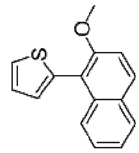
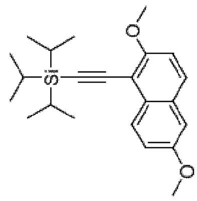
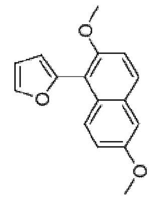
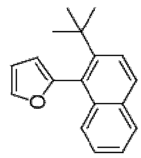
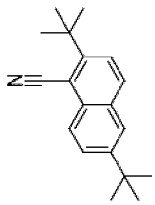
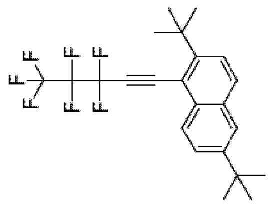
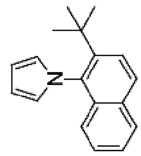
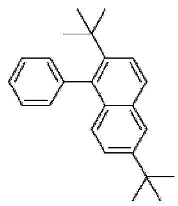
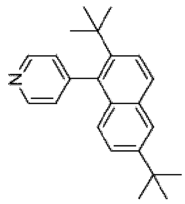
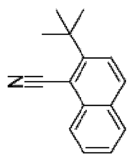
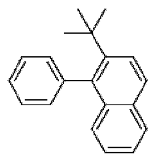
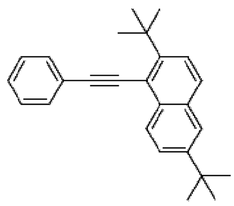
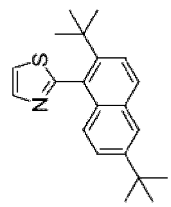
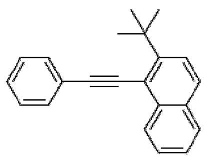
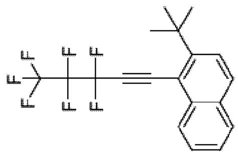
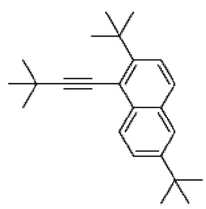
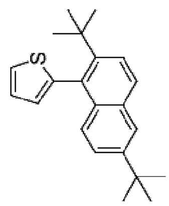
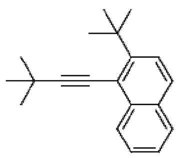
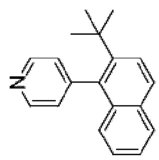
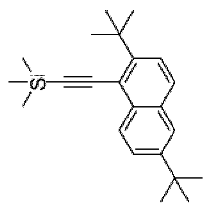
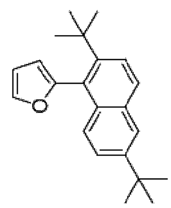
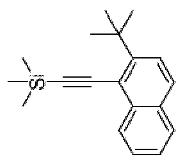
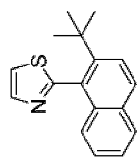

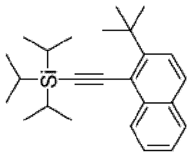
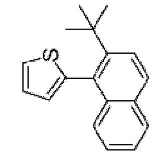
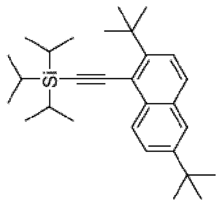
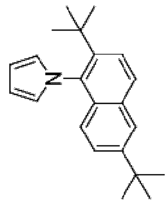

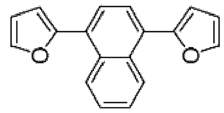

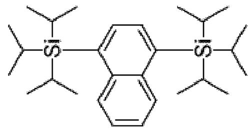

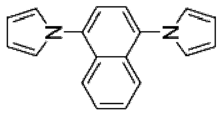

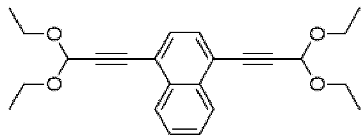

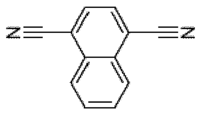

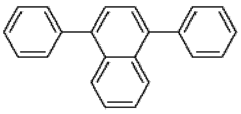

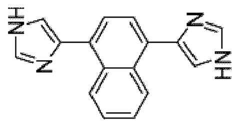

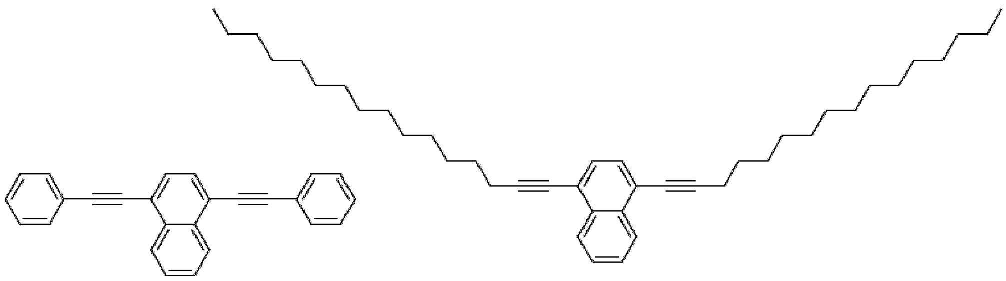

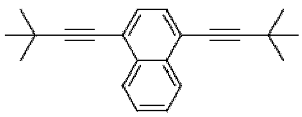

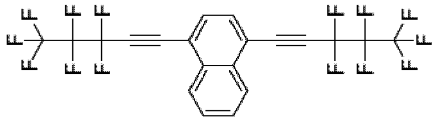

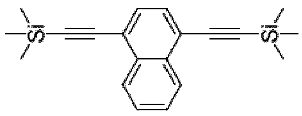

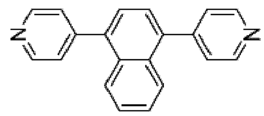

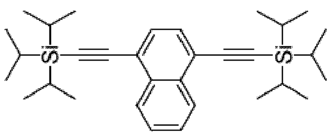

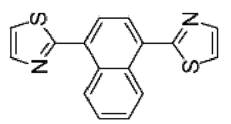

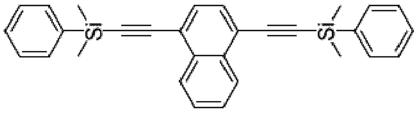

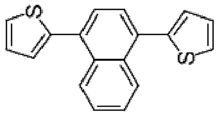

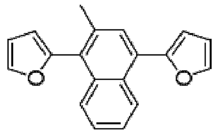

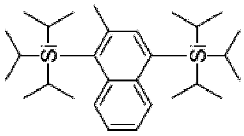

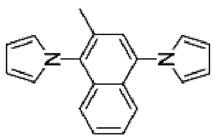

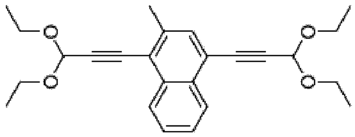

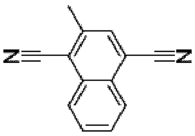

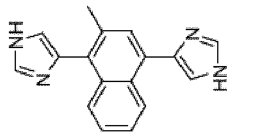

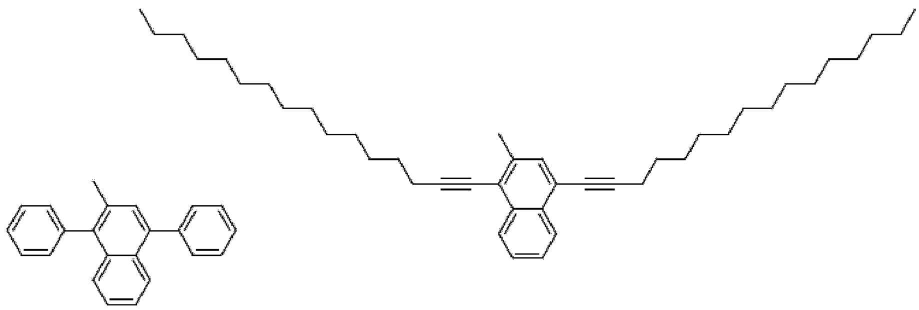

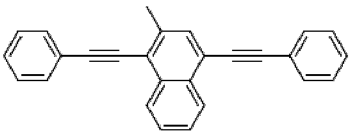

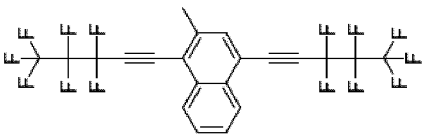

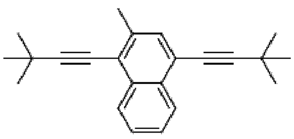

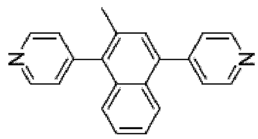

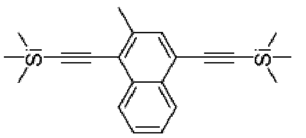

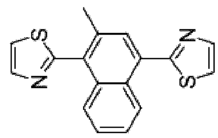

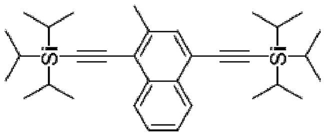

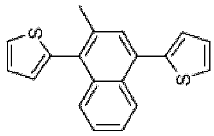

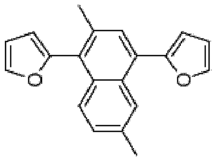

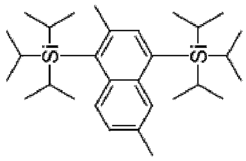

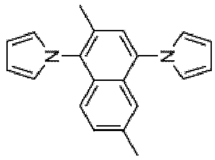

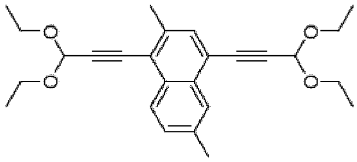

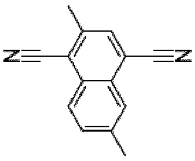

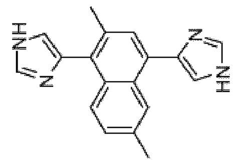

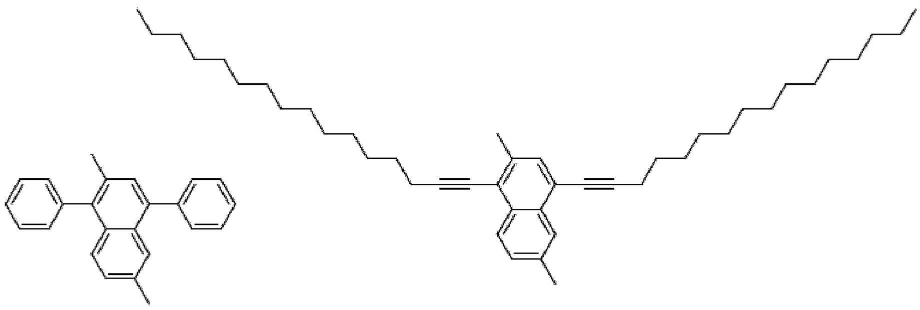

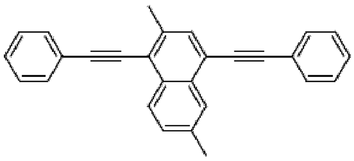

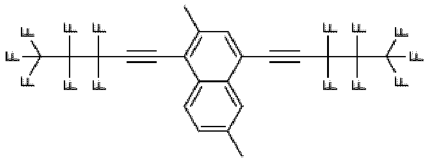

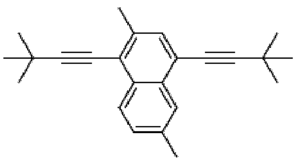

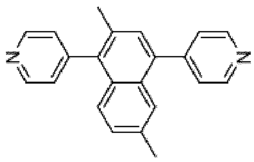

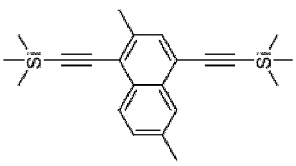

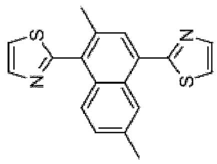

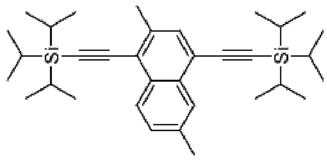
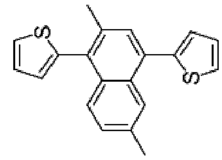

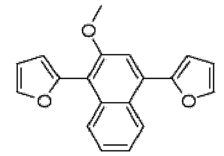
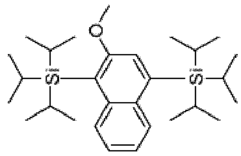

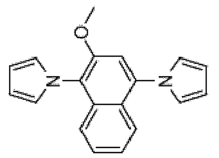
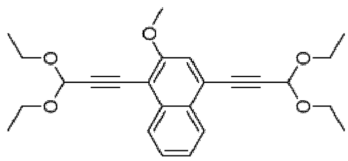

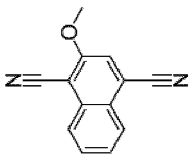
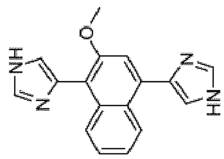

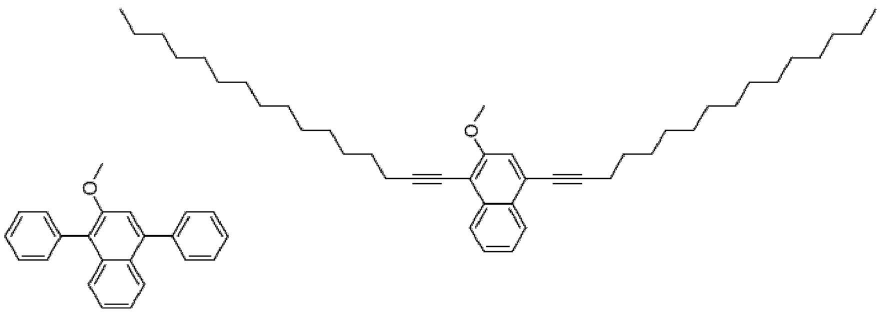

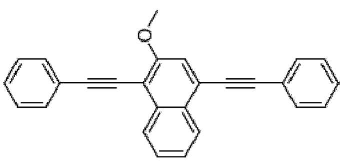
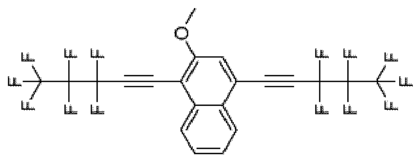

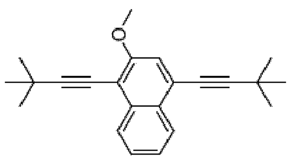
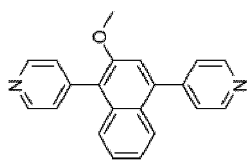

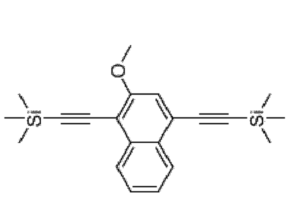

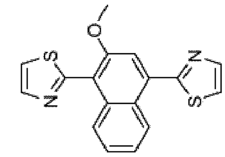

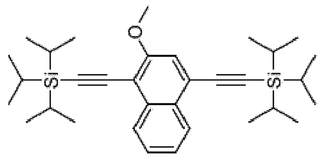

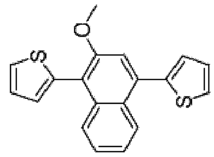

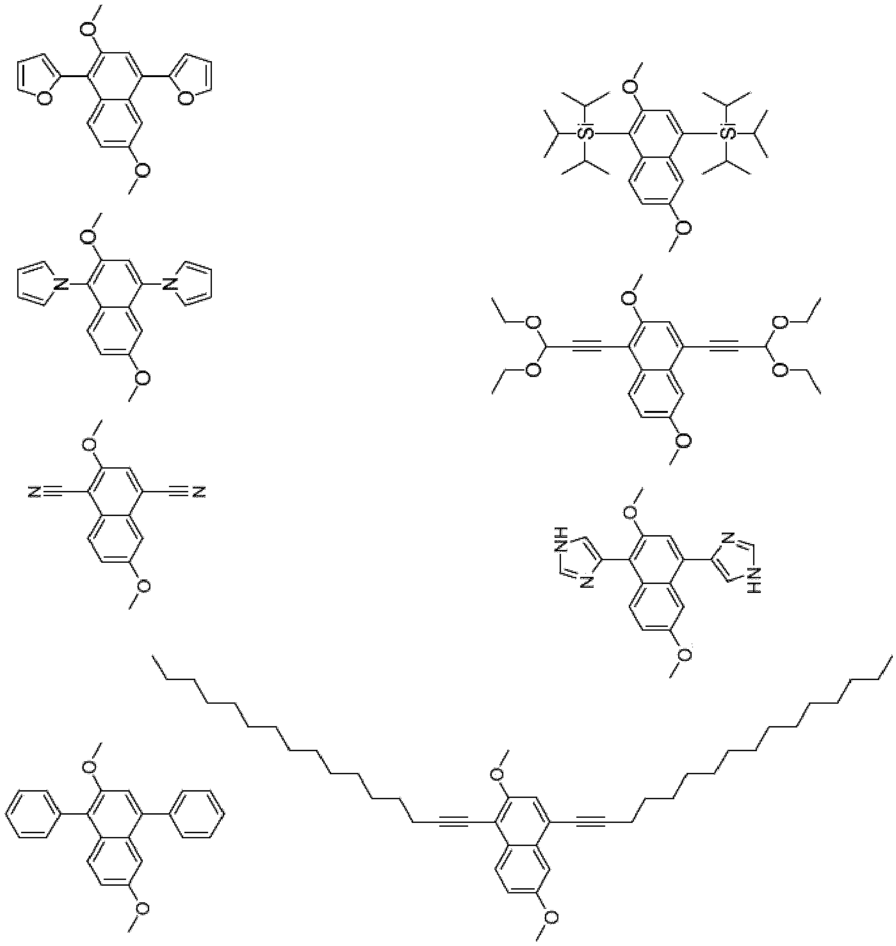

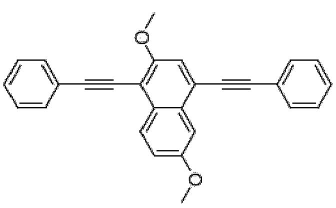

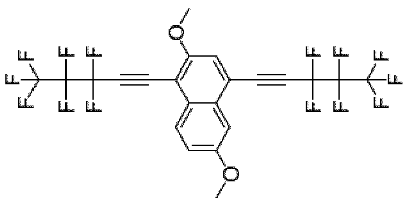

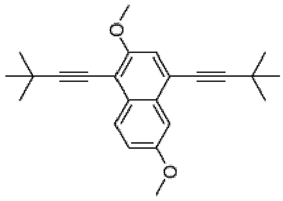 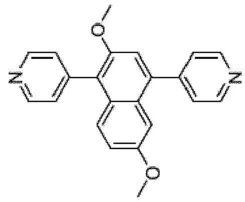

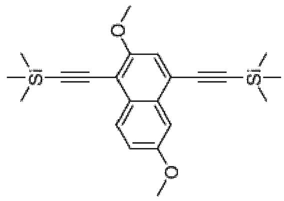 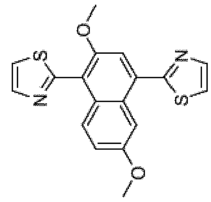

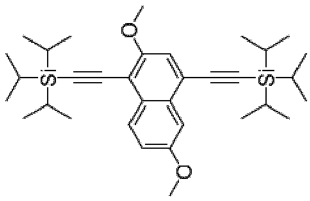 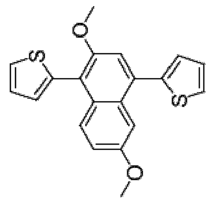

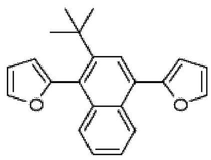 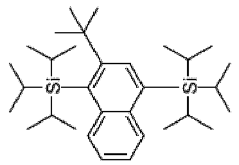

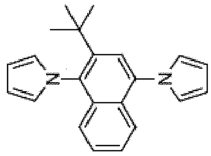

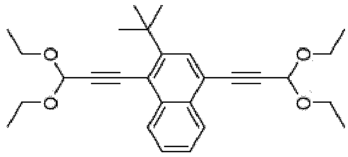

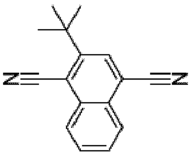

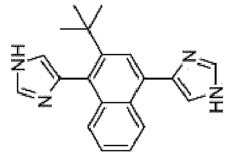

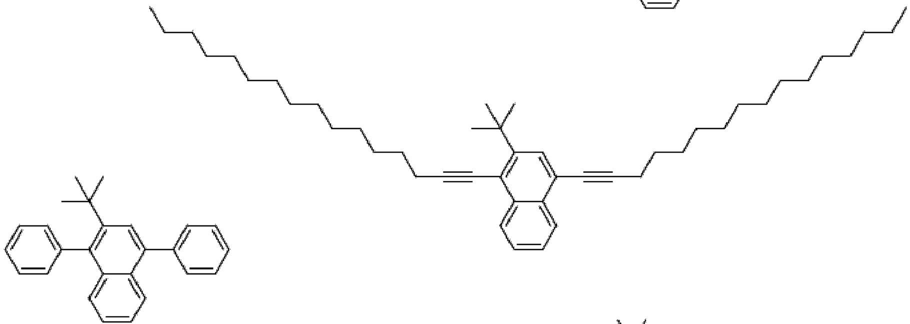

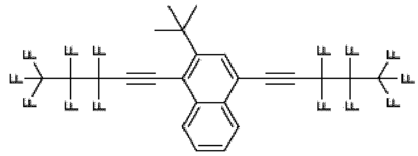

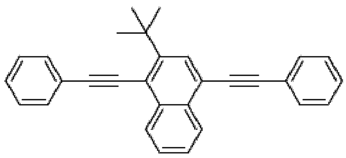

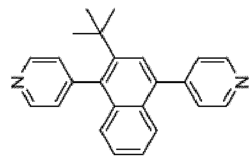

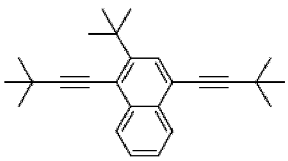

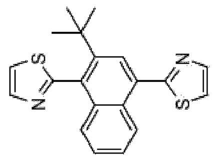

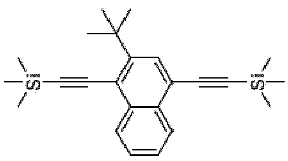

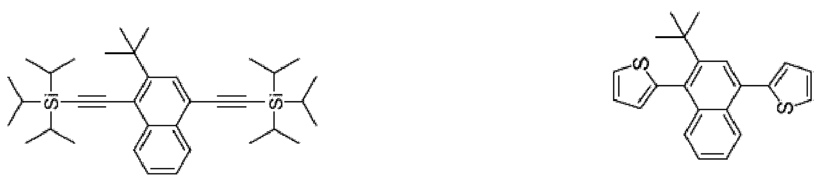

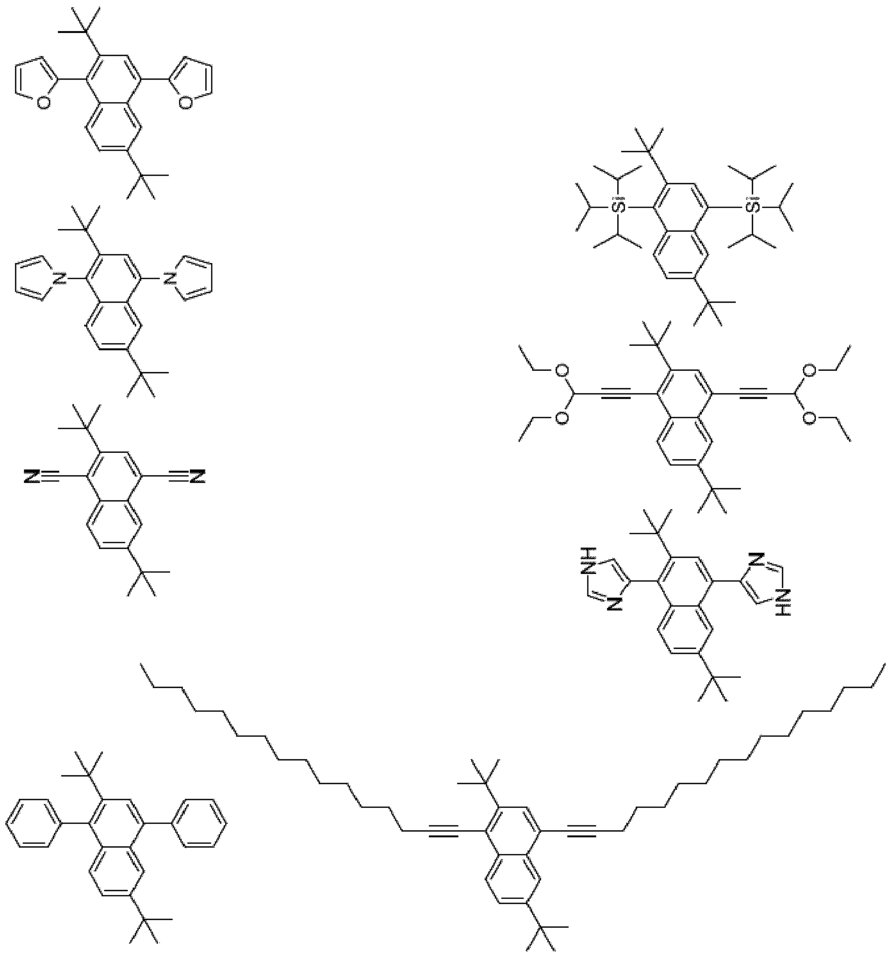

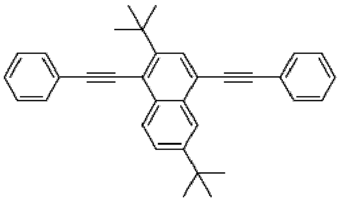

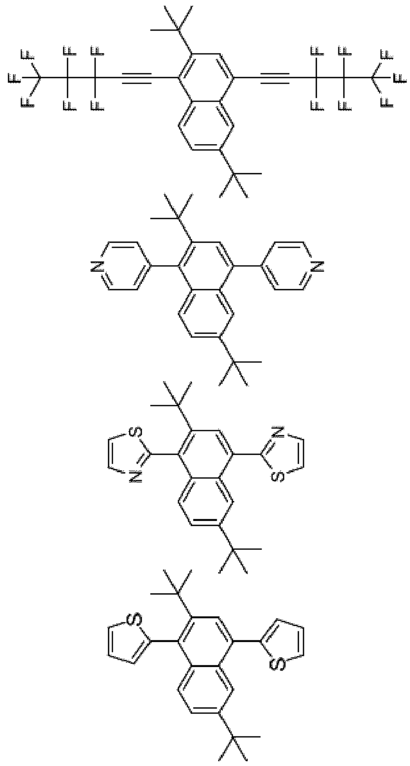

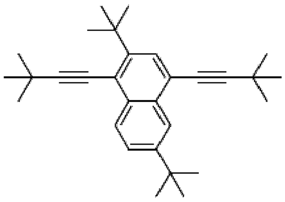

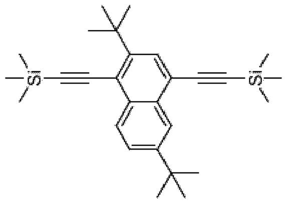

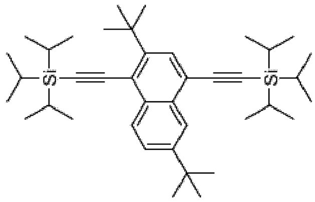

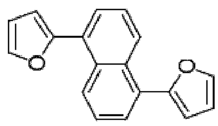

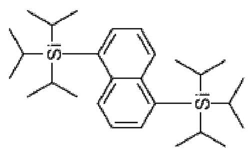

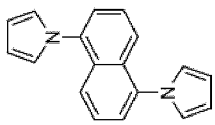

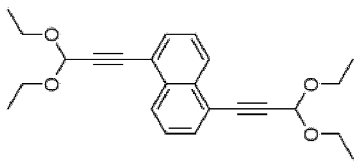

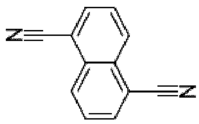

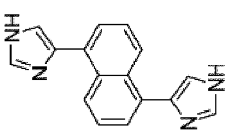

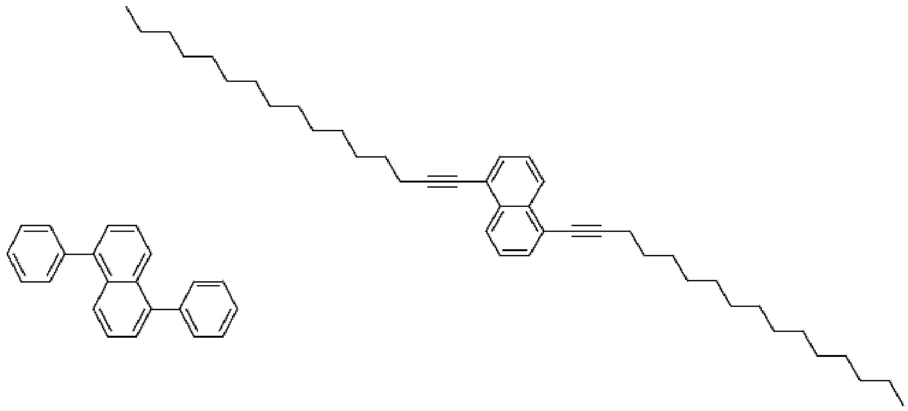

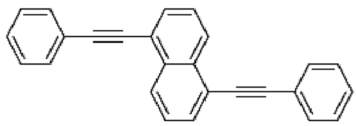

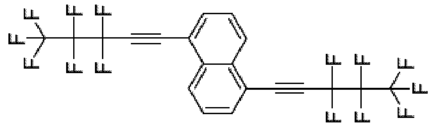

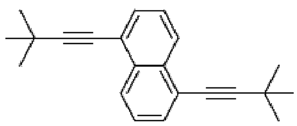

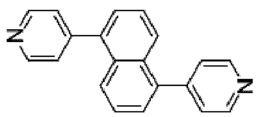

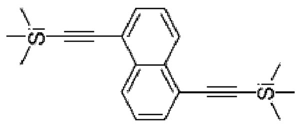

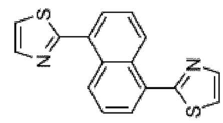

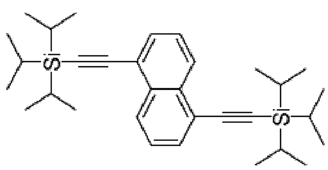

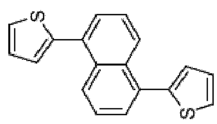

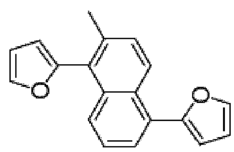

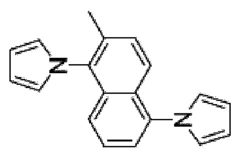

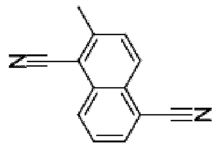

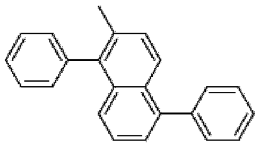

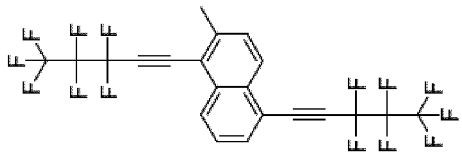

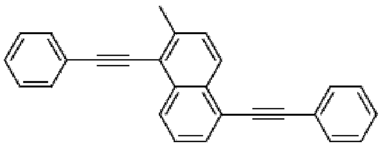

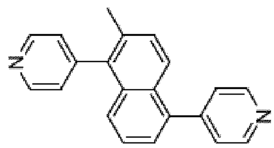

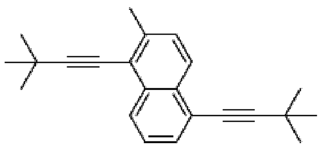

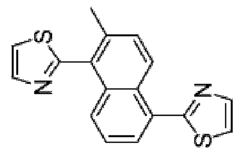

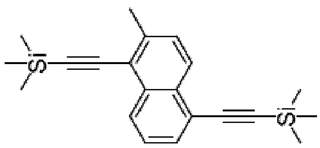

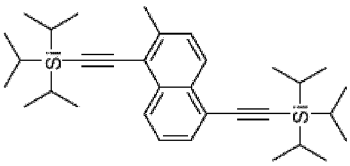

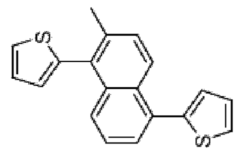

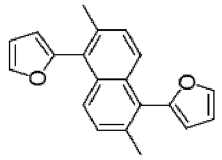

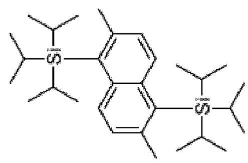

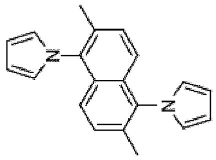

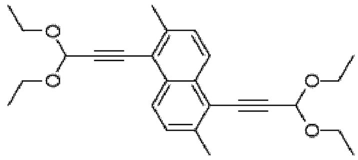

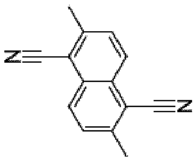

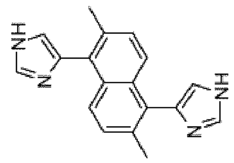

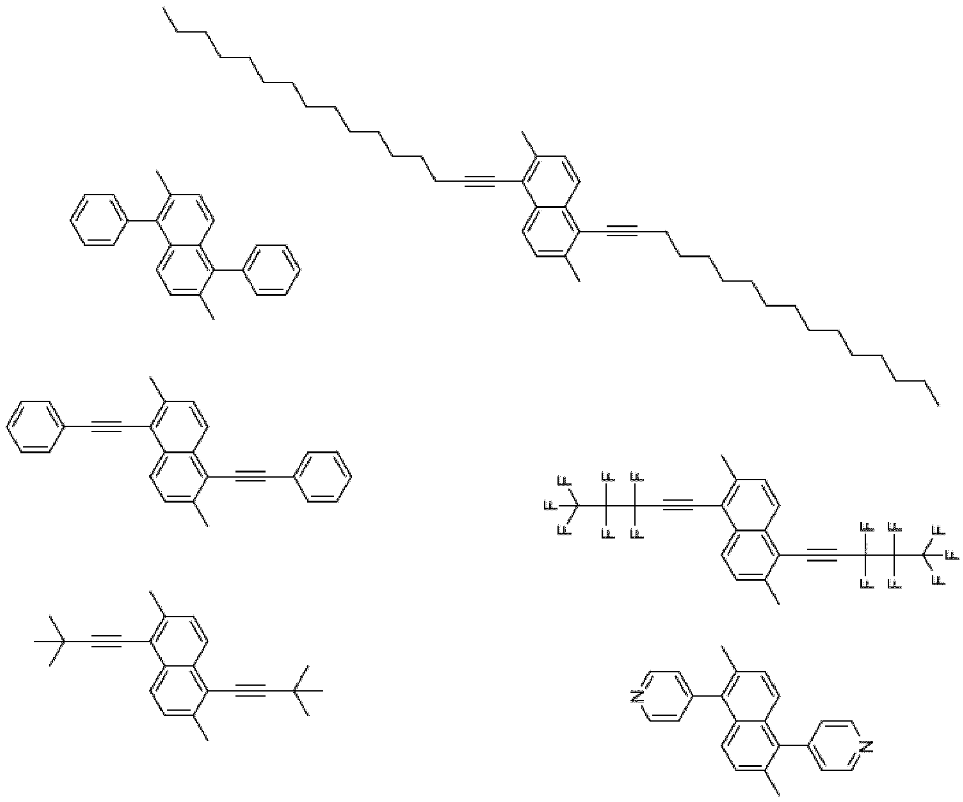

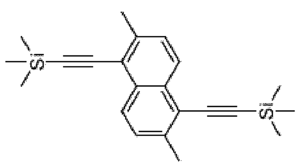

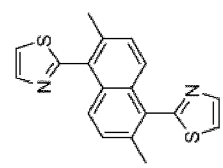

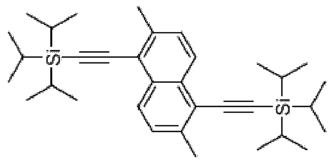

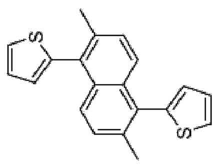

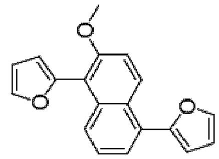

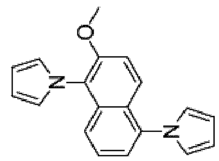

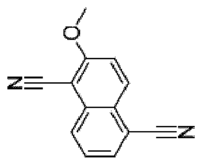

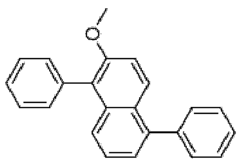

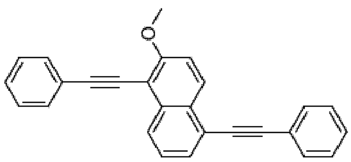

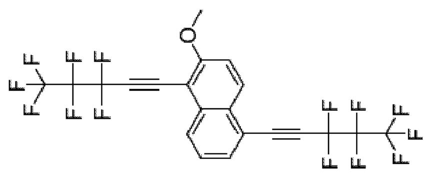

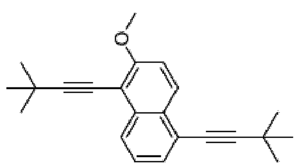

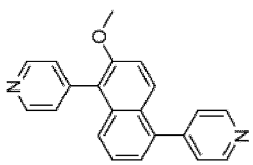

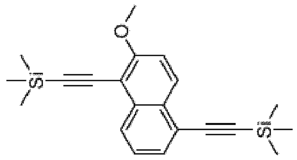

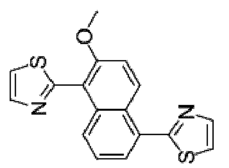

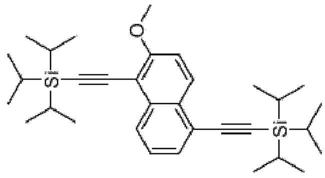

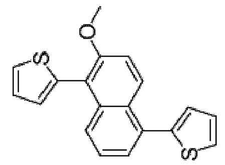

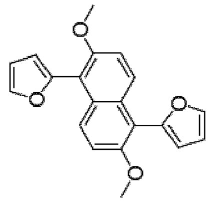

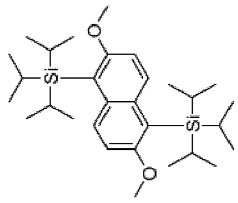

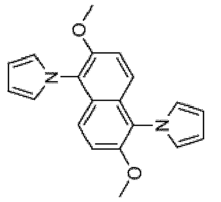

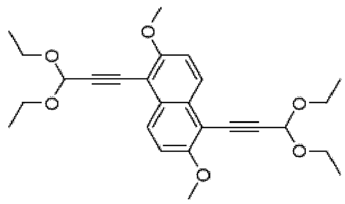

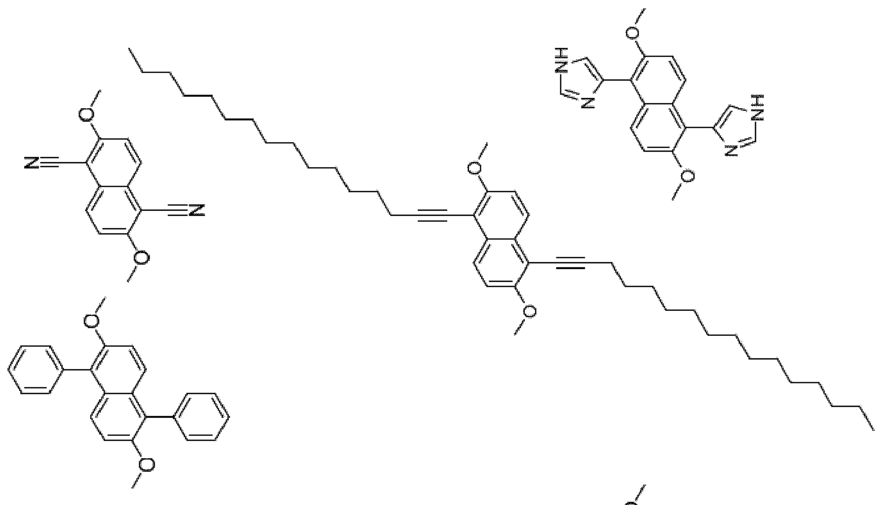

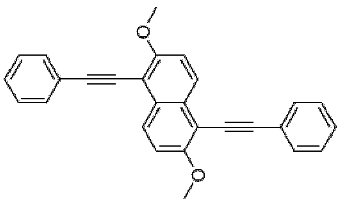

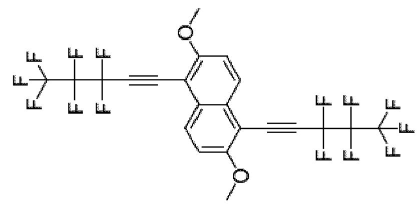

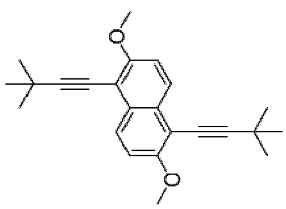

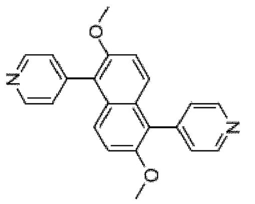

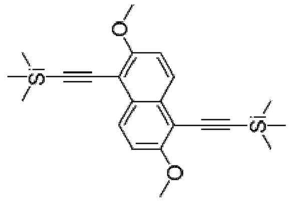

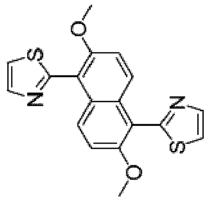

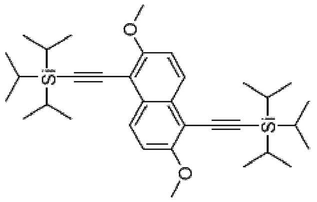

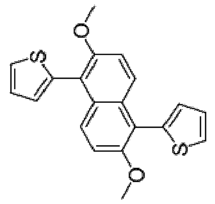

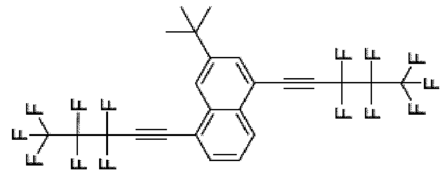

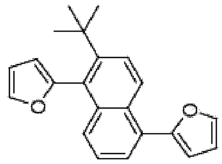

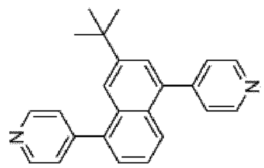

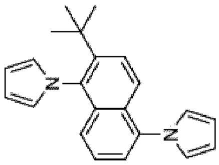

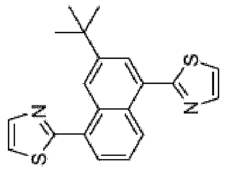

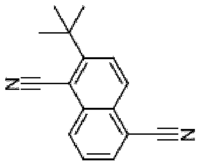

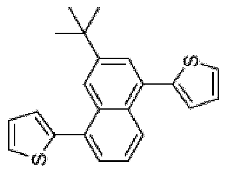

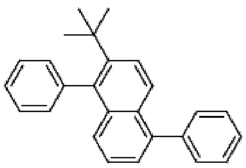

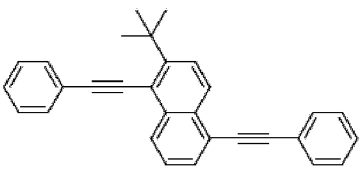

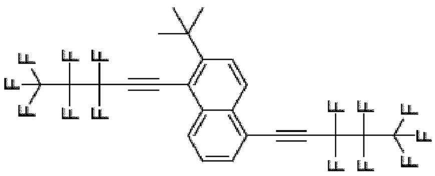

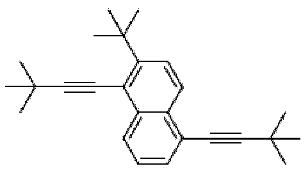
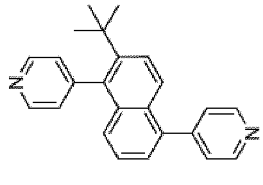
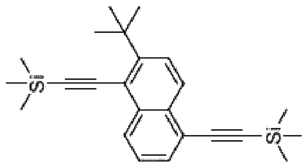
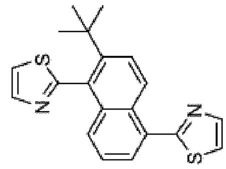
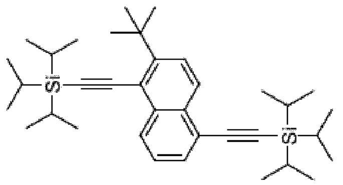
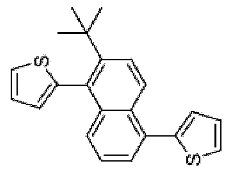
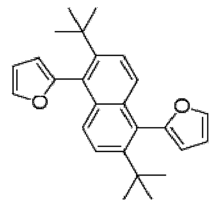
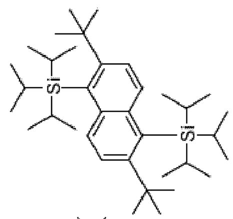
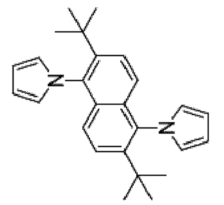
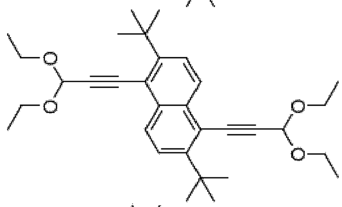
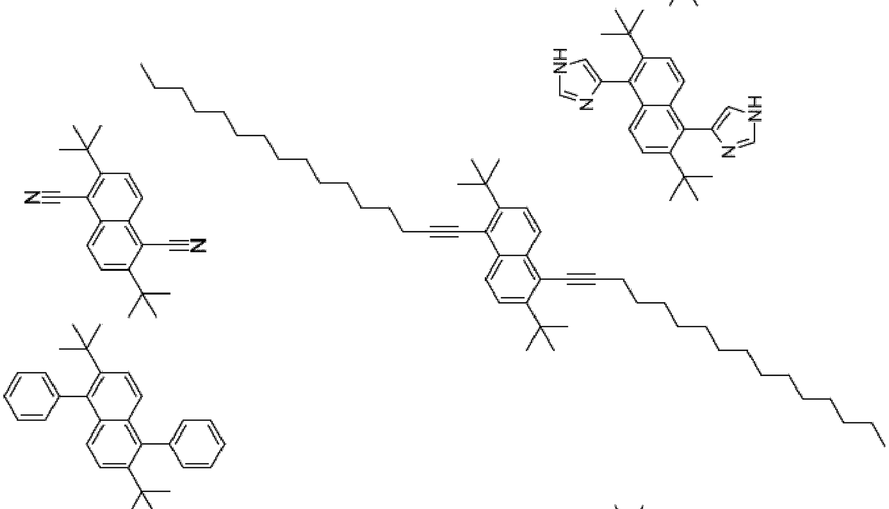
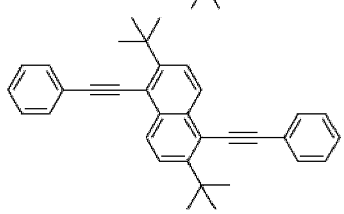
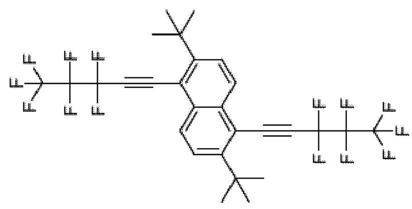

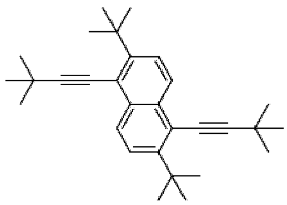

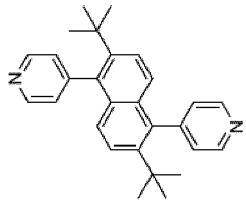

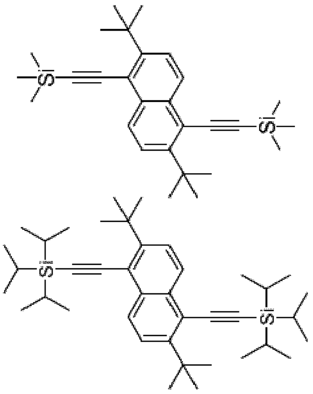

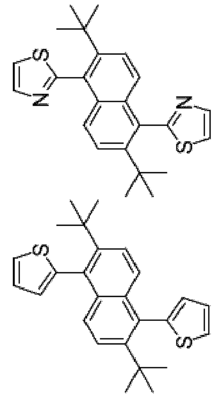

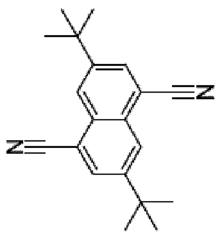

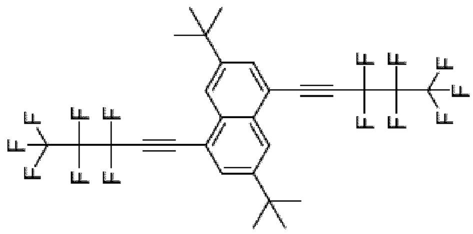

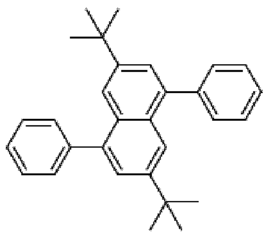

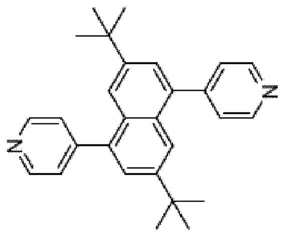

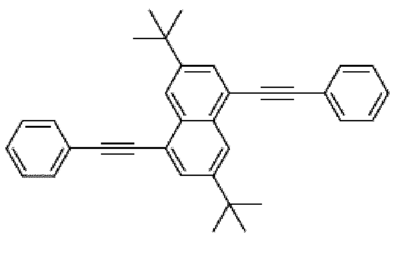

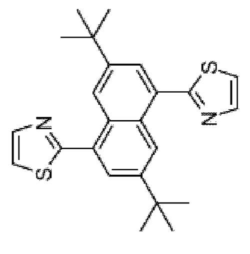

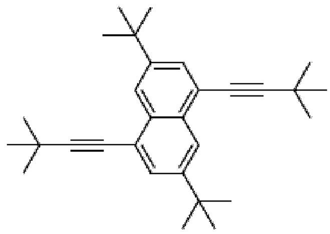

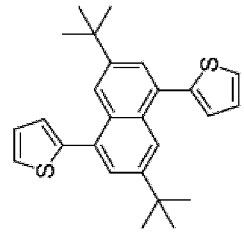

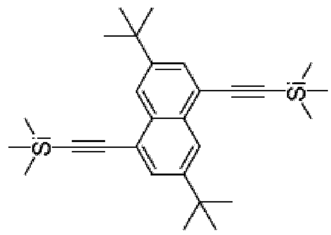

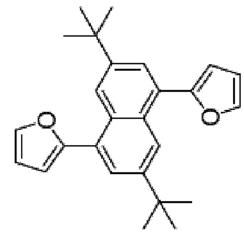

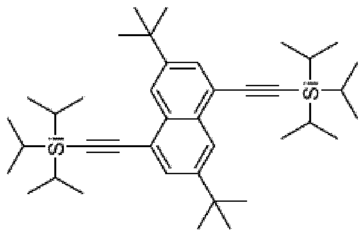

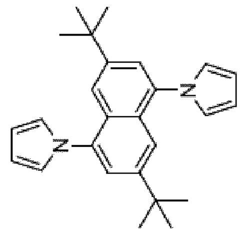

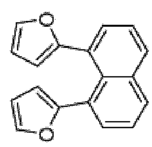

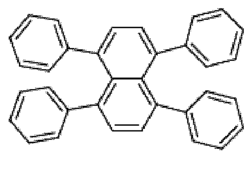

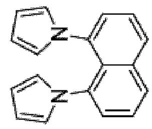

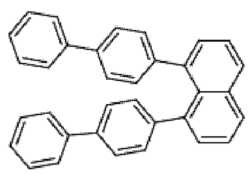

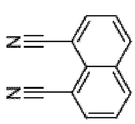

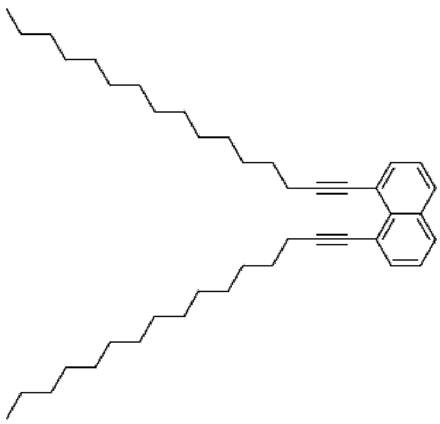

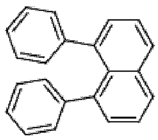

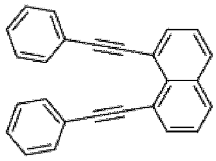

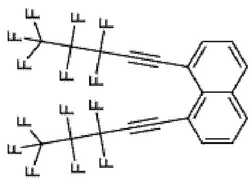

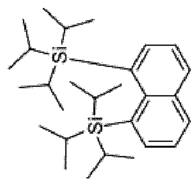

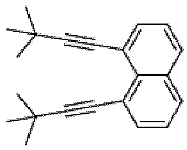

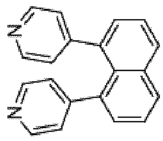

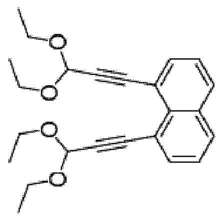

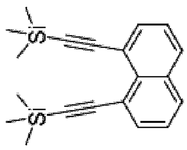

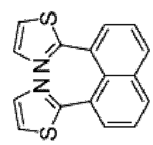

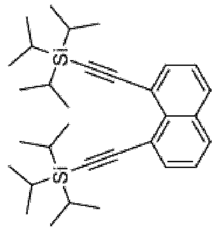

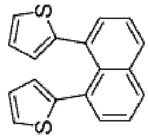

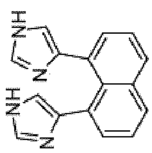

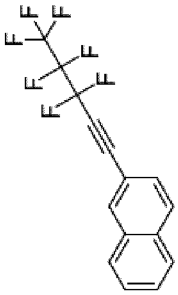

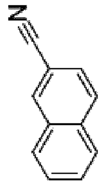

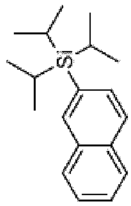

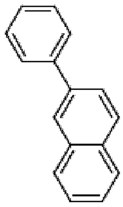

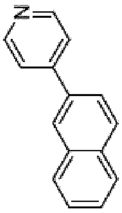

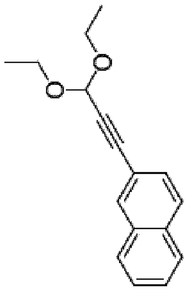

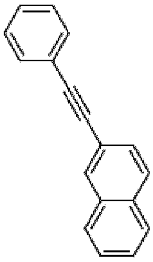

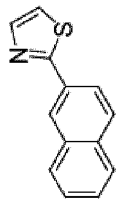

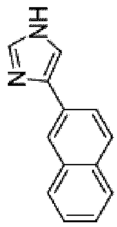

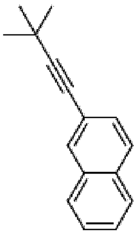

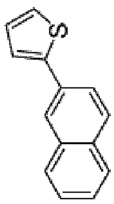

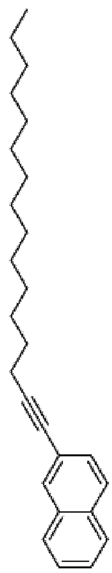

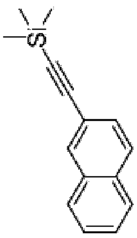

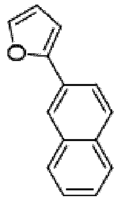

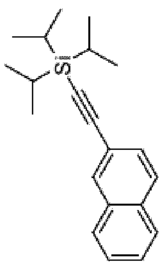

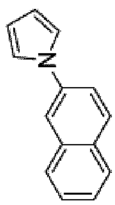

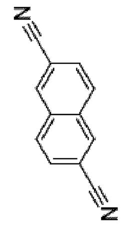

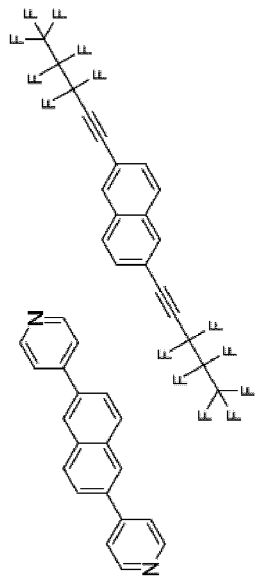

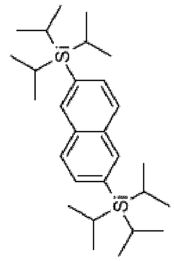

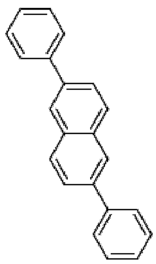

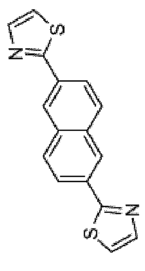

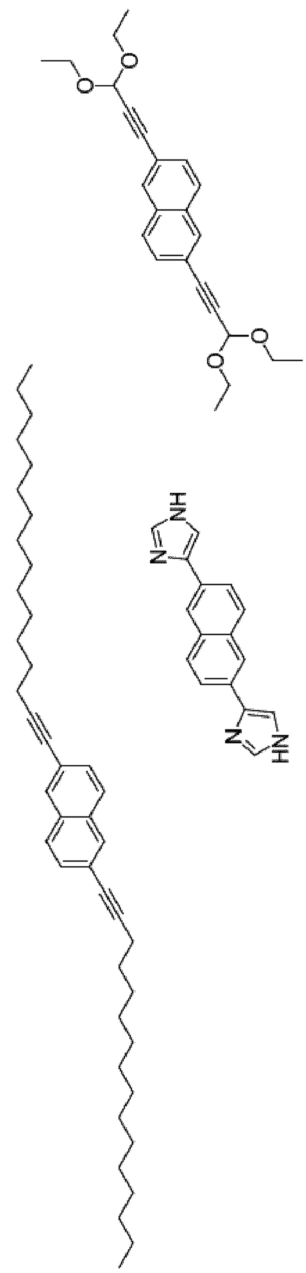

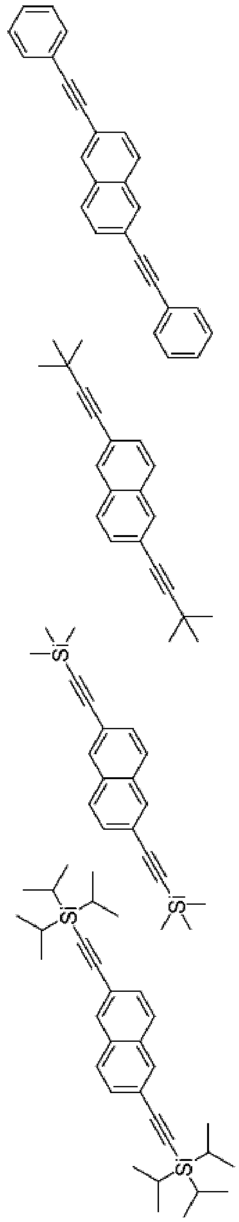

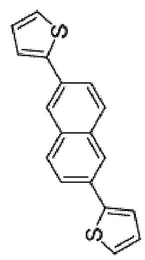

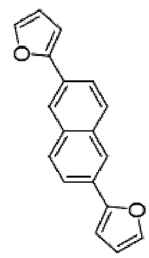

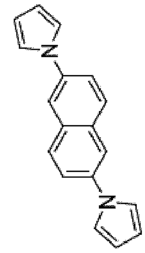

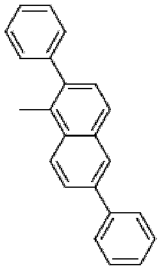

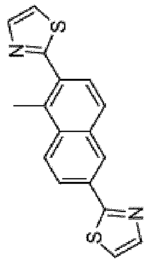

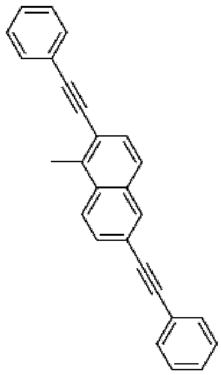

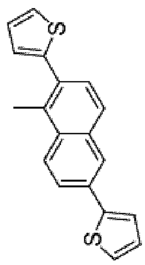

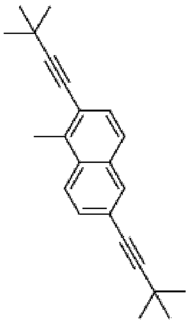

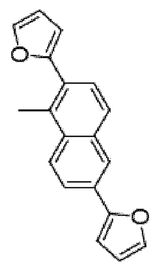

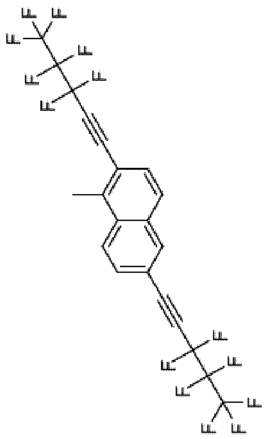

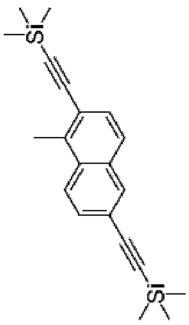

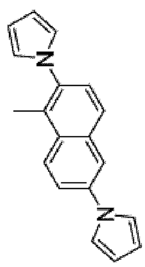

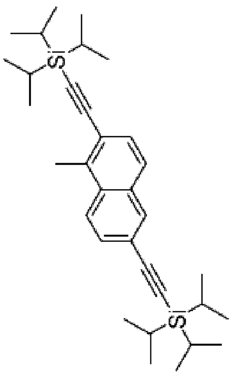

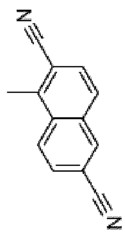

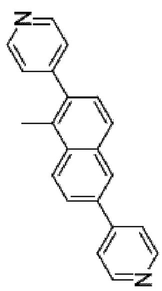

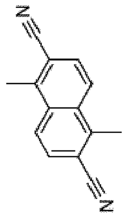

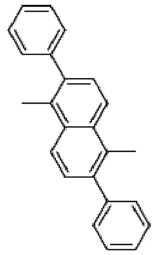

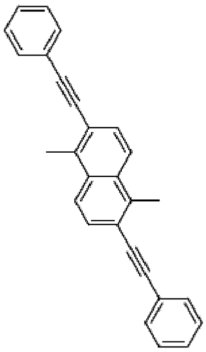

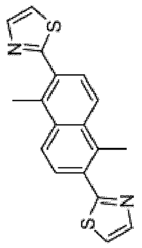

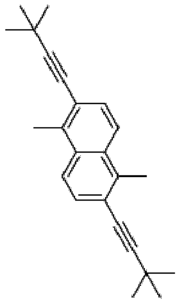

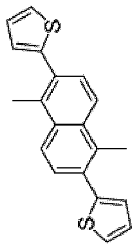

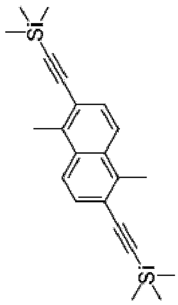

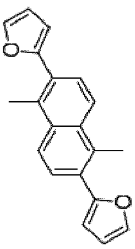

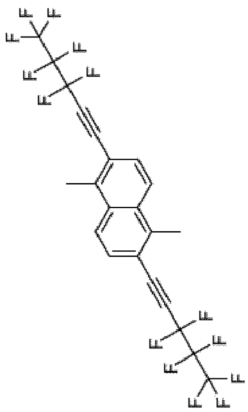

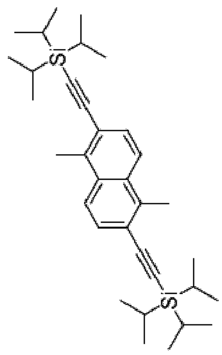

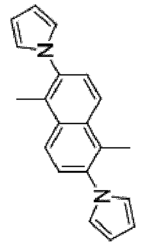

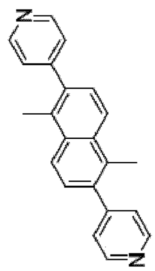

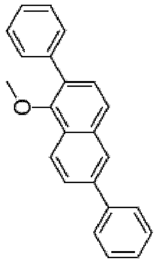

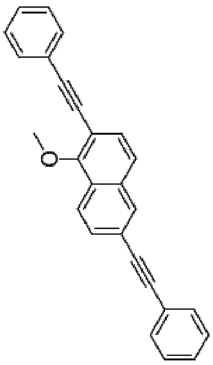

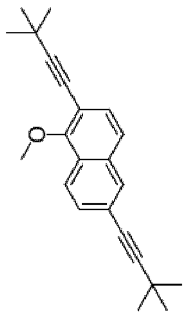

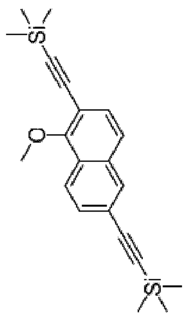

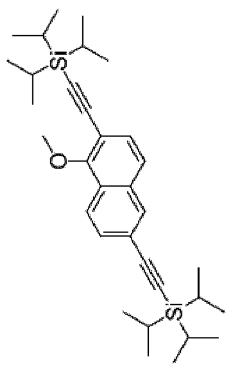

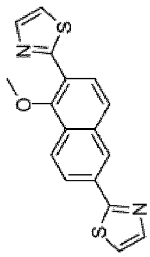

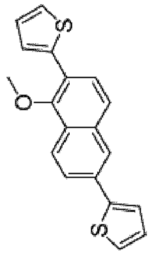

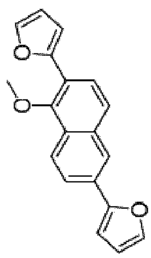

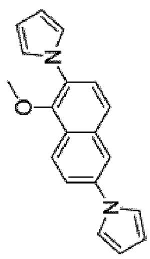

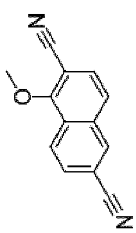

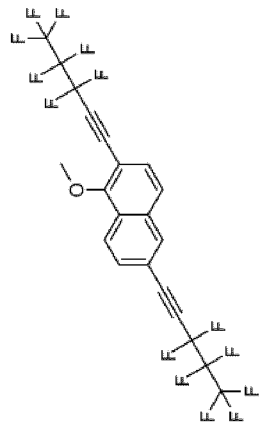

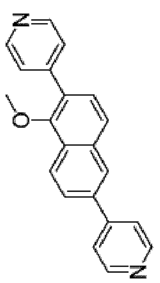

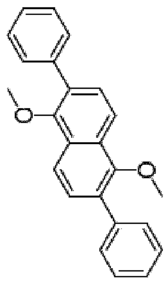

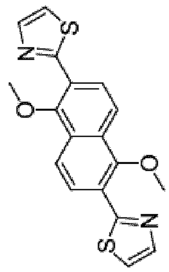

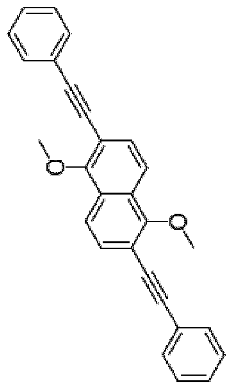

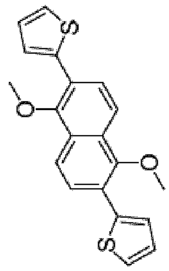

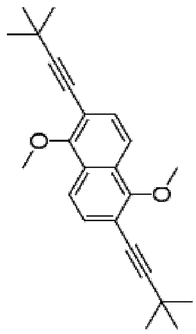

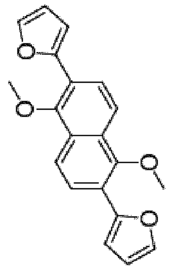

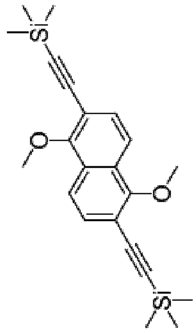

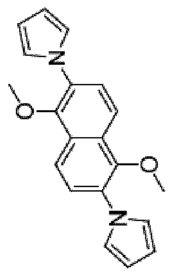

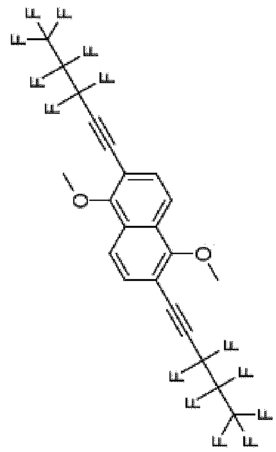

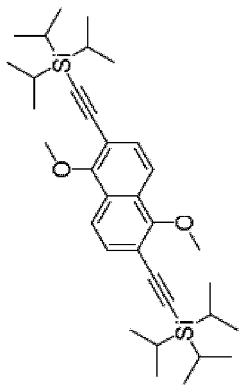

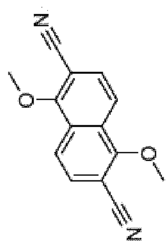

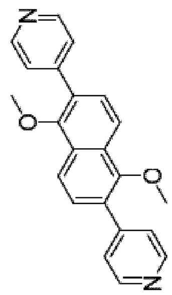

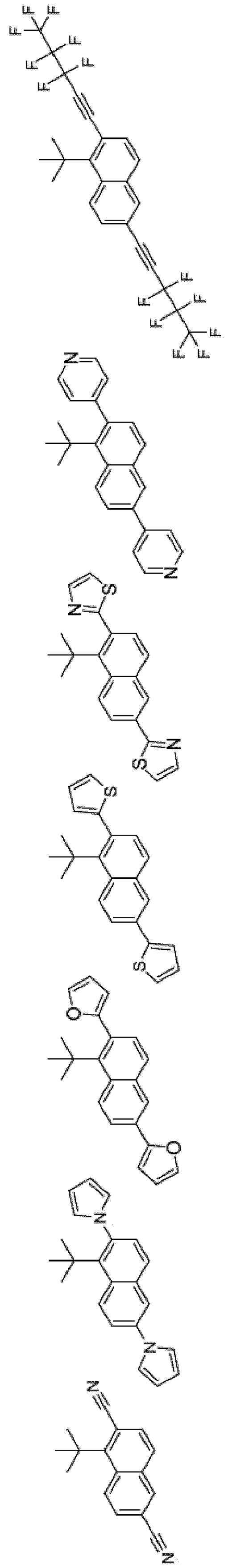
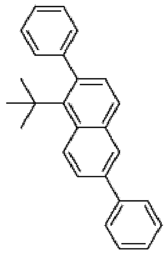
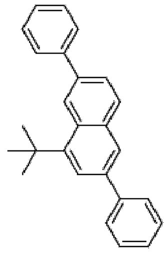
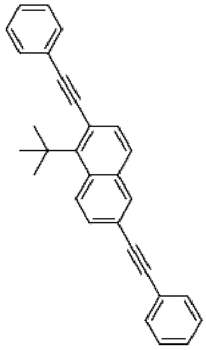
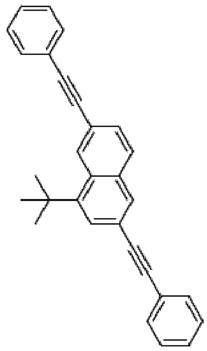
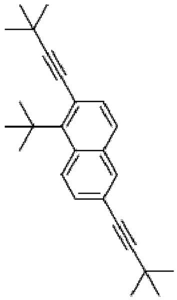
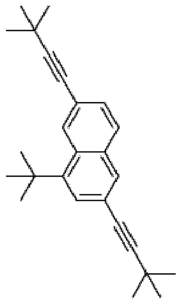
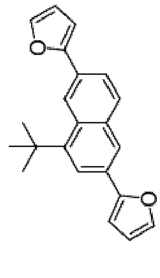
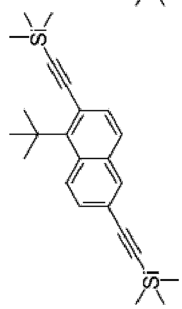
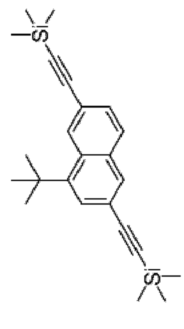
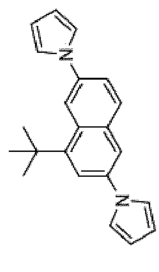
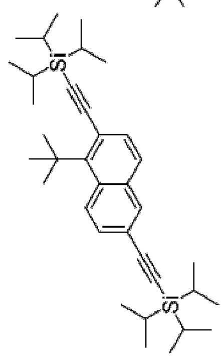
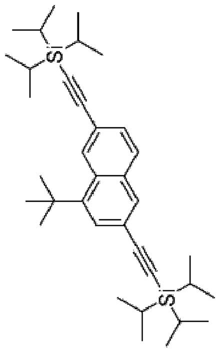
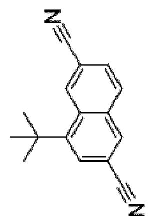

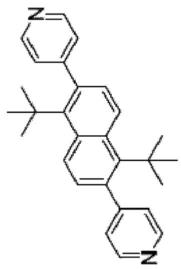

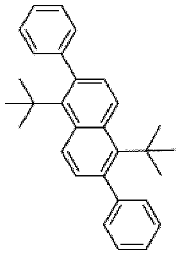

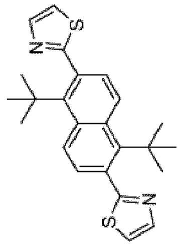

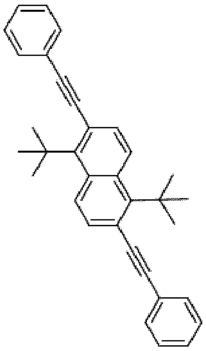

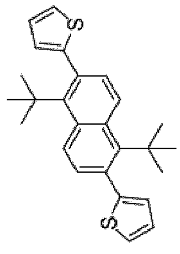

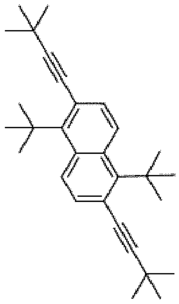

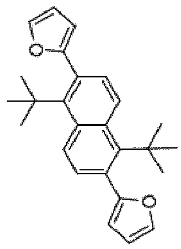

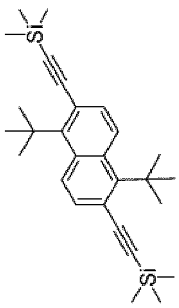

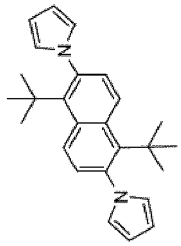

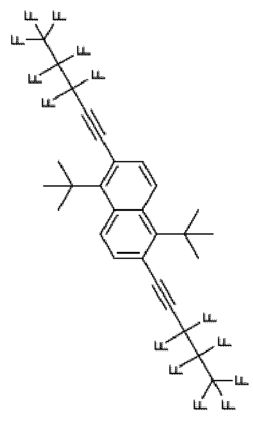

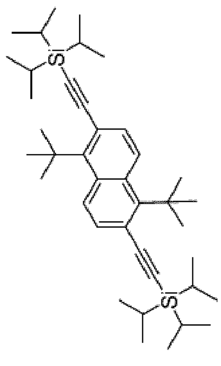

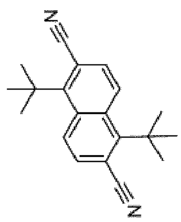

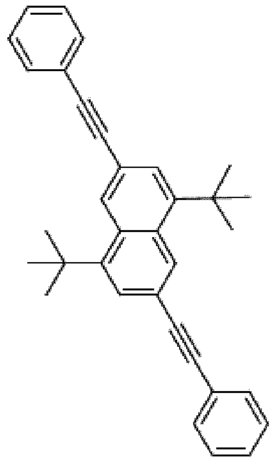

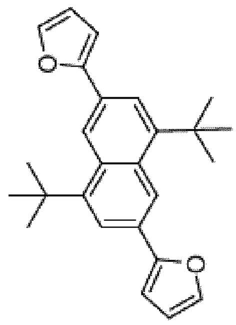

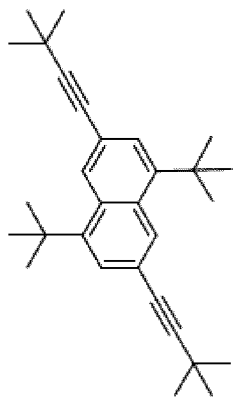

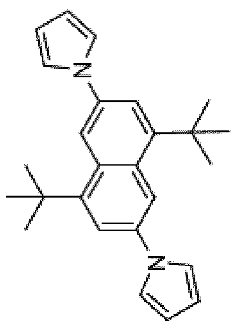

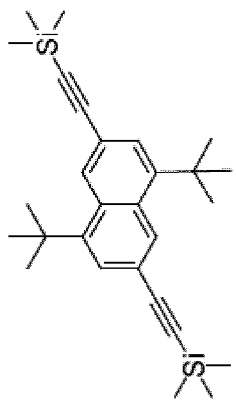

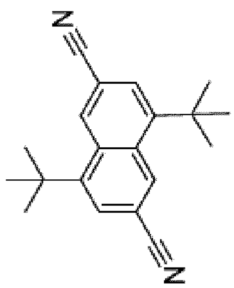

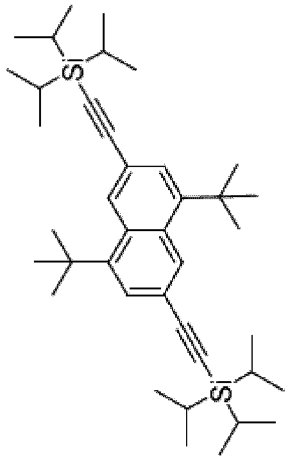

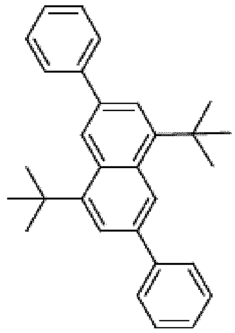

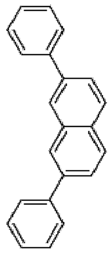

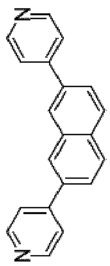

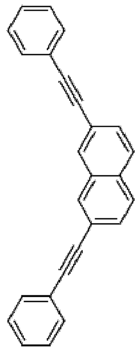

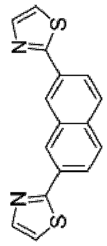

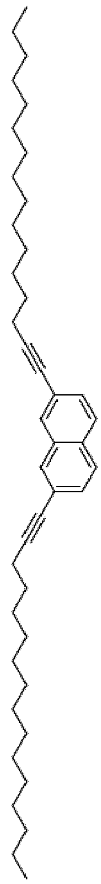

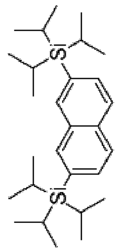

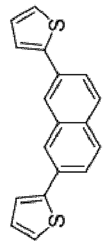

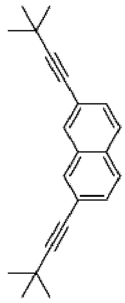

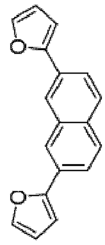

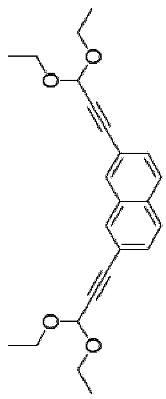

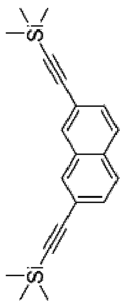

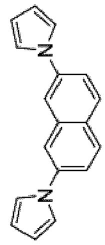

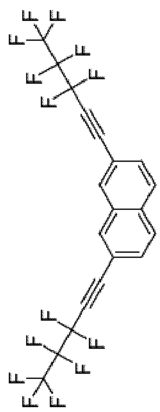

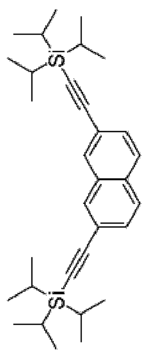

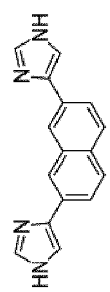

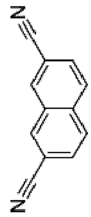

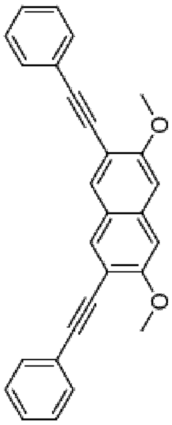
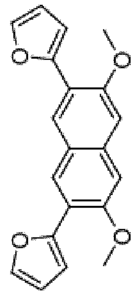
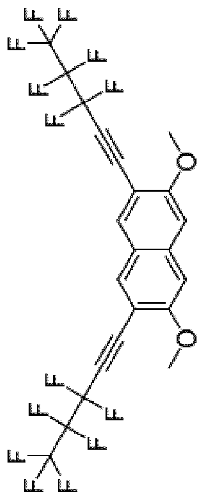
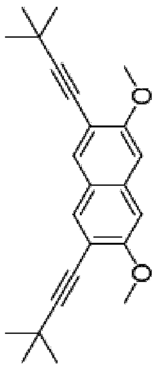
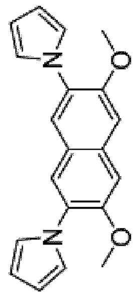
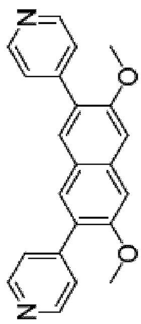
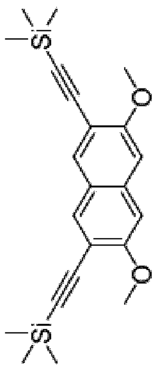
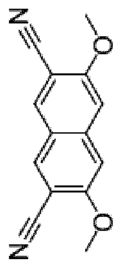
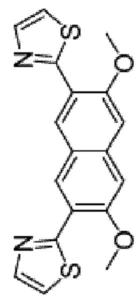
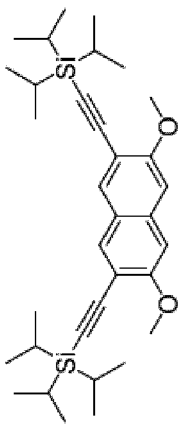
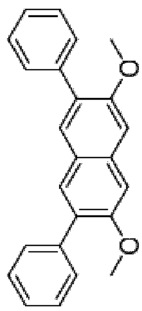
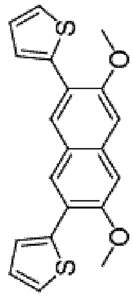

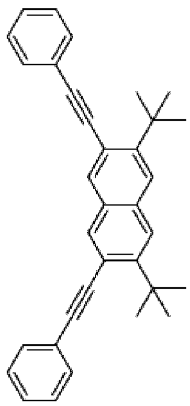

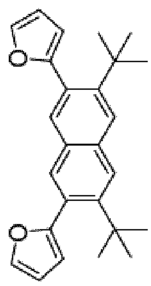

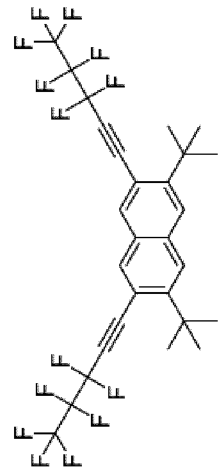

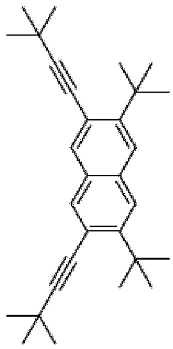

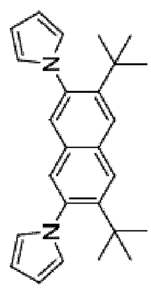

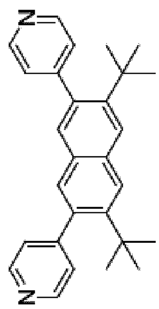

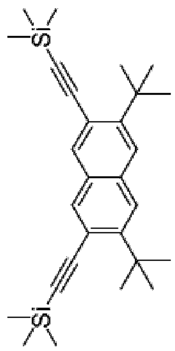

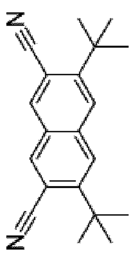

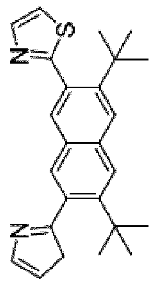

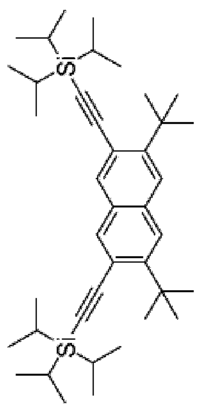

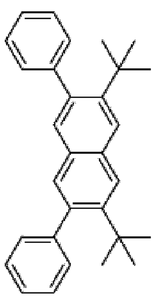

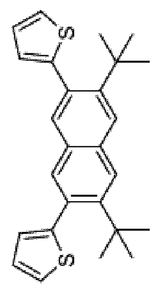

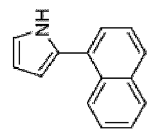
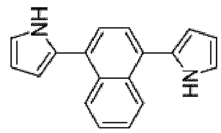
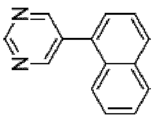
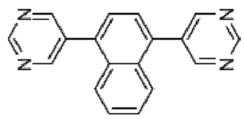
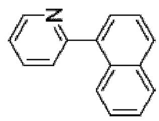
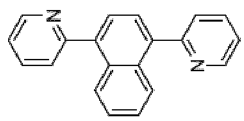
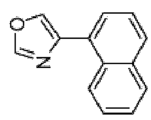
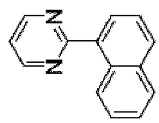
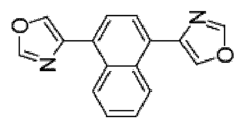
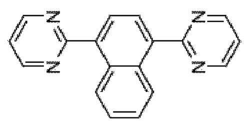
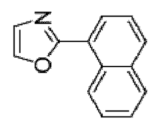
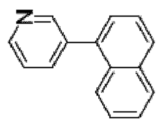
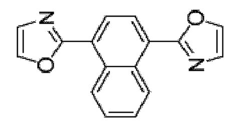
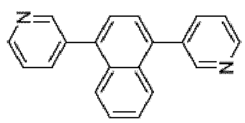
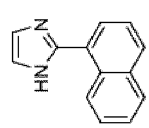
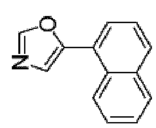
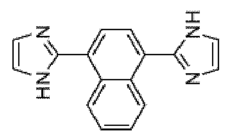
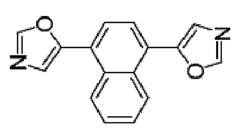
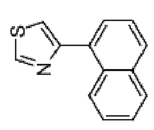
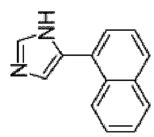
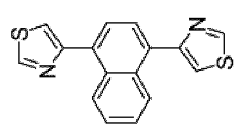
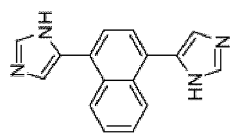
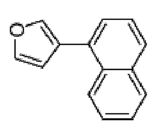
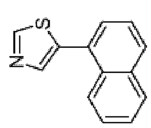
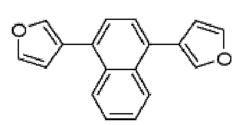
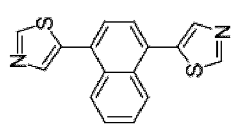
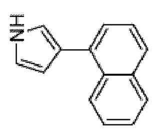
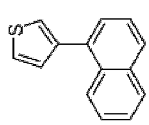
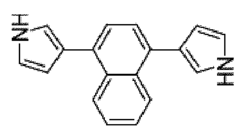
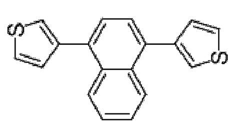
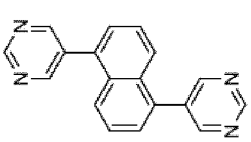
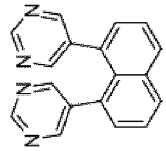

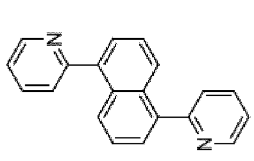
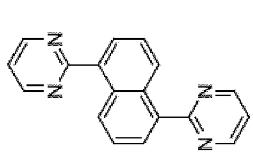
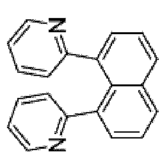
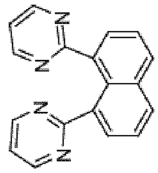

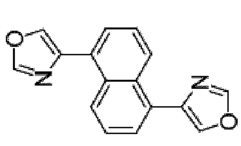
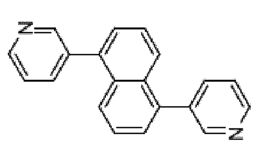
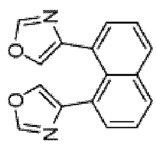
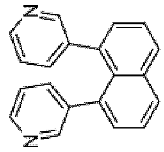

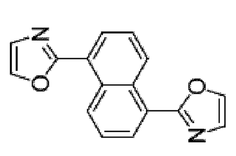
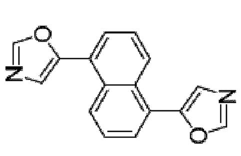
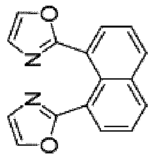
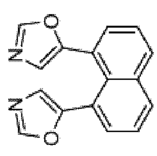

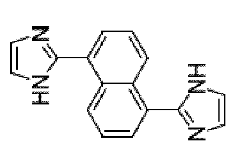
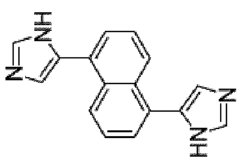
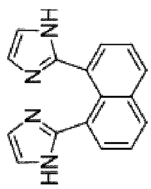
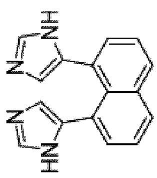

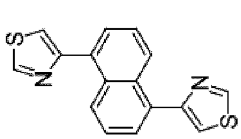
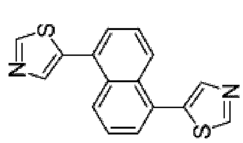
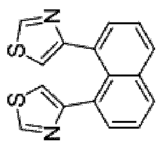
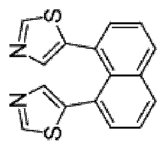

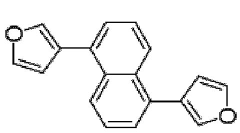
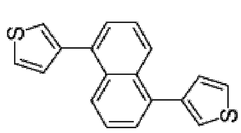
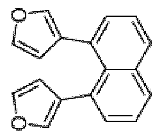
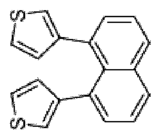

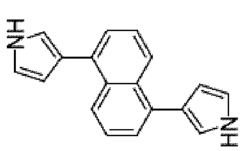
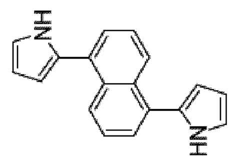
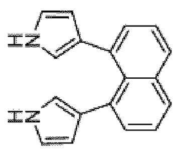
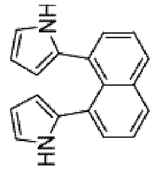

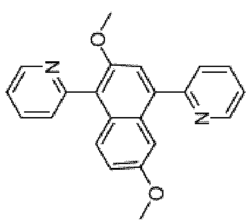
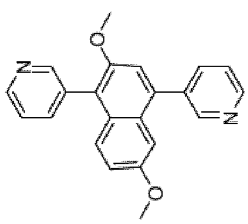

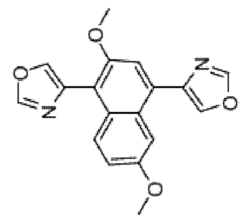

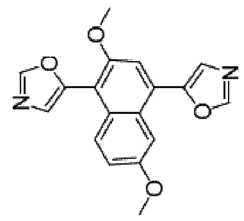

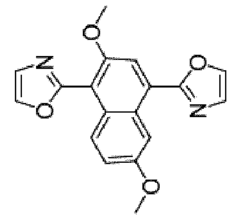

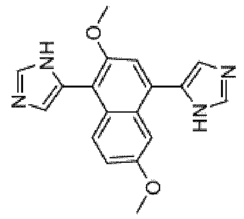

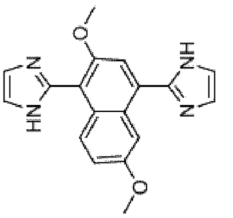

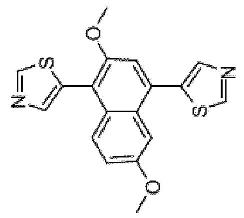

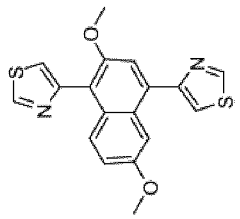

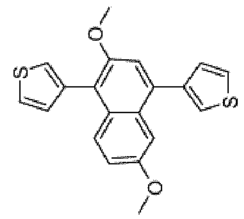

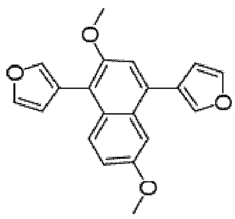

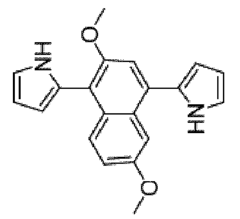

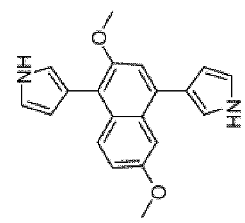

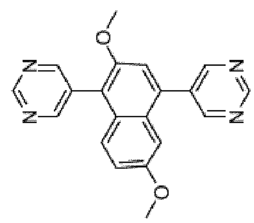

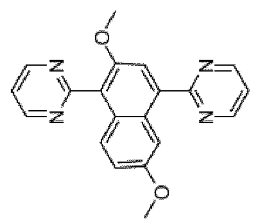

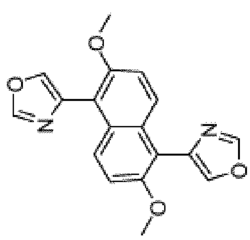

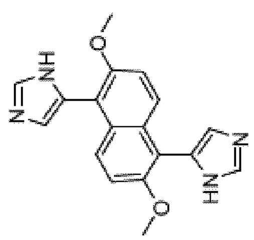

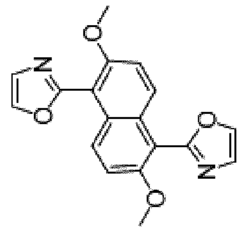
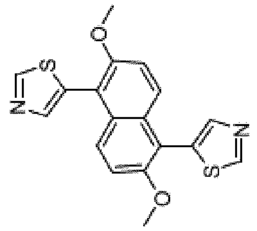
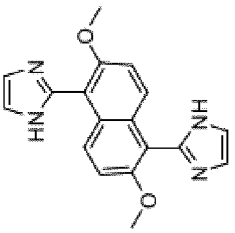
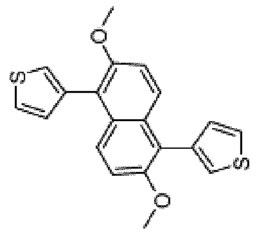
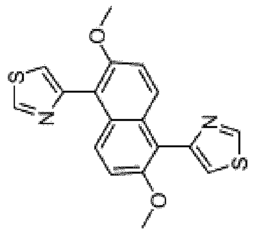
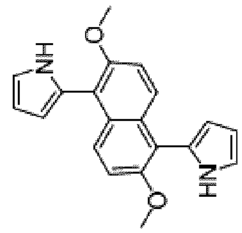
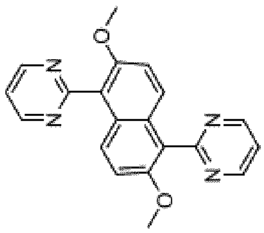
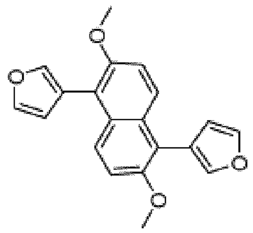
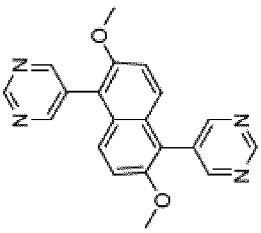
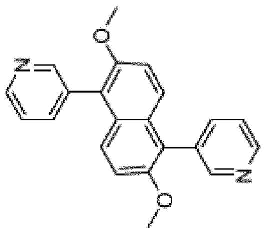
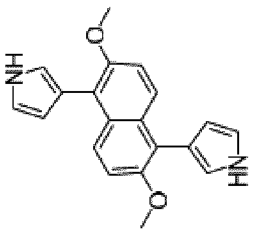
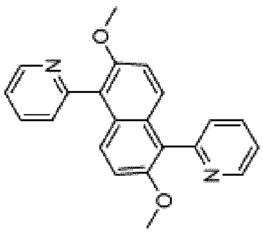
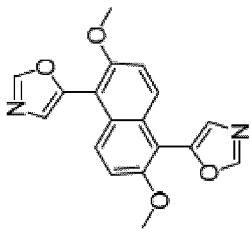
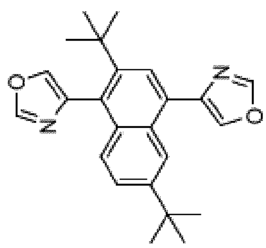

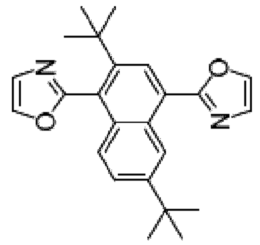
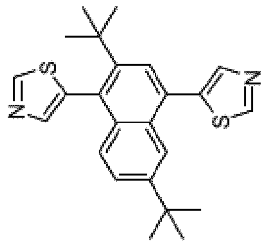

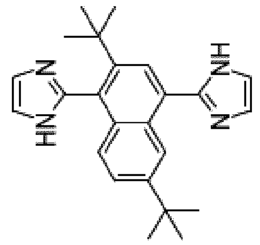
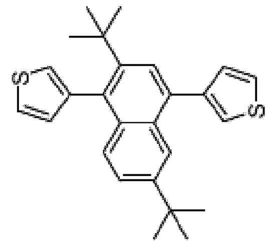

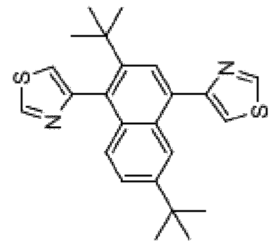
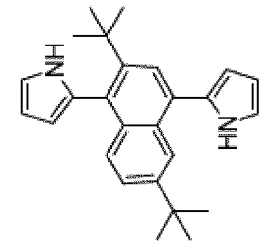
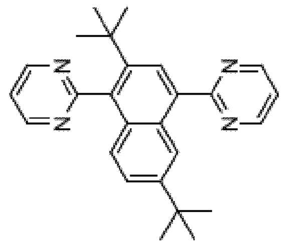

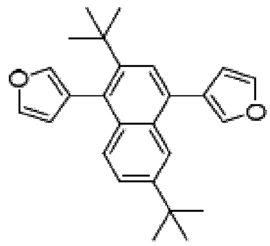
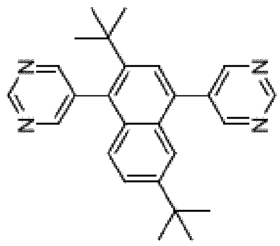
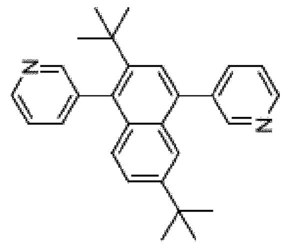

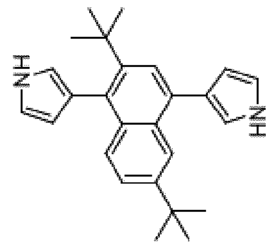
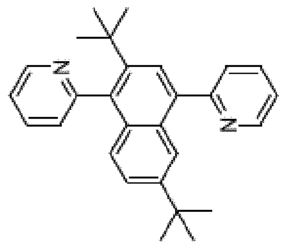
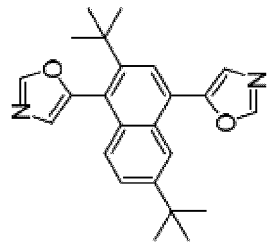

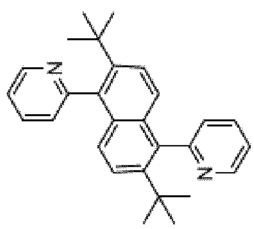
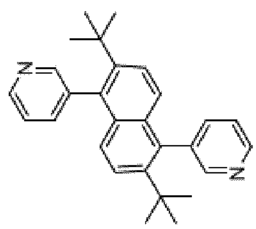

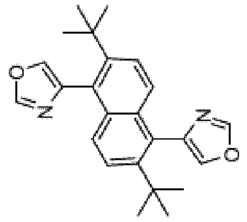
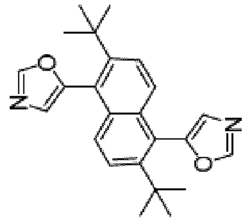

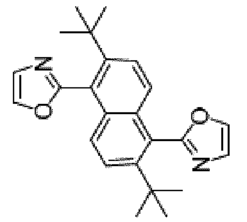
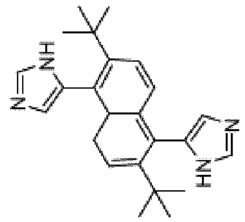

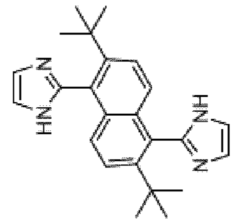
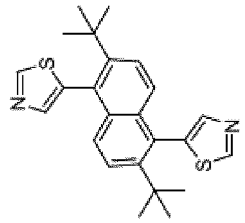

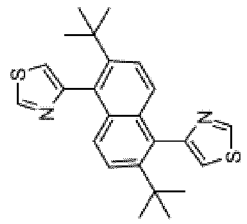
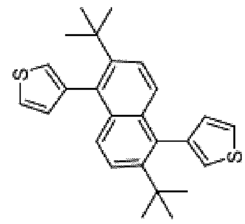

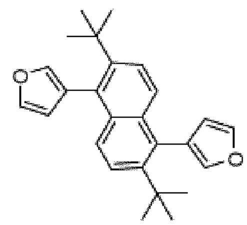
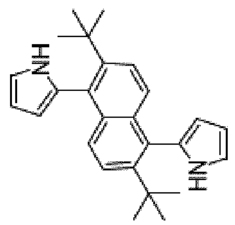

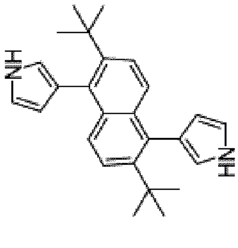
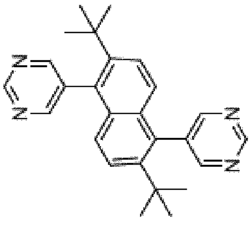
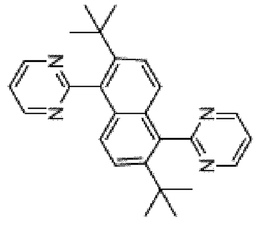

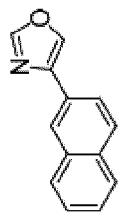
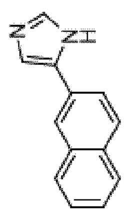

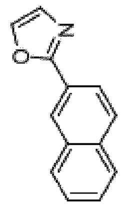
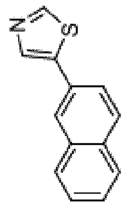

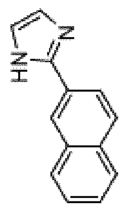
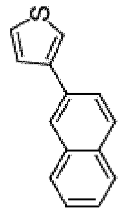

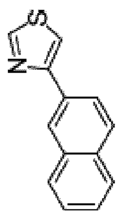
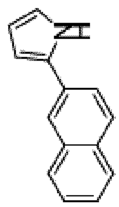
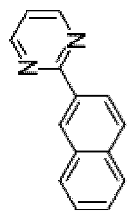

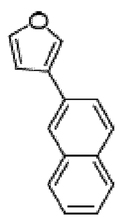
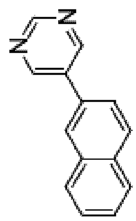
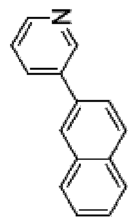

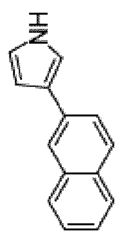
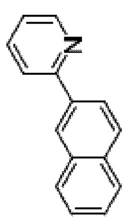
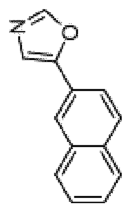

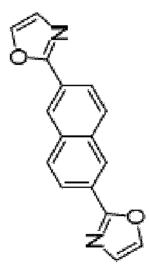
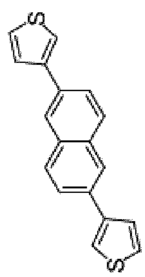
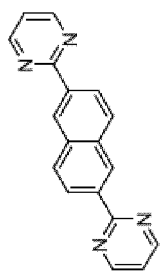

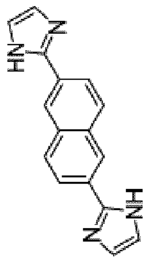 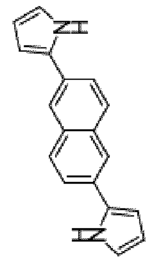 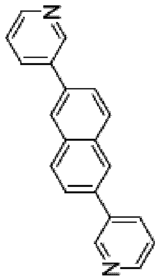

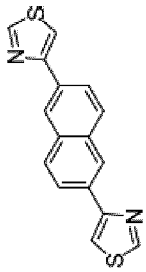 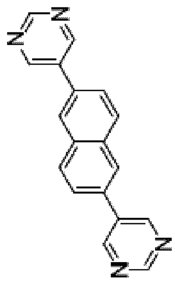 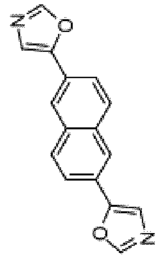

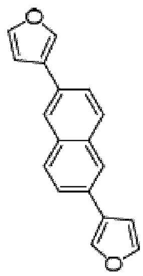 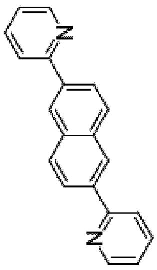 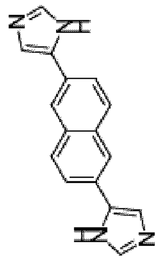

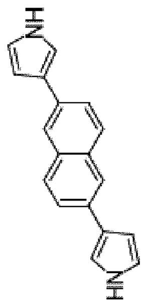 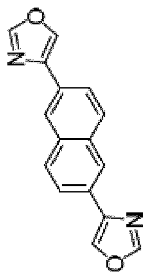 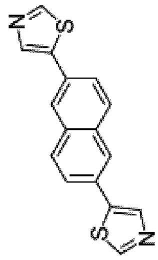

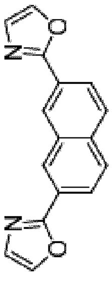 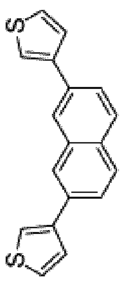 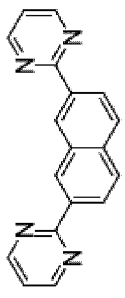

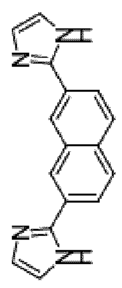
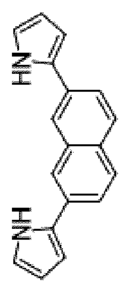
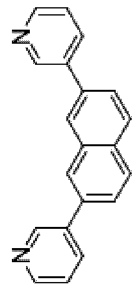
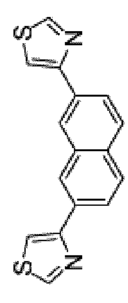
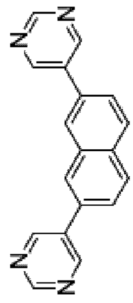
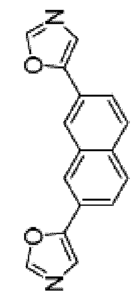
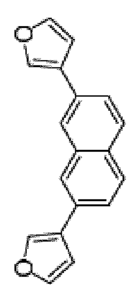
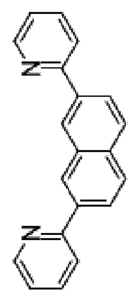
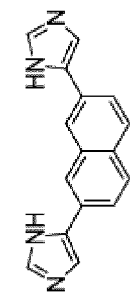
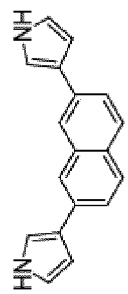
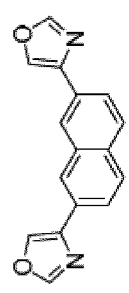
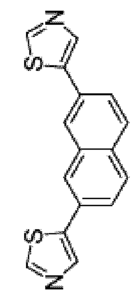
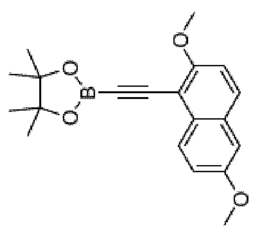
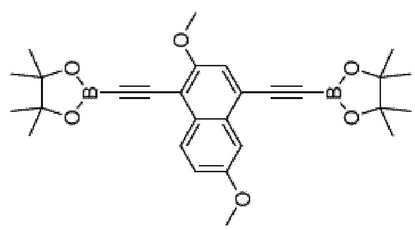

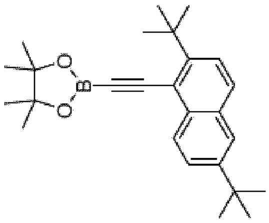

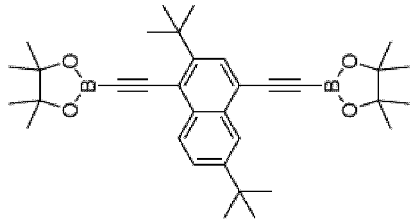

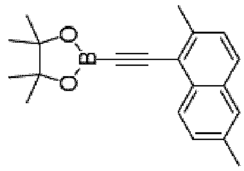

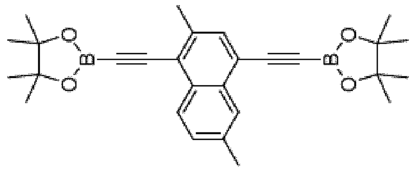

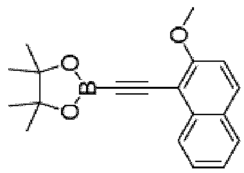

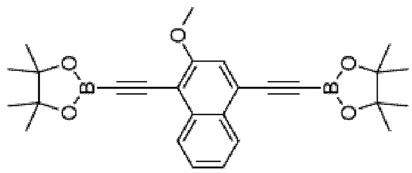

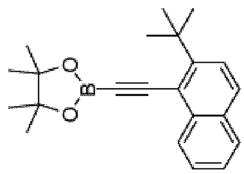

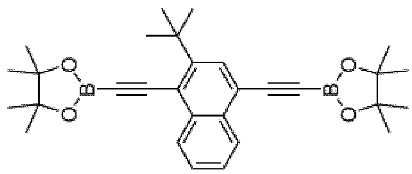

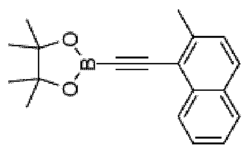

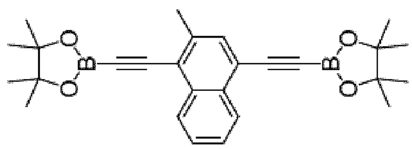

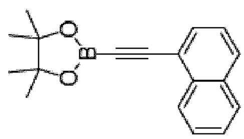

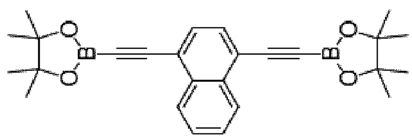

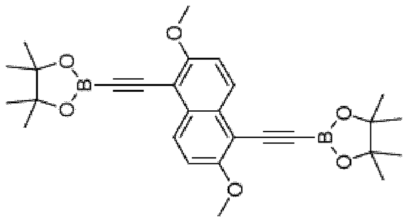

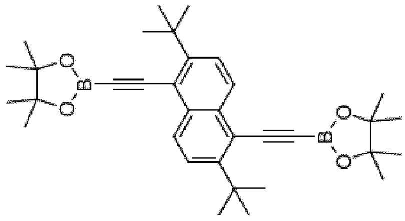

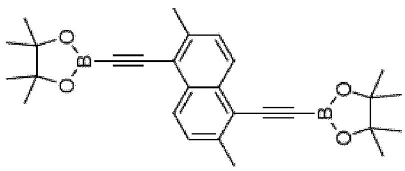

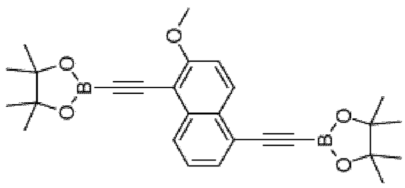

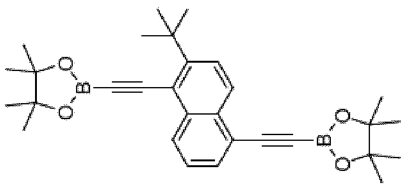

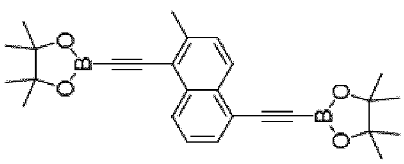

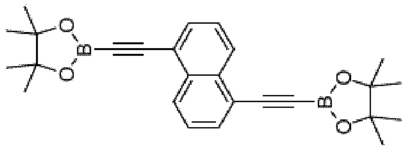

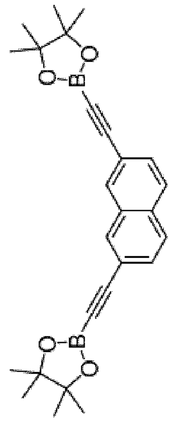

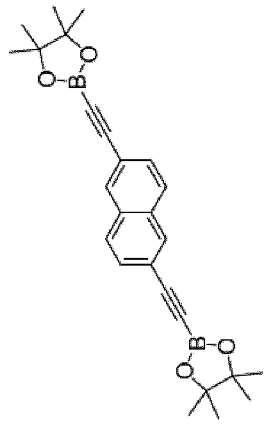

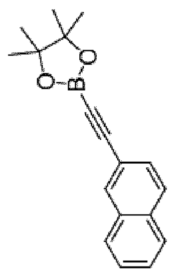

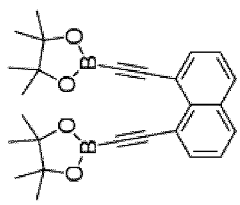

The photon upconversion composition of the present invention can contain only one kind of a naphthalene compound substituted with a specific group-containing substituent, or can contain two or more kinds thereof. Also the photon upconversion composition of the present invention can contain a naphthalene compound substituted with a specific group-containing substituent, and any other component than a naphthalene compound substituted with a specific group-containing substituent (the other component). The other component includes, for example, a donor compound (sensitizer) capable of donating the energy obtained by photoabsorption to the naphthalene compound. In the following description where the photon upconversion composition contains a donor compound, the naphthalene compound substituted with a specific group-containing substituent contained in the composition is referred to as an "acceptor compound".

It is presumed that the photon upconversion composition containing an acceptor compound and a donor compound can provide photon upconversion emission in triplet-triplet annihilation according to the mechanism, for example, as shown in Fig. 1. Hereinunder the UC light emission mechanism is described. Here, the donor compound and the acceptor compound are such that the lowest excited singlet energy level $S_{1,D}$ of the donor compound is lower than the lowest excited triplet energy level $S_{1,A}$ of the acceptor compound, and the lowest excited triplet energy level $T_{1,D}$ of the donor compound is higher than the lowest excited triplet energy level $T_{1,A}$ of the acceptor compound. The irradiation light to the composition is the excitation light for the donor compound. In Fig. 1, "ISC" indicates intersystem crossing, "TET" indicates triplet energy transfer from the donor compound to the acceptor compound, and "TTA" indicates triplet-triplet annihilation.

First, when a photon upconversion composition containing an acceptor compound and a donor compound is irradiated with an excitation light, the molecule of the donor compound (donor molecule) absorbs the light to be excited in an excited singlet state ($S_{1,D}$), and then undergoes intersystem crossing to transition into an excited triplet state ($T_{1,D}$), as shown in Fig. 1. The energy of the donor molecule thus being in the excited singlet state transfers to the molecule of the acceptor compound (acceptor molecule). As a result of this, in the acceptor compound thus being in an excited triplet state ($T_{1,A}$), the triplets of two molecules meet to undergo triplet-triplet annihilation and one molecule thereof transitions into an excited singlet state ($S_{1,A}$). Namely, there occurs photon upconversion by the triplet-triplet annihilation of the acceptor molecules. The acceptor molecule thus being in an excited singlet state emits fluorescence (UC light) and deactivates, thereby, the composition provides UC light emission. At that time, the excited singlet state ($S_{1,A}$) to form through photon upconversion by triplet-triplet annihilation has an extremely high energy level, and therefore by radiation deactivation from the excited singlet state, a UC light having a higher energy (having a shorter wavelength) than the excitation light is thus formed.

[Donor Compound]

The donor compound for use in the photon upconversion composition of the present invention is preferably a compound satisfying the following requirement (A), more preferably a compound satisfying all the following requirements (A) to (C).

(A) Having a lower lowest excited singlet energy level $S_{1,D}$ than the lowest excited singlet energy level $S_{1,A}$ of the acceptor compound, and having a higher lowest excited triplet energy level $T_{1,D}$ than the lowest excited triplet energy level $T_{1,A}$ of the acceptor compound.
(B) Having a high absorbance.
(C) Having a low absorbance in the region at least overlapping with the wavelength region of the UC light among the UV region.

The molecule of the donor compound satisfying the requirement (A) has a lower lowest excited singlet energy level $S_{1,D}$ than the lowest excited singlet energy level $S_{1,A}$ of the acceptor compound, and therefore can be excited to be in an excited singlet state by an irradiation light having a longer wavelength than UC light. Also the molecule of the donor compound satisfying the requirement (A) has a higher lowest excited triplet energy level $T_{1,D}$ than the lowest excited triplet energy level $T_{1,A}$ of the acceptor compound, and therefore the excited triplet energy thereof can be readily transferred to the acceptor molecule. This makes it possible to more reliably allow the mechanism of UC light emission shown in Fig. 1 to function.

The difference between the lowest excited singlet energy level $S_{1,A}$ of the acceptor compound and the lowest excited singlet energy level $S_{1,D}$ of the donor compound, ($S_{1,A}$-$S_{1,D}$) is preferably 0.3 to 2 eV, more preferably 0.4 to 1 eV, even more preferably 0.4 to 0.8 eV

The difference between the lowest excited triplet energy level $T_{1,D}$ of the donor compound and the lowest excited triplet energy level $T_{1,A}$ of the acceptor compound, ($T_{1,D}$-$T_{1,A}$) is preferably 0.01 to 1 eV, more preferably 0.01 to 0.5 eV, even more preferably 0.01 to 0.2 eV.

Regarding the measurement method for the lowest excited singlet energy level $S_{1,A}$, $S_{1,D}$ and the lowest excited triplet energy level $T_{1,A}$, $T_{1,D}$, reference can be made to the description in the section of (Measurement method for lowest

excited singlet energy level $S_1$ and lowest excited triplet energy level $T_1$) given hereinunder. These energy levels can be determined also by density-functional calculation.

The compound satisfying the requirement (A) and also satisfying the requirements (B) and (C) can efficiently absorb a radiation light, but on the other hand does not absorb a UC light, and therefore using the compound of the type as a donor compound, the threshold excitation intensity Icn can be lowered more and the emission efficiency of UC light emission can be increased more. Here, "threshold excitation intensity Icn" is an excitation light intensity at the refraction point on the double logarithmic chart that shows the excitation light intensity dependence of UC light emission intensity, and a lower value thereof means a lower excitation light intensity necessary for UC light emission, or in other words, means that UC light emission occurs more efficiently.

In the requirement (B), "high absorbance" means that the absorbance index is 50,000 $M^{-1}m^{-1}$ or more.

In the requirement (C), "low absorbance" means that the absorbance index is 40,000 $M^{-1}cm^{-1}$ or less.

Specific examples of the donor compound are shown below, but the donor compound usable in the present invention should not be limitatively interpreted by these specific examples.

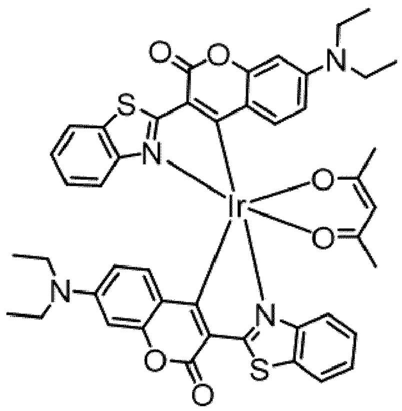

Compound D1

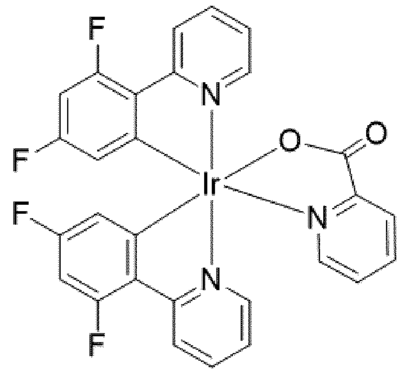

Compound D2

Of these compounds, Compound D1 has a large absorbance index, and additionally has an extremely low absorbance in the wavelength region of UC light emission of the naphthalene compound used in the present invention, and is therefore favorable as a donor compound. In addition to these compounds, organic donor compounds not containing a metal element are also favorably employable here. As the organic donor compound, especially preferred are thermally activated delayed fluorescent materials. Among thermally activated delayed fluorescent materials, those that have heretofore been used as donors for photon upconversion compositions can be appropriately selected and used here.

(Measurement method for lowest excited singlet energy level $S_1$ and lowest excited triplet energy level $T_1$)

The lowest excited singlet energy level $S_1$ of the compound for use in the present invention can be determined by measuring the fluorescent spectrum after preparing a solution of the compound, and by converting the wavelength of the fluorescence peak on the shortest wavelength side into an energy value according to the following conversion expression. The lowest excited triplet energy level $T_1$ of the compound for use in the present invention can be determined by measuring the phosphorescent spectrum after preparing a solution of the compound, and by converting the wavelength value of the fluorescence peak is converted into an energy value according to the following conversion expression. The solvent to be used in preparing a solution of the compound is selected as to be able to dissolve the compound (for example, tetrahydrofuran). The concentration of the solution can be one that enables spectrometry (for example, 100 μM). For measuring the emission spectrum, JASCO FP-8300 by JASCO Corporation can be used with a xenon lamp as an excitation light source.

$$\text{Conversion Expression: } S_1\ [\text{eV}] = 1239.85/\lambda_F$$

$$\text{Conversion Expression: } T_1\ [\text{eV}] = 1239.85/\lambda_P$$

In the above expressions, $\lambda_F$ is a fluorescence peak wavelength [nm], and $\lambda_P$ is a phosphorescence peak

wavelength [nm].

[Blending Ratio of acceptor compound and donor compound]

When the photon upconversion composition contains a naphthalene compound substituted with a specific group-containing substituent (acceptor compound) and a donor compound, the molar ratio of the donor compound to the acceptor compound [(molar number of donor compound/molar number of acceptor compound) × 100] is preferably 0.01 to 20%, more preferably 0.1 to 10%, even more preferably 1 to 5%. When the absorbance of the donor compound at the emission wavelength of the acceptor compound is small, the concentration of the donor compound can be increased to enhance absorption of excitation light.

[Other Component than donor compound]

The photon upconversion composition of the present invention can further contain any other component. The other component includes a solvent capable of dissolving the naphthalene compound substituted with a specific group-containing substituent (acceptor compound) and the donor compound, a matrix material such as a polymer capable of keeping these compounds dispersed in a solid, and an additive such as a surfactant.

The solvent and the polymer can be appropriately selected from known ones for use herein. For example, the solvent includes dimethylformamide, tetrahydrofuran, chloroform and a mixed solvent thereof. The polymer includes polystyrene, poly(alkyl methacrylate), poly(alkyl acrylate), poly(N-alkylacrylamide), and polyvinyl alcohol. In addition, bioplastics are also usable. Bioplastics contain regenerable organic resources-derived substances as raw materials and chemically or biologically synthesized ones can be appropriately selected and used. For example, cellulose and protein can be employed, and a biodegradable biopolymer is especially preferably employed. In the present invention, two or more kinds of these can be used as mixed. The polymer can have a glass transition temperature of room temperature (25°C) or higher, or the glass transition temperature thereof can be lower than room temperature. In the case where a polymer having a glass transition temperature of room temperature or higher is used as a matrix material, a film of the composition is stiff, in which therefore molecular diffusion occurs little so that the energy transfer between compound molecules is essentially performed by energy diffusion. In the case where a polymer having a glass transition temperature of lower than room temperature is used as a matrix material, a film of the composition is soft, in which therefore energy transfer can be attained by molecular diffusion. The glass transition temperature of the polymer can be measured with a differential scanning calorimeter. The method of dispersing the naphthalene compound and a donor compound of the present invention in a polymer is not specifically limited. For example, the compounds can be dispersed via a step of dissolving them in a common solvent followed by mixing, or a solution prepared by dissolving the naphthalene compound and a donor compound of the present invention in a nonvolatile solvent can be dispersed in the polymer. The nonvolatile solvent can be a nonvolatile liquid that well dissolve the compounds, and can be appropriately selected from surfactants (e.g., Triton$^R$ X-100), low-molecular-weight organic solvents (e.g., hexadecane) or the like for use herein.

When the photon upconversion composition contains a solvent, the proportion of the naphthalene compound relative to the total amount of the composition is preferably 0.005 to 5% by weight, more preferably 0.05 to 5% by weight, even more preferably 0.5 to 1% by weight.

When the photon upconversion composition contains a polymer, the proportion of the naphthalene compound relative to the total amount of the composition is preferably 0.1 to 80% by weight, more preferably 1 to 80% by weight, even more preferably 10 to 50% by weight.

[Usefulness of photon upconversion composition]

The photon upconversion composition of the present invention can efficiently convert a radiation light such as a visible light into a UV light and emit it, at a lower excitation light intensity. In addition, the photon upconversion composition of the present invention is characterized by having an especially low threshold excitation intensity $I_{th}$. Consequently, the photon upconversion mechanism of the composition can act even with a weak light such as sunlight or indoor lighting to emit a UV light as a UC light. Accordingly, the photon upconversion composition of the present invention is useful as a creation source for a UV light and can effectively contribute to efficiency improvement in various sites utilizing a UV light.

For example, when a film of the photon upconversion composition of the present invention is applied to a transparent substance such as a transparent glass sheet or a transparent resin board and exposed to sunlight, a UV light can be obtained at low cost. In the case where the resultant UV light is used for activation of photocatalysts, the photocatalysts can be made to act at good energy efficiency, and the efficiency in artificial photosynthesis or the like utilizing the photocatalysts can be increased. For example, hydrogen can be produced from water and sunlight by the action of photocatalysts, which, therefore greatly contribute toward improvement of fuel efficiency for fuel cell vehicles in which the fuel cells generate power by the use of hydrogen. The transparent substance to which the film is applied

may be any substance capable of transmitting at least a part of a UC light (preferably 10% or more, more preferably 50% or more, even more preferably 90% or more, especially preferably 99% or more), and especially can be a substance capable of transmitting at least a part of a UC light in a wavelength region to be utilized. In the case where the film is applied to a transparent substance, it can be directly stuck to the surface of a transparent substance, or can be laminated on the surface of a transparent substance, or can be detachably layered on the surface of a transparent substance. Also the film can be sandwiched between two transparent substances. Further, in the case where a collective lens is selected as a transparent substance, and where the film is applied thereto, a UC light can be collected in a specific spot to attain further more efficient utilization of energy.

It has been medically clarified that a UV light (ultraviolet light) falling within a range of 360 to 400 nm has a myopia-suppressing effect. Therefore, a film capable of emitting a UC light containing a UV light falling within a range of 360 to 400 nm can be used for myopia suppression. For example, by applying such a film to windowpanes, glasses, goggles, contact lenses and the like, the sunlight or indoor light impinging on the film is converted into a UV light, and can effectively exhibit myopia suppression for humans (especially children and students) and animals. Further by applying the film to LED lights, smartphones and displays of personal computers and televisions, the illumination light can be converted into a UV light to effectively exhibit myopia suppression for operators. The photon upconversion composition of the present invention is useful also as a film to be a creation source for such a UV light falling within a range of 360 to 400 nm. Products capable of exhibiting a myopia-suppressing effect can be produced by applying such a film to a transparent material, or can be produced by mixing the naphthalene compound of the present invention with a transparent material such as a transparent resin to produce transparent products such as windowpanes, glasses, goggles, contact lenses and transparent sheets. In that manner, the photon upconversion composition of the present invention is applicable to a wide range of transparent products such as films, lenses and transparent sheets, and are useful for production of myopia-suppressing transparent products. The word "transparent" as referred to herein means that the substance can transmit at least a light falling within a range of 360 nm to 400 nm and a light necessary for causing photon upconversion for the composition of the present invention.

<Compound represented by general formula (1')>

Of the naphthalene compound for use in the present invention, the compound represented by the following general formula (1) is a novel compound.

General Formula (1')

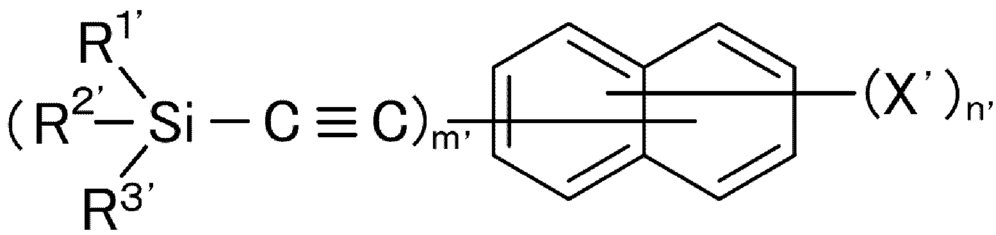

In the general formula (1'), $R^{1'}$, $R^{2'}$ and $R^{3'}$ each independently represent a substituted or unsubstituted alkyl group, the total carbon number of $R^{1'}$, $R^{2'}$ and $R^{3'}$ is 6 or more, X' represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom, m' represents an integer of any of 1 to 8, n' represents an integer of any of 0 to 7, and m'+n' is an integer of any of 1 to 8.

Regarding the description, the preferred range and the specific examples of $R^{1'}$, $R^{2'}$ and $R^{3'}$, and X', m' and n' in the general formula (1'), reference can be made to the description relating to $R^1$, $R^2$ and $R^3$, and X, m and n in the general formula (1). However, the total carbon number of $R^{1'}$, $R^{2'}$ and $R^{3'}$ is 6 or more.

<Synthesis Method for Compound represented by general formula (1')>

The compound represented by the general formula (1') can be synthesized by combining known reactions. For example, a compound where the 1-position and the 4-position of the naphthalene ring each are a substituted or unsubstituted trialkylsilylethynyl group can be synthesized by reacting the following two compounds.

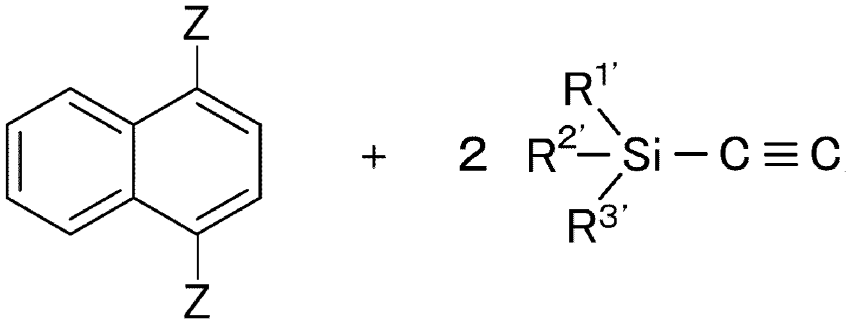

Regarding the description of $R^{1'}$ to $R^{3'}$ in the above reaction formula, reference can be made to the corresponding description of the general formula (1'). Z represents a halogen atom, including a fluorine atom, a chlorine atom, a bromine atom or an iodine atom, and is preferably a chlorine atom, a bromine atom or an iodine atom.

To the above reaction, applied is a known coupling reaction, for which any known reaction conditions can be appropriately selected and used. For the details of the above reaction, reference can be made to Synthesis Examples given hereinunder. The compound represented by the general formula (1') can also be synthesized by combining any other known synthesis reactions.

<Film>

Next, the film of the present invention is described.

The film of the present invention contains the photon upconversion composition of the present invention.

Regarding the description of the photon upconversion composition of the present invention, reference can be made to the description in the section of <Photon Upconversion Composition> given hereinabove.

Embodiments of the film of the present invention include those produced by forming the photon upconversion composition into films, and those produced by forming a layer of the photon upconversion composition on a substrate. In the latter embodiment, the film can be formed of the layered photon upconversion composition alone, or the film can be composed of a layered photon upconversion composition and a substrate. The method of forming the photon upconversion composition into a film is not specifically limited, and a known film molding method such as calendering, extrusion or inflation molding can be employed. The method of layering the photon upconversion composition is not also specifically limited, for example, any of a dry process or a wet process is employable. The substrate is not also specifically limited, and for example, glass, transparent plastics, quartz and silicon can be employed.

From the viewpoint of facilitating molding or layering film formation to control the mechanical properties of films, the photon upconversion composition for films preferably contains a naphthalene compound substituted with a specific group-containing substituent and a polymer, or contains a naphthalene compound substituted with a specific group-containing substituent (acceptor compound), a donor compound and a polymer. The polymer for use in the composition can be appropriately selected from known polymers for films and used, for example, including a polystyrene, a poly(alkyl acrylate), a ;poly(alkyl methacrylate), a poly(N-alkylacrylamide), an epoxy resin, and a polyvinyl alcohol. In the case where a hydrophilic polymer such as a polyvinyl alcohol is used, preferably a surfactant is added to the composition for improving the dispersibility of the compounds.

The film of the photon upconversion composition can be a single-layered film or a multilayered film. When the film is a multilayered film, preferably, at least the adjacent layers differ in the composition.

As an embodiment of the film of the present invention, also mentioned is an impregnated film produced by impregnating a porous film with a liquid material of the naphthalene compound substituted with a specific group-containing substituent. As the liquid material, employable is a solution of the naphthalene compound substituted with a specific group-containing substituent, or a solution of the naphthalene compound substituted with a specific group-containing substituent (acceptor compound) and a donor compound, a melt liquid prepared by heating and melting the naphthalene compound substituted with a specific group-containing substituent, or a melt liquid prepared by heating a mixture of the naphthalene compound substituted with a specific group-containing substituent (acceptor compound) and a donor compound to be in a molten state. The melt liquid prepared by heating the mixture to be in a molten state can be one where a solid donor compound is dispersed in a melt liquid of the acceptor compound, or one where both the acceptor compound and a donor compound are in a molten state. Of those, a film prepared by impregnation with a solution is preferred as readily obtaining stable products. As the porous film, employable is an organic porous film where an organic filler is dispersed in a matrix polymer. As commercial products, a microporous film (by 3M Company) is employable. In addition, a PTFE membrane film (by Tokyo Garasu Kikai Co., Ltd.) and a polyethylene porous sheet (by Flon Chemical Co., Ltd.) are also employable.

The thickness of the film of the present invention is preferably 10 nm to 1cm, more preferably 100 nm to 500 μm, even more preferably 1 μm to 100 μm. In the case where the layer of the photon upconversion composition is a multilayer, the total thickness thereof falls within the above-mentioned range.

The film of the present invention can be composed of a film alone containing the photon upconversion composition of the present invention, or can contain any other layer or film. The other film includes an oxygen barrier film. In the case where an oxygen barrier film is used, preferably, the film containing the photon upconversion composition is entirely covered and sealed up with the oxygen barrier film. As the oxygen barrier film, usable is a polyvinyl alcohol film, or a film of a copolymer of a vinyl alcohol and any other monomer. For example, preferred is a film of an ethylene-vinyl alcohol copolymer.

<Method for converting visible light into UV light>

Next described is a method of converting a visible light into a UV light.

The method for converting a visible light into a UV light of the present invention is a method of applying a visible light to the photon upconversion composition of the present invention or to the film of the present invention to convert it into a UV light.

Regarding the description of the photon upconversion composition of the present invention and the definition of visible light and UV light, reference can be made to the description in the section of <Photon Upconversion Composition> given hereinabove, and regarding the description of the film of the present invention, reference can be made to the description in the section of <Film> also given hereinabove.

For converting a visible light into a UV light according to the method of the present invention, first, the photon upconversion composition of the present invention or the film of the present invention is irradiated with a visible light. The visible light to be applied can be a single light having an emission maximum wavelength at a specific wavelength in a visible region, or can be a composite light synthesized from plural visible lights differing in the emission maximum wavelength, or can also be a composite light synthesized from lights of continuous wavelengths in a visible region. Also the light to be applied to the composition can contain, in addition to a visible light, any other light than a visible light. Examples of the irradiation source include sunlight, LED, Xe lamp, and laser. The irradiation intensity is preferably 0.1 to 1000 $mW/cm^2$, more preferably 0.5 to 100 $mW/cm^2$, even more preferably 1 to 50 $mW/cm^2$. The irradiation time is not specifically limited, and can be, for example, 1 minute or longer.

In the method of the present invention, the photon upconversion composition of the present invention or the film of the present invention is made to emit a UV light as a UC light by irradiation with such a visible light. The emission can be recognized within a UV region (in a wavelength range of 200 to 400 nm), but preferably the UV emission is recognized within a range of 360 to 400 nm. Here, "the emission is recognized within a range of 360 to 400 nm" means that the emission maximum wavelength of the UV light falls within a range of 360 to 400 nm or the emission maximum wavelength thereof is around a range of 360 to 400 nm, and 50% or more of the emission intensity at the emission maximum wavelength is recognized in a range of 360 nm to 400 nm. As described above, a UV light falling within a range of 360 to 400 nm exhibits a myopia-suppressing effect, and therefore the UV light obtained in the embodiment of emitting a UV light within the range can be effectively utilized for myopia suppression.

Examples

The features of the present invention will be described more specifically with reference to Examples and Comparative Examples given below. The materials, the amounts used, the proportions, the processes, the procedures and the like shown below may be appropriately modified unless they deviate from the substance of the invention. Accordingly, the scope of the invention should not be construed as being limited to the specific examples shown below. Hereinunder a spectrophotometer (V-780 by JASCO Corporation) was used for absorption spectrometry, and a fluorospectrophotometer (FP-8300 by JASCO Corporation) was used for emission spectrometry. The UC efficiency $\eta_{UC}$ was calculated according to a relative method using Coumarin 6 as a standard substance. In the relative method, used were the fluorescence quantum yield of a Coumarin 6 solution, the absorbance and the emission spectrum of each solution targeted for measurement, the excitation light intensity in measurement and the refractive index of the solvent used, and in emission spectrometry, used was a multichannel spectrometer (MCPD-9800 by Otsuka Electronics Co., Ltd.)

[Acceptor Compound and Donor Compound used in Example]

Hereinunder the acceptor compounds and the donor compounds used in Examples are shown.

Acceptor Compounds

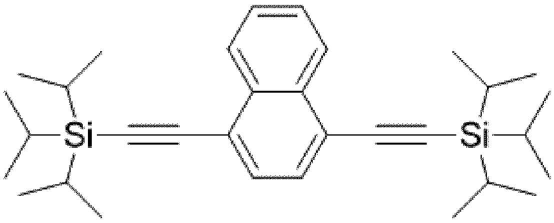

Compound A1

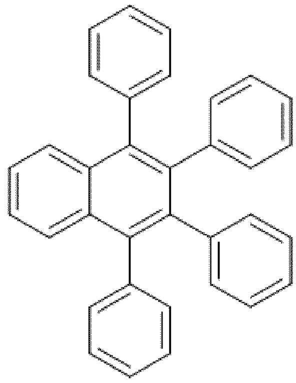

Compound A2

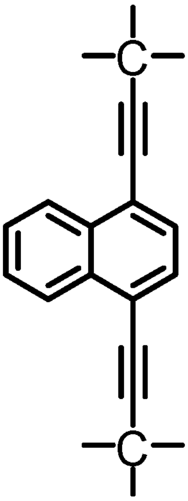

Compound A3

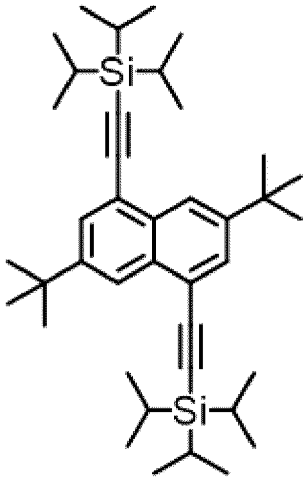

Compound A4

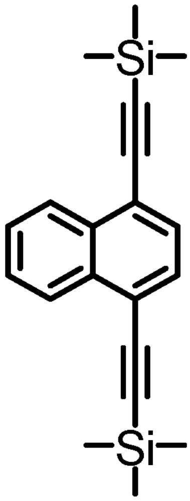

Compound A5

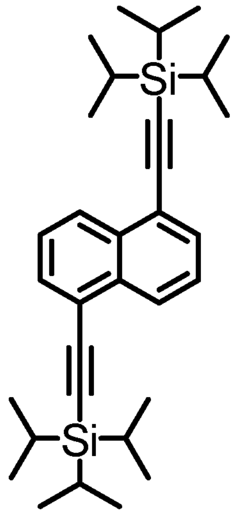

Compound A6

Donor Compounds

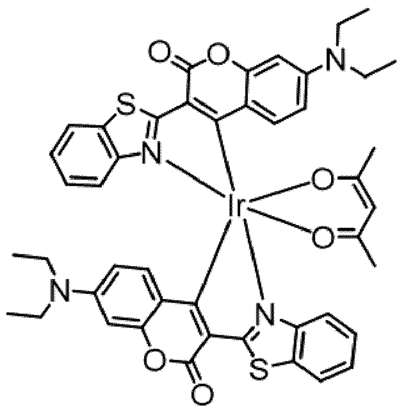

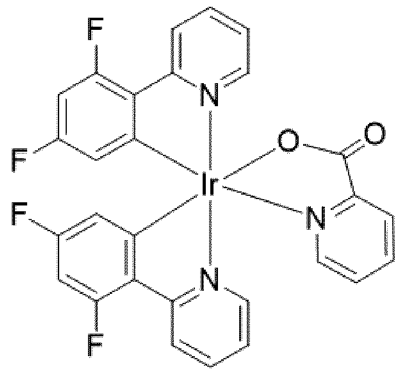

Compound D1 Compound D2

Table 1 shows the absorption maximum wavelength of main absorption peaks of these acceptor compounds and donor compounds, as well as the emission maximum wavelength, the lowest excited singlet energy level $S_1$ and the lowest excited triplet energy level $T_1$ thereof. $S_1$ of the acceptor is a value calculated from the emission maximum wavelength, and $S_1$ of the donor is a value calculated from the absorption maximum wavelength. $T_1$ of the acceptor is a value calculated in density functional theory (DFT), and $T_1$ of the donor is a value calculated from the emission maximum wavelength.

[Table 1]

| | Absorption Maximum Wavelength of main absorption peaks (nm) | Emission Maximum Wavelength (nm) | $S_1$ (eV) | $T_1$ (eV) |
|---|---|---|---|---|
| Compound A1 | 350 nm | 356 nm | 3.48 | 2.10 |
| Compound A2 | (Note) | 360 nm | 3.44 | 2.59 |
| Compound A3 | 341 nm | 350 nm | 3.54 | 2.17 |
| Compound A4 | 348 nm | 365 nm | 3.40 | 2.32 |
| Compound A5 | 348 nm | 356 nm | 3.48 | 2.08 |
| Compound A6 | 343 nm | 367 nm | 3.38 | 2.24 |
| Compound D1 | 445 nm, 474 nm | 567 nm | 2.62 | 2.19 |
| Compound D2 | 381 nm | 477 nm | 3.25 | 2.60 |
| (Note) Unmeasured, but presence of absorption in a UV region was confirmed. | | | | |

(Synthesis Example 1) Synthesis of Compound A1

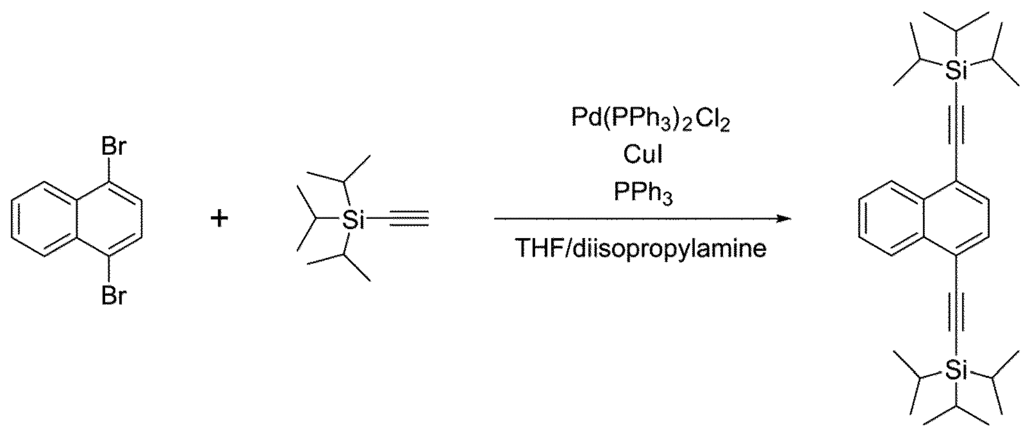


1,4-Dibromonaphthalene (1.44 g, 5.03 mmol), bis(triphenylphosphine)palladium(II) dichloride (80.6 mg, 0.115 mmol), copper iodide (60.4 mg, 0.317 mmol) and triphenyl phosphine (82.0 mg, 0.313 mmol) were dissolved in tetrahydrofuran (40 mL) in a nitrogen atmosphere, and then diisopropylamine (36 mL) was added thereto. The solution was heated up to 100°C, then triisopropylsilylacetylene (3.52 g, 19.3 mmol) was dropwise added, and stirred for 10 hours. The reaction liquid was cooled down to room temperature, then tetrahydrofuran and diisopropylamine were evaporated away under reduced pressure, and the residue was extracted out in chloroform. The chloroform solution was dried with sodium sulfate, and then chloroform was evaporated away under reduced pressure. The resultant crude product was purified through column chromatography using n-hexane as a developing solvent, and recrystallized with methanol. According to the above process, the intended Compound A1 was obtained at an actual yield of 1.30 g and a percent yield of 53%.

$^1$H-NMR (400 MHz, $CDCl_3$, TMS): $\delta$ 8.41-8.38 (m, 2H), 7.63 (s, 2H), 7.61-7.58 (m, 2H), 1.20 (s, 36H), 1.19 (s, 6H).
$^{13}$C-NMR (101 MHz, $CDCl_3$, TMS): $\delta$ 133.22, 130.14, 127.17, 126.63, 121.79, 104.77, 97.79, 18.77, 11.41.
Elemental analysis, calculated for $C_{32}H_{48}Si_2$: C 78.61 H 9.90; found C 78.63 H 9.94.

(Synthesis Example 2) Synthesis of Compound A3

1,4-Dibromonaphthalene (1.45 g, 5.06 mmol), bis(triphenylphosphine)palladium(II) dichloride (75.1 mg, 0.107 mmol), copper iodide (83.7 mg, 0.439 mmol) and triphenyl phosphine (96.5 mg, 0.368 mmol) were dissolved in tetrahydrofuran (40 mL) in a nitrogen atmosphere, and then diisopropylamine (40 mL) was added. The solution was heated up to 100°C, and 3,3-dimethyl-1-butyne (1.30 g, 15.8 mmol) was dropwise added thereto. The solution after the reaction was cooled down to room temperature, then tetrahydrofuran and diisopropylamine were evaporated away under reduced pressure, and the residue was extracted out in dichloromethane. Subsequently, silica gel column chromatography using n-hexane as a developing solvent and recrystallization with methanol were performed. Further, purification by sublimation and recrystallization with methanol were performed. According to the above process, the intended Compound A3 was obtained at an actual yield of 126 mg and a percent yield of 8%.

$^1$H-NMR (400 MHz, $CDCl_3$, TMS): $\delta$ 8.32-8.28 (m, 2H), 7.58-7.54 (m, 2H), 7.51 (s, 2H), 1.42 (s, 18H).
Elemental analysis, calculated for $C_{22}H_{24}$: C 91.61 H 8.39; found C 91.40 H 8.34.

(Synthesis Example 3) Synthesis of Compound A4

2,6-Di-tert-butylnaphthalene (1.21 g, 5.03 mmol) was put into a three-neck flask, and kept in a nitrogen atmosphere. With cooling at -13°C, 10 mL of dichloromethane was added, and then bromine (0.57 mL) was added. After stirred for 1 hour, this was statically kept in an atmosphere at -25°C. After 3 days, this was stirred at room temperature for 8 hours, and then again statically left at -25°C. After 6 days, this was quenched with an aqueous sodium sulfite solution, and then extracted with chloroform. The solvent was evaporated away to obtain a precursor of A4 at an actual yield of 1.94 g and a percent yield of 96%.

The resultant precursor (1.20 g, 3.01 mmol), bis(triphenylphosphine)palladium(II) dichloride (50.1 mg, 0.0714 mmol), copper iodide (38.3 mg, 0.201 mmol), and triphenylphosphine (49.8 mg, 0.190 mmol) were put into a three-neck flask, and in a nitrogen atmosphere, tetrahydrofuran and diisopropylamine were added each in an amount of 30 mL. The solution was heated up to 100°C, and triisopropylsilylacetylene (1.44 g, 7.90 mmol) was dropwise added. After stirred overnight, this as restored to room temperature, the solvent was evaporated away, and the residue was extracted

in dichloromethane. The resultant crude product was purified through silica gel column chromatography using n-hexane as a developing solvent, gel permeation chromatography using chloroform, and reprecipitation using methanol and n-hexane. According to the above process, the intended Compound A4 was obtained at an actual yield of 324 mg and a percent yield of 18%.

$^1$H-NMR (400 MHz, $CDCl_3$, TMS): $\delta$ 8.33-8.32 (d, 2H), 7.78-7.77 (d, 2H), 1.41 (s, 18H), 1.22-1.21 (42H). Elemental analysis, calculated for $C_{40}H_{64}Si_2$: C 79.92 H 10.73; found C 79.66 H 10.68.

(Synthesis Example 4) Synthesis of Compound A5

1,4-Dibromonaphthalene (1.52 g, 5.33 mmol), bis(triphenylphosphine)palladium(II) dichloride (79.1 mg, 0.113 mmol), copper iodide (79.7 mg, 0.418 mmol) and triphenyl phosphine (109.7 mg, 0.418 mmol) were dissolved in tetrahydrofuran (39 mL) and diisopropylamine (38 mL) in a nitrogen atmosphere. The solution was heated up to 100°C, and trimethylsilylacetylene (1.52 g, 15.5 mmol) was dropwise added. After the reaction, this was separated in a mode of liquid separation using diethyl ether and an aqueous ammonium chloride solution, and the organic phase was extracted in chloroform. The organic phase was further extracted in dichloromethane, then dried with anhydrous sodium sulfate, and dichloromethane was evaporated away under reduced pressure. The resultant crude product was purified through silica gel column chromatography using n-hexane and chloroform as developing solvents, and recrystallized with methanol. According to the above process, the intended Compound A5 was obtained at an actual yield of 176 mg and a percent yield of 10%.
$^1$H-NMR (400 MHz, $CDCl_3$, TMS): $\delta$ 8.35-8.31 (m, 2H), 7.62-7.58 (4H), 0.33 (s, 18H). Elemental analysis, calculated for $C_{20}H_{24}Si_2$: C 74.93 H 7.55; found C 74.65 H 7.41.

(Synthesis Example 5) Synthesis of Compound A6

1,5-Dibromonaphthalene (0.80 g, 2.8 mmol), bis(triphenylphosphine)palladium(II) dichloride (44 mg, 0.063 mmol), copper iodide (33 mg, 0.17 mmol) and triphenyl phosphine (45 mg, 0.17 mmol) were dissolved in tetrahydrofuran (28 ml) in a nitrogen atmosphere, and then diisopropylamine (29 mL) was added. The solution was heated up to 100°C, and triisopropylsilylacetylene (1.1 g, 6.0 mmol) was dropwise added. After the reaction, tetrahydrofuran and diisopropylamine were evaporated away under reduced pressure, and the residue was extracted in dichloromethane. The dichloromethane solution was dried with anhydrous sodium sulfate, and then dichloromethane was evaporated away under reduced pressure. The resultant crude product was purified through silica gel column chromatography using n-hexane as a developing solvent, and recrystallized with methanol. According to the above process, the intended Compound A6 was obtained at an actual yield of 1.15 g and a percent yield of 83%.

$^1$H-NMR (400 MHz, $CDCl_3$, TMS): $\delta$ 8.39-8.37 (2H), 7.76-7.74 (2H), 7.52-7.48 (2H), 1.20-1.18 (42H). Elemental analysis, calculated for $C_{32}H_{48}Si_2$: C 78.61 H 9.90; found C 78.39 H 9.86.

(Example 1) Preparation and Evaluation of Photon Upconversion Composition containing Compound A1 and Compound D1

[1] Preparation of Solution containing Compound A1 and Compound D1

In a glove box in an Ar atmosphere, Compound A1 and Compound D1 were dissolved in tetrahydrofuran to prepare a tetrahydrofuran solution (composition 1). Here, the concentration of Compound A1 was 10 mM and the concentration of Compound D1 was 100 μM.
A tetrahydrofuran solution (composition 2) of Compound A1 and Compound D1 was prepared in the same manner except that the concentration of Compound D1 was changed to 300 μM.
The deaerated composition 1 was irradiated with a 445-nm excitation light to measure an emission spectrum, and the results are shown in Fig. 2. Plural emission spectra shown in Fig. 2 were measured by stepwise changing the radiation intensity of the excitation light to fall within a range of 0.030 mW/cm$^2$ to 5.8 W/cm$^2$. The emission peak appearing in the UV region of the emission spectra were plotted in Fig. 3 (double logarithmic graph) in which the intensity at the emission maximum wavelength (peak intensity) is on the vertical axis, and the excitation light intensity was on the horizontal axis. Also the composition 2 was irradiated with a 445-nm excitation light under the same condition to measure emission spectra, and the emission peak intensity observed in the UV region was plotted in the double logarithmic graph of Fig. 3. In Fig. 3, "100 μM" and "300 μM" each are the concentration of Compound D1, corresponding to the composition 1 and the composition 2. "1 sun" indicates a light intensity 1.4 mW/cm$^2$ contained in a range of 440 to 450 nm of AM 1.5 sunlight, and "$I_{th}$" indicates a threshold excitation intensity.

As shown in Fig. 2, a high emission peak observed in the UV region of the emission spectrum of the composition 1. The emission peak appeared in excitation light irradiation at a wavelength at which Compound D1 exhibits strong absorption, and is an emission peak overlapping with the fluorescence peak of Compound A1. Accordingly, the emission peak can be said to be derived from fluorescence radiation from Compound A1 with the UC emission mechanism of Fig. 1 (UC emission).

Also as shown in Fig. 3, when the emission peak intensity observed in the UV region is plotted and fitted on the double logarithmic graph, there appear a first line with an inclination 2, a second line with an inclination 1 and a refraction point where the first line and the second line intersect. Here, in the region corresponding to the first line, there are a few triplet excitons since the excitation light intensity is low, which indicates that the probability of triplet-triplet annihilation is smaller than the probability of nonradiative deactivation of triplet excitons. Namely, the first line reflects a state where the number of the triplet excitons to undergo triplet-triplet annihilation is smaller than the number of the triplet excitons to undergo nonradiative deactivation. On the other hand, the refraction point reflects a state where the number of the triplet excitons to undergo triplet-triplet annihilation is equal to the number of the triplet excitons to undergo nonradiative deactivation, and further indicates that by increasing the excitation light intensity to be more than the excitation light intensity at the refraction point (threshold excitation intensity $I_{th}$), the photon upconversion emission by triplet-triplet annihilation can be predominant over the nonradiative deactivation of triplet excitons. In other words, when the threshold excitation intensity $I_{th}$ is smaller, UC emission can occur more efficiently at a lower excitation light intensity. In this respect, Fig. 3 is referred to, in which the refraction point $I_{th}$ of the composition 1 is 2.3 mW/cm$^2$, and the refraction point $I_{th}$ of the composition 2 is 1.1 mW/cm$^2$ and both are extremely small values. From this, it is known that the photon upconversion composition of the present invention can attain high UC efficiency exceeding $I_{th}$ thereof even with weak irradiation light such as sunlight or indoor light.

Next, the deaerated composition 1 was actually irradiated with AM 1.5 simulated solar light from a solar simulator equipped with a 450-nm long pass filter to measure the emission spectrum thereof. The measurement results are shown in Fig. 4. Also the composition 1 was radiated with a light from a desktop LED light to measure the emission spectrum, and the results are shown in Fig. 5. For comparison, Compound A1 alone was dissolved in a tetrahydrofuran solution at a concentration of 10 mM (solution of Compound A1), and the solution was also irradiated with a simulated solar light and an LED light under the same condition as above to measure the emission spectra. The results are additionally shown in Figs. 4 and 5.

In the measurement, the radiation intensity of the simulated solar light applied to the composition 1 was 1.4 mW/cm$^2$ at 445 nm $\pm$ 5 nm, and the irradiation intensity of the desktop LED light was 1 mW/cm$^2$ at a wavelength region of less than 500 nm. The long-pass filter put on the solar simulator is to cut off the light falling within a short wavelength region to thereby prevent Compound A1 from being directly excited by the simulated solar light.

As shown in Figs. 4 and 5, in any case of irradiation with a simulated solar light or an LED light, a large emission peak observed in the UV region of the emission spectrum of the composition 1. On the other hand, the emission spectrum of the solution of Compound A1 alone did not give an emission peak in the UV region like that from the composition 1. From the results, it is known that the emission peak in the UV region given by the composition 1 is photon upconversion emission by triplet-triplet annihilation of Compound A1 utilizing the triplet excitation energy of Compound D1. This confirms that the photon upconversion composition of the present invention can provide photon upconversion emission by triplet-triplet annihilation even with a sunlight or an indoor light such as a desktop LED light.

[2] Production of Film containing Compound A1 and Compound D1

Next, films containing Compound A1 and Compound D1 were produced as follows.

(Production of Film 1)

In a glove box in an Ar atmosphere, polystyrene, Compound A1 and Compound D1 were dissolved in chloroform to prepare a solution. Here, the blending amount of Compound A1 was 30% by weight relative to the total amount of the polymer, Compound A1 and Compound D1, and the ratio of Compound A1 to Compound D1 was Compound A1/Compound D1 = 100/1 (by mol). The solution was applied onto a glass substrate according to a drop-casting method to form Film 1, another glass substrate was put thereon, and the gap between the two glass substrate was sealed up with an epoxy adhesive (Araldite$^R$). In that manner, a sealed structure of glass substrate/film/glass substrate was produced.

(Production of Films 2 to 4)

Films 2 to 4 were produced in the same manner as that for Film 1, except that the polymer shown in Table 2 was used in place of polystyrene, and the blending amount of Compound A1 (proportion relative to the total amount of

the polymer, Compound A1 and Compound D1) was 23% by weight in Films 2 and 3, and was 7.6% by weight in Film 4. Glass substrates were applied to these and sealed up with Araldite$^R$ to produce sealed structures.

(Production of Film 5)

Film 5 was produced in the same manner as that for Film 1, except that as a solution used for film formation, the solution prepared by dissolving Compound A1 and Compound D1 in chloroform in a molar ratio of Compound A1/Compound D1 = 100/1 was used. Glass substrates were applied to this and sealed up with Araldite$^R$ to produce a sealed structure.

(Production of Film 6)

In an atmosphere, a surfactant (Pluronic F127), Compound A1 and Compound D1 were dissolved in chloroform in a molar ratio of Pluronic F127/Compound A1/Compound D1 = 960/100/1. The solution was dried, and an aqueous solution of polyvinyl alcohol was added to the residue to prepare a liquid material. At that time, the proportion of Pluronic F127 was 11% by weight relative to the total amount of Pluronic F127, Compound A1, Compound D1 and polyvinyl alcohol. Film 6 was formed of the liquid material according to a drop-casting method.

(Production of Film 7)

In an atmosphere, Compound A1 and Compound D1 were dissolved in a surfactant (TX-100), and mixed with an aqueous solution of polyvinyl alcohol to prepare a liquid material. At that time, the concentration of Compound A1 in TX-100 was 10 mM, and the concentration of Compound D1 in TX-100 was 100 μM. The proportion of TX-100 was 1% by weight relative to the total amount of Compound A1, Compound D1, polyvinyl alcohol and TX-100. Film 7 was formed of the liquid material according to a drop-casting method.

[Table 2]

| | Acceptor Compound | Donor Compound | Surfactant | Polymer |
|---|---|---|---|---|
| Film 1 | Compound A1 | Compound D1 | N/A | Polystyrene |
| Film 2 | Compound A1 | Compound D1 | N/A | Poly(methyl methacrylate) |
| Film 3 | Compound A1 | Compound D1 | N/A | Poly(N-isopropylacrylamide) |
| Film 4 | Compound A1 | Compound D1 | N/A | Poly(butyl acrylate) |
| Film 5 | Compound A1 | Compound D1 | N/A | N/A |
| Film 6 | Compound A1 | Compound D1 | Pluronic F127 | Polyvinyl alcohol |
| Film 7 | Compound A1 | Compound D1 | TX-100 | Polyvinyl alcohol |

The structural formulae of the polymers used in Films 1 to 4, 6 and 7, and those of the surfactants used in Films 6 and 7 are shown below.

Polymer

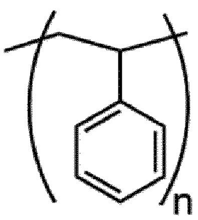

ポリスチレン

n:約 1900, Tg:100℃

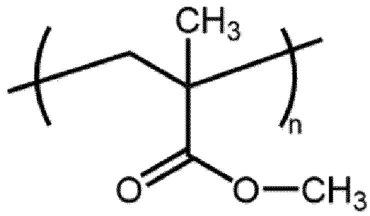

ポリメチルメタクリレート

n:約 1200, Tg:105℃

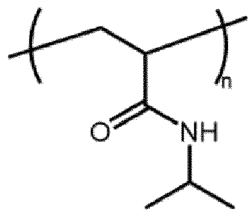

ポリ(N-イソプロピルアクリルアミド)

n:約 350, Tg:130℃

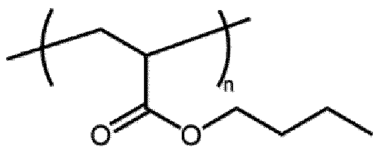

ポリ(ブチルアクリレート)

n:約 160, Tg:-49℃

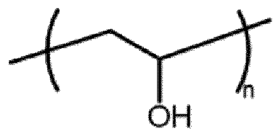

ポリビニルアルコール

n:約 1000, Tg:80℃

| | | |
|---|---|---|
| Polystyrene | Polymethyl methacrylate | Poly(N-isopropylacrylamide) |
| n: about 1900, Tg: 100°C | n: about 1200, Tg: 105°C | n: about 350, Tg: 130°C |
| Poly(butyl acrylate) | Polyvinyl alcohol | |
| n: about 160, Tg: -49°C | n: about 1000, Tg: 80°C | |

Surfactant

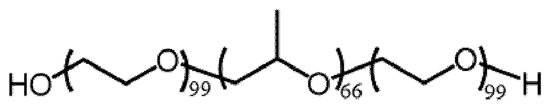

Pluronic F127

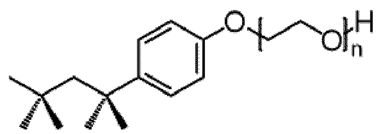

TX-100

UC mission spectra of the films produced are shown in Figs. 6 to 12. In Figs. 6 to 12, the wavelength indicated by $\lambda_{ex}$ is a wavelength of the irradiated excitation light. The excitation light intensity was 450 mW/$cm^2$, and a 425-nm short-pass filter was arranged between the sample and the spectroscope.

As shown in Figs. 6 to 12, an emission peak derived from photon upconversion emission was observed in the UV region in all the emission spectra of Films 1 to 7.

Here, of the polymers used in the present Example, polystyrene, poly(methyl methacrylate) and poly(N-isopropylacrylamide) are polymers having a glass transition temperature of higher than room temperature, and exhibit a hard nature. Consequently, in Films 1 to 3 using these polymers, molecular diffusion did not occur, and it is presumed that the excited triplet energy transfer from Compound D1 to Compound A1 as well as the triplet-triplet annihilation in Compound A1 could be attained via energy diffusion.

On the other hand, poly(butyl acrylate) used in Film 4 is a polymer having a glass transition temperature of lower than room temperature, and exhibits a soft nature. Consequently, in Film 4, it is presumed that the excited triplet energy transfer from and the triplet-triplet annihilation could be attained not only via energy diffusion but also by molecular diffusion of Compound A1 and Compound D1.

Film 5 does not contain a matrix material, and is in a state where the donor compound dispersed in the solid

acceptor compound. Consequently, in Film 5, there did not occur molecular diffusion, and it is considered that the excited triplet energy transfer from Compound D1 to Compound A1 as well as the triplet-triplet annihilation in Compound A1 would be attained via energy diffusion.

The polyvinyl alcohol used in Films 6 and 7 is a polymer having a glass transition temperature of higher than room temperature and has an oxygen barrier performance. In Films 5 and 6, Compound A1 and Compound D1 were dissolved in the soft liquid (surfactant) in the solid polyvinyl alcohol, and therefore it is presumed that the excited triplet energy transfer from and the triplet-triplet annihilation could be attained not only via energy diffusion but also by molecular diffusion of Compound A1 and Compound D1.

(Production of Film 8)

In a glove box in an Ar atmosphere, Compound A1 and Compound D1 were dissolved in hexyl benzoate to prepare a solution, and a microporous film (porous film formed of polypropylene and an organic filler, by 3M Company) was immersed in the solution to produce a film impregnated with the solution of Compound A1 and Compound D1 (Film 8). Here, the concentration of Compound A1 in the solution was 10 mM, and the concentration of Compound D1 therein was 100 $\mu$M. A 15-$\mu$m thick film of an ethylene-vinyl alcohol copolymer (EVOH film) was arranged above and below the produced Film 8, and the periphery of the film was heat-sealed to obtain a sealed structure of EVOH film/Film 8/EVOH film. Here, the EVOH film is an "Eval" film (ethylene 32 mol%, grade: EF-F) produced by Kuraray Co., Ltd., and was used as an oxygen barrier film.

Fig. 13 shows a UC emission spectrum of the produced Film 8. In measurement, the wavelength $\lambda_{ex}$ of the excitation light was 460 nm, and a 400-nm long wavelength cut filter was arranged between the sample and the spectroscope. The long wavelength cut filter was used also in the measurement of Figs. 14 and 17 mentioned below. The range of 350 to 400 nm of each UC emission spectrum was integrated to calculate the emission intensity of Film 8. In an unfolded state, the emission intensity was 112.7 $\mu W/cm^2$, and was 211.0 $\mu W/cm^2$, in a two-folded state. It is known that the myopia-suppressing effect of a 360 to 400-nm UV light can be exhibited at 50 $\mu W/cm^2$ or more, and from the measurement results, it is confirmed that the film of the present invention is effective for myopia suppression.

(Production of Film 9)

In a glove box in an Ar atmosphere, a mixture of Compound A1 and Compound D1 was heated to melt Compound A1, thereby obtaining a liquid material in a state where Compound D1 dissolved in the melt of Compound A1. Here, the molar ratio of Compound A1 to Compound D1 (Compound A1/Compound D1) was 220/1. A microporous film (by 3M Company) was immersed in the liquid material to produce a Compound A1 and Compound D1-impregnated film (Film 9). Film 9 was put between two glass substrates, and the gap thereof was sealed up with an epoxy adhesive (Araldite$^R$) to produce a sealed structure of glass substrate/Film 9/glass substrate.

Of the produced Film 9, UC emission spectra with a 445-nm excitation light were measured with stepwise varying the excitation light intensity within a range of from 6.5 $mW/cm^2$ to 39.2 $W/cm^2$. The results are shown in Fig. 14. The threshold excitation intensity $I_{th}$ of Film 9 derived from the excitation light intensity dependence of the UC emission intensity was 408.9 $mW/cm^2$. This indicates that the film produced by melting a naphthalene compound is also useful as a photon upconversion material.

(Example 2) Preparation and Evaluation of Photon Upconversion Composition containing Compound A2 and Compound D2

In a glove box in an Ar atmosphere, Compound A2 and Compound D2 were dissolved in toluene to prepare a toluene solution (composition 3). Here, the concentration of Compound A2 was 10 mM, and the concentration of Compound D2 was 100 $\mu$M.

Emission having a peak in a range of 360 to 400 nm was observed, and photon upconversion was recognized.

(Example 3) Preparation and Evaluation of Photon Upconversion Composition containing Compound A3 and Compound D1

In a glove box in an Ar atmosphere, Compound A3 and Compound D1 were dissolved in tetrahydrofuran to prepare a tetrahydrofuran solution (composition 4). Here, the concentration of Compound A3 was 10 mM, and the concentration of Compound D1 was 100 $\mu$M.

Of the composition 4, UC emission spectra with a 445-nm excitation light were measured with stepwise varying the excitation light intensity within a range of from 0.11 $mW/cm^2$ to 7.4 $W/cm^2$. The results are shown in Fig. 15. The threshold excitation intensity $I_{th}$ of the composition 4 was 7.0 $mW/cm^2$, and the UC efficiency $\eta_{UC}$ thereof was 18.8% at

7.4 $W/cm^2$.

Of the composition 4, time dependence of UC emission after stopping excitation light irradiation was measured, and the results are shown in Fig. 16. As shown in Fig. 16, UC emission of the composition 4 annihilated little even after 30 minutes.

(Example 4) Preparation and Evaluation of Photon Upconversion Composition containing Compound A4 and Compound D1

A tetrahydrofuran solution of Compound A4 and Compound D1 (composition 5) was prepared in the same manner as in Example 3 except that Compound A4 was used in place of Compound A3.

Of the composition 5, UC emission spectrum with a 445-nm excitation light was measured with stepwise varying the excitation light intensity in a range of from 0.12 $mW/cm^2$ to 37.8 $W/cm^2$. The results are shown in Fig. 17. The threshold excitation intensity $I_{th}$ of the composition 5 was 27.5 $mW/cm^2$, and the UC efficiency $\eta_{UC}$ thereof was 13.0% at 3.1 $W/cm^2$.

Also the fluorescence quantum yield of a solution and a crystal of Compound A4 was measured, and both were nearly the same values. When an ordinary fluorescent material is formed into a crystal, the fluorescence quantum yield thereof greatly lowers, but the crystal of Compound A4 had the same fluorescence quantum yield as the solution thereof. It is presumed that owing to the steric hindrance of the t-butyl group that Compound A4 has, a suitable space could be maintained between the crystal molecules. From this, it is known that, when Compound A4 is mixed with a resin such as an epoxy resin, a polystyrene or a polymethyl methacrylate, it can exhibit a high fluorescence quantum yield even at a high concentration, and Compound A4 can be effectively applied as a UV creation source for myopia-suppressing eyeglasses or resin films.

(Example 5) Preparation and Evaluation of Photon Upconversion Composition containing Compound A5 and Compound D1

A tetrahydrofuran solution of Compound A5 and Compound D1 (composition 6) was prepared in the same manner as in Example 3 except that Compound A5 was used in place of Compound A3.

Of the composition 6, UC emission spectrum with a 445-nm excitation light was measured with stepwise varying the excitation light intensity in a range of from 0.66 $mW/cm^2$ to 119 $W/cm^2$. The results are shown in Fig. 18. The threshold excitation intensity $I_{th}$ of the composition 6 was 181.5 $mW/cm^2$, and the UC efficiency $\eta_{UC}$ thereof was 14.2% at 119 $W/cm^2$.

(Example 6) Preparation and Evaluation of Photon Upconversion Composition containing Compound A6 and Compound D1

A tetrahydrofuran solution of Compound A6 and Compound D1 (composition 7) was prepared in the same manner as in Example 3 except that Compound A6 was used in place of Compound A3.

Of the composition 7, UC emission spectrum with a 445-nm excitation light was measured with stepwise varying the excitation light intensity in a range of from 4.1 $mW/cm^2$ to 104 $W/cm^2$. The results are shown in Fig. 19. The threshold excitation intensity $I_{th}$ of the composition 7 was 511 $mW/cm^2$, and the UC efficiency $\eta_{UC}$ thereof was 11.3% at 66.7 $W/cm^2$.

Films 1 to 9 and the films formed of the compositions 3 to 7 were individually adhered to eyeglasses or glass products to measure the emission spectra thereof, which all gave an emission peak in a range of 360 to 400 nm. From this, it is confirmed that the film of the present invention exhibits a photon upconversion effect on actual products.

Industrial Applicability

The photon upconversion composition of the present invention can efficiently convert an excitation light into a UV light at a lower excitation light intensity, and therefore can effectively create a UV light even with a weak light such as sunlight or indoor light. Consequently, the photon upconversion composition of the present invention can greatly contribute toward improving the efficiency of solar devices such as solar cells and photocatalysts. Accordingly, the industrial applicability of the present invention is great.

## Claims

1. A photon upconversion composition containing a naphthalene compound substituted with a substituent containing at least one selected from the group consisting of an alkynyl group, a substituted silyl group, a benzene ring, an heteroaromatic ring, a cyano group and a halogen atom.

2. The photon upconversion composition according to claim 1, wherein the naphthalene compound has a substituted silyl group.

3. The photon upconversion composition according to claim 1 or 2, wherein the naphthalene compound has an alkynyl group.

4. The photon upconversion composition according to any one of claims 1 to 3, wherein the naphthalene compound has a substituted or unsubstituted phenyl group.

5. The photon upconversion composition according to any one of claims 1 to 4, wherein the naphthalene compound has 2 to 4 above-mentioned substituents.

6. The photon upconversion composition according to any one of claims 1 to 5, wherein the naphthalene compound is a 1,4-di-substituted naphthalene.

7. The photon upconversion composition according to claim 1, wherein the naphthalene compound has a structure represented by the following general formula (1):

General Formula (1)

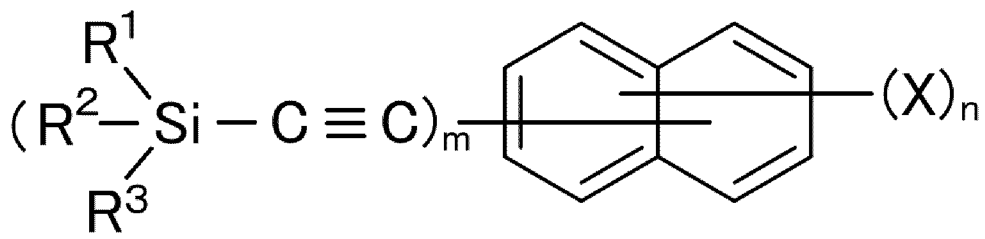

wherein $R^1$, $R^2$ and $R^3$ each independently represent a substituted or unsubstituted alkyl group,
X represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom,
m represents an integer of any of 1 to 8,
n represents an integer of any of 0 to 7,
m+n is an integer of any of 1 to 8.

8. The photon upconversion composition according to claim 7, wherein n in the general formula (1) is 1 or more.

9. The photon upconversion composition according to claim 1, wherein the naphthalene compound has a structure represented by the following general formula (2):

General Formula (2)

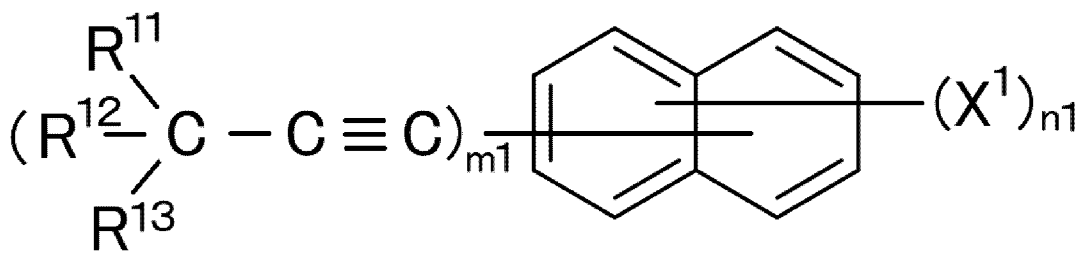

wherein $R^{11}$, $R^{12}$ and $R^{13}$ each independently represent a hydrogen atom or a substituted or unsubstituted alkyl group, at least one of $R^{11}$, $R^{12}$ and $R^{13}$ is a substituted or unsubstituted alkyl group,
$X^1$ represents a group of a combination of one or more selected from the group consisting of an alkynyl group, an alkenyl group, an alkyl group, an aromatic ring group, a heteroaromatic ring group, an alkoxy group, a carboxy

group, a cyano group and a halogen atom,
m1 represents an integer of any of 1 to 8,
n1 represents an integer of any of 0 to 7,
m1+n1 is an integer of any of 1 to 8.

**10.** The photon upconversion composition according to claim 9, wherein n1 in the general formula (2) is 1 or more.

**11.** A film containing the photon upconversion composition of any one of claims 1 to 10.

**12.** A myopia-suppressing transparent product containing the photon upconversion composition of any one of claims 1 to 10 or the film of claim 11.

**13.** A method for converting a visible light into a UV light by irradiating the photon upconversion composition of any one of claims 1 to 10 or the film of claim 11 with a visible light.

**14.** The method according to claim 13, wherein the UV light has a wavelength in a range of 360 to 400 nm.

**15.** A compound represented by the following general formula (1'):

General Formula (1')

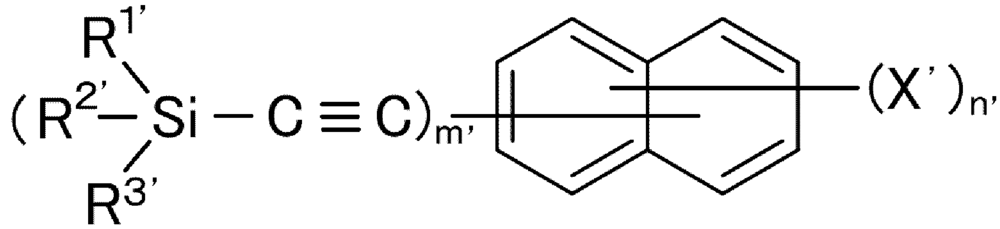

wherein $R^{1'}$, $R^{2'}$ and $R^{3'}$ each independently represent a substituted or unsubstituted alkyl group,
the total carbon number of $R^{1'}$, $R^{2'}$ and $R^{3'}$ is 6 or more,
X' represents a group of a combination of one or more selected from the group consisting of an alkynyl group, an alkenyl group, an alkyl group, an aromatic ring group, a heteroaromatic ring group, an alkoxy group, a carboxy group, a cyano group and a halogen atom,
m' represents an integer of any of 1 to 8,
n' represents an integer of any of 0 to 7,
m'+n' is an integer of any of 1 to 8.

FIG. 1

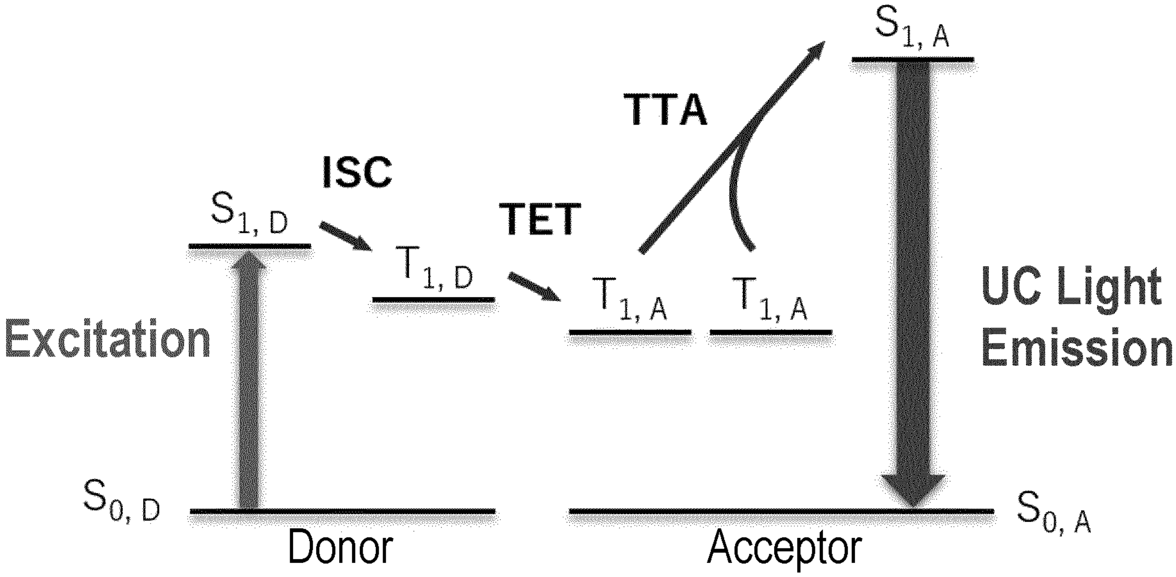
S1, A
TTA
ISC
S1, D
TET
T1, D
T1, A
T1, A
UC Light
Emission
Excitation
S0, D
Donor
Acceptor
S0, A

FIG. 2

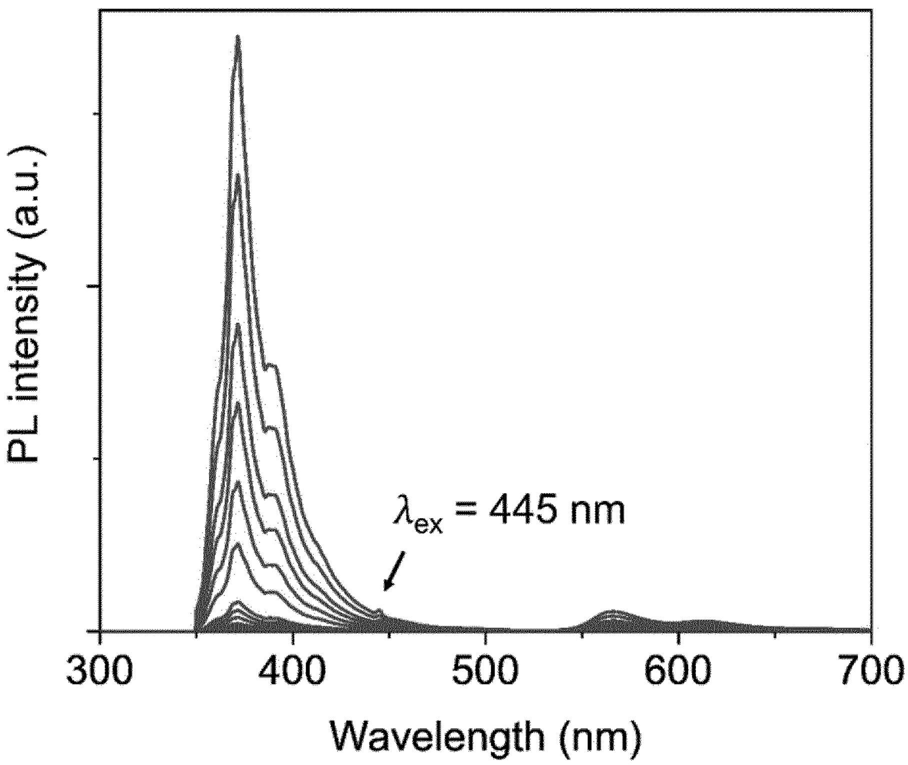
PL intensity (a.u.)
λex = 445 nm
300
400
500
600
700
Wavelength (nm)

FIG. 3

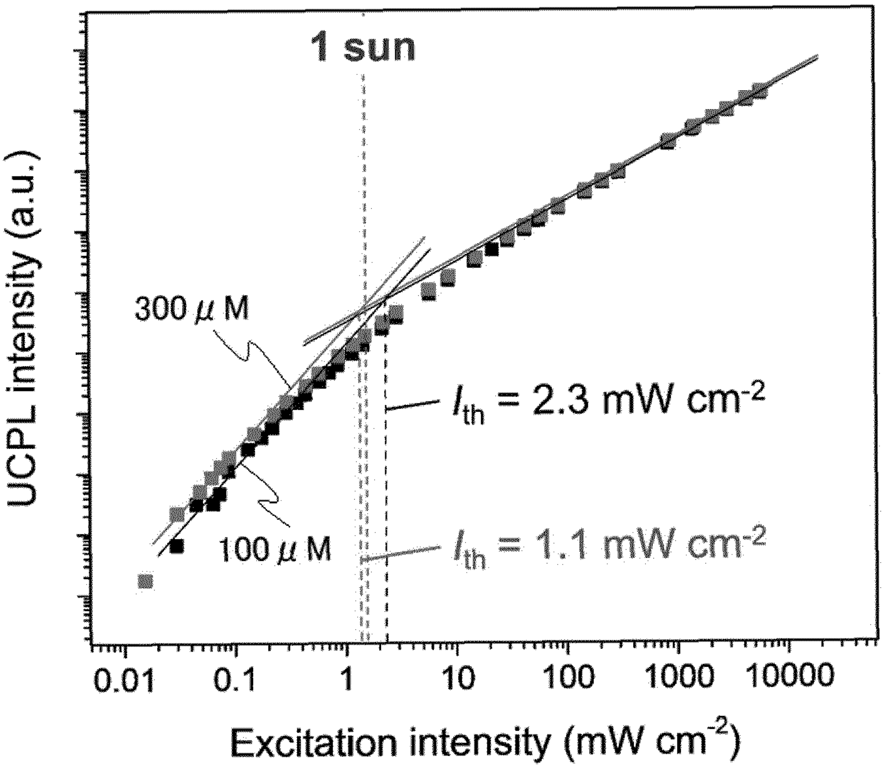
1 sun
300 μM
100 μM
$I_{th}$ = 2.3 mW cm-2
$I_{th}$ = 1.1 mW cm-2
UCPL intensity (a.u.)
0.01
0.1
1
10
100
1000
10000
Excitation intensity (mW cm-2)

FIG. 4

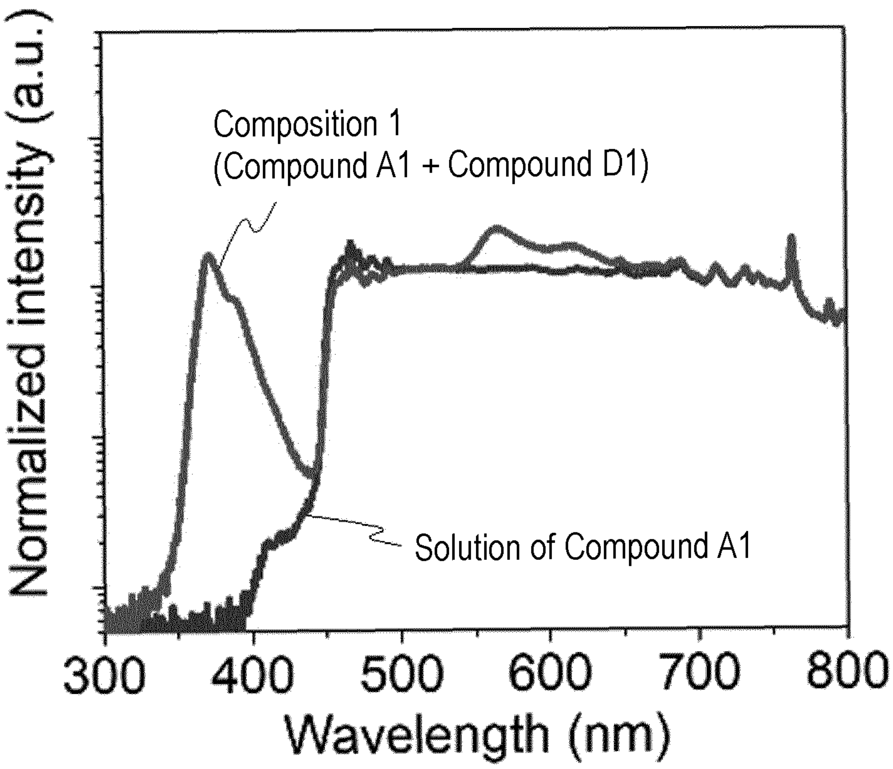
Composition 1
(Compound A1 + Compound D1)
Solution of Compound A1
Normalized intensity (a.u.)
300
400
500
600
700
800
Wavelength (nm)

FIG. 5

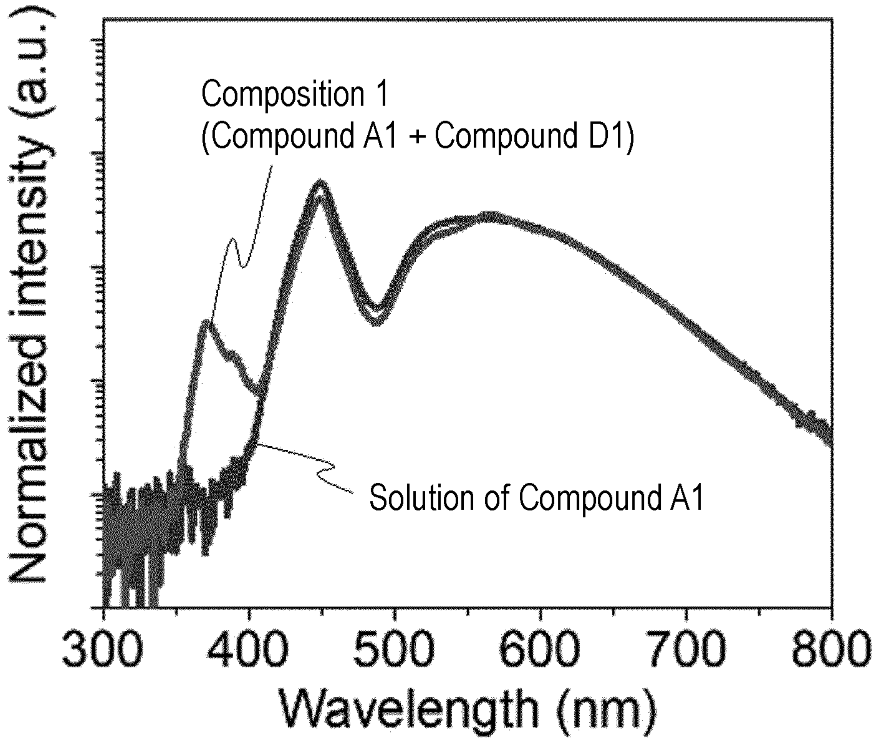
Composition 1
(Compound A1 + Compound D1)
Solution of Compound A1
Normalized intensity (a.u.)
300
400
500
600
700
800
Wavelength (nm)

FIG. 6

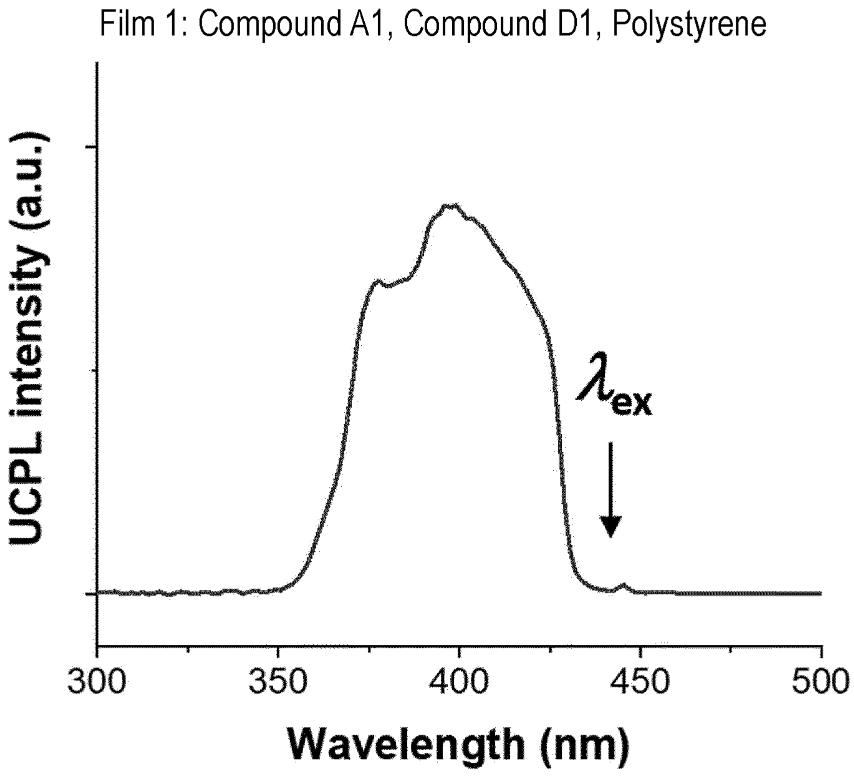
Film 1: Compound A1, Compound D1, Polystyrene
UCPL intensity (a.u.)
$\lambda_{ex}$
300
350
400
450
500
Wavelength (nm)

FIG. 7

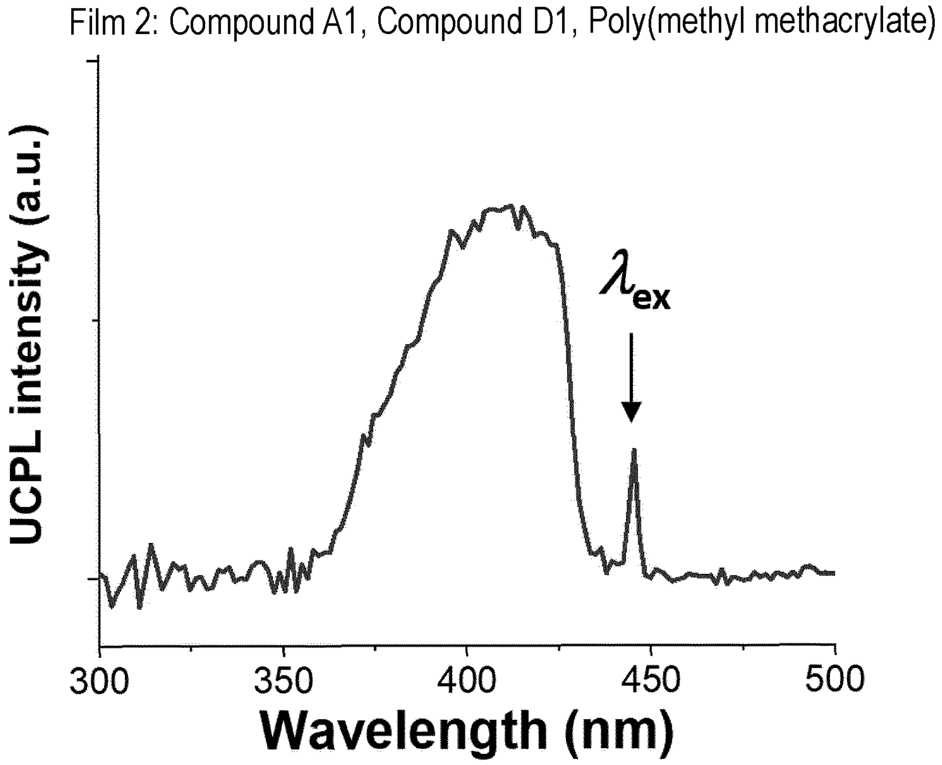
Film 2: Compound A1, Compound D1, Poly(methyl methacrylate)
UCPL intensity (a.u.)
$\lambda_{ex}$
300
350
400
450
500
Wavelength (nm)

FIG. 8

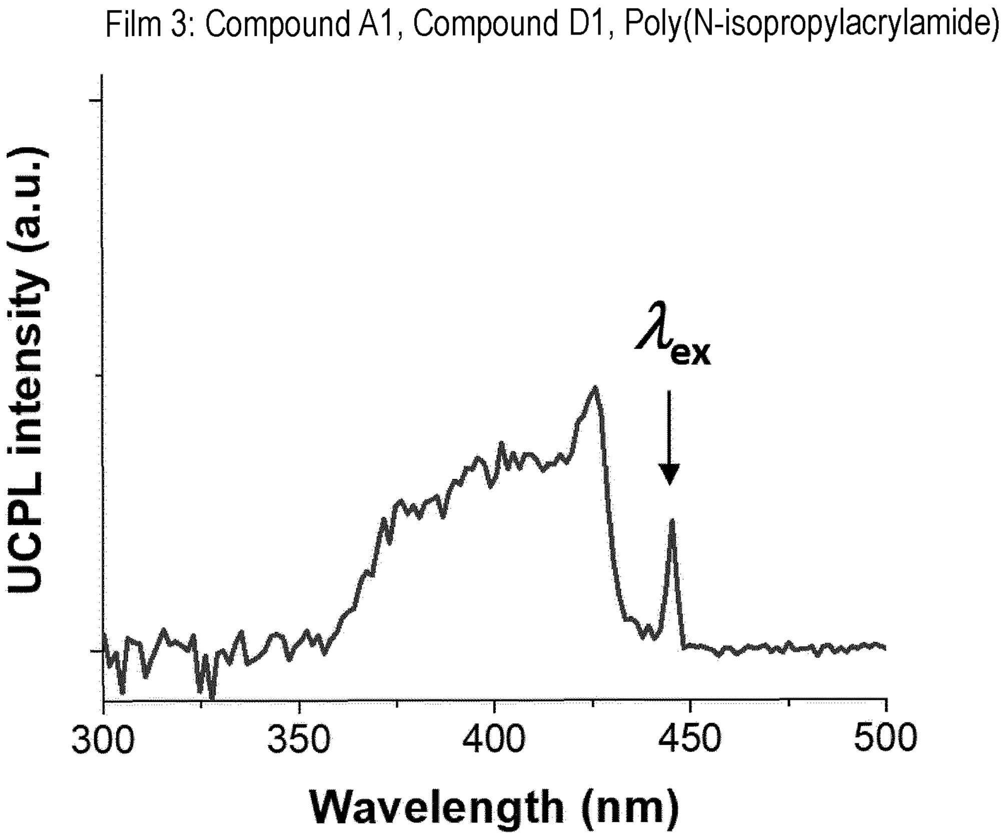
Film 3: Compound A1, Compound D1, Poly(N-isopropylacrylamide)
UCPL intensity (a.u.)
$\lambda_{ex}$
300
350
400
450
500
Wavelength (nm)

FIG. 9

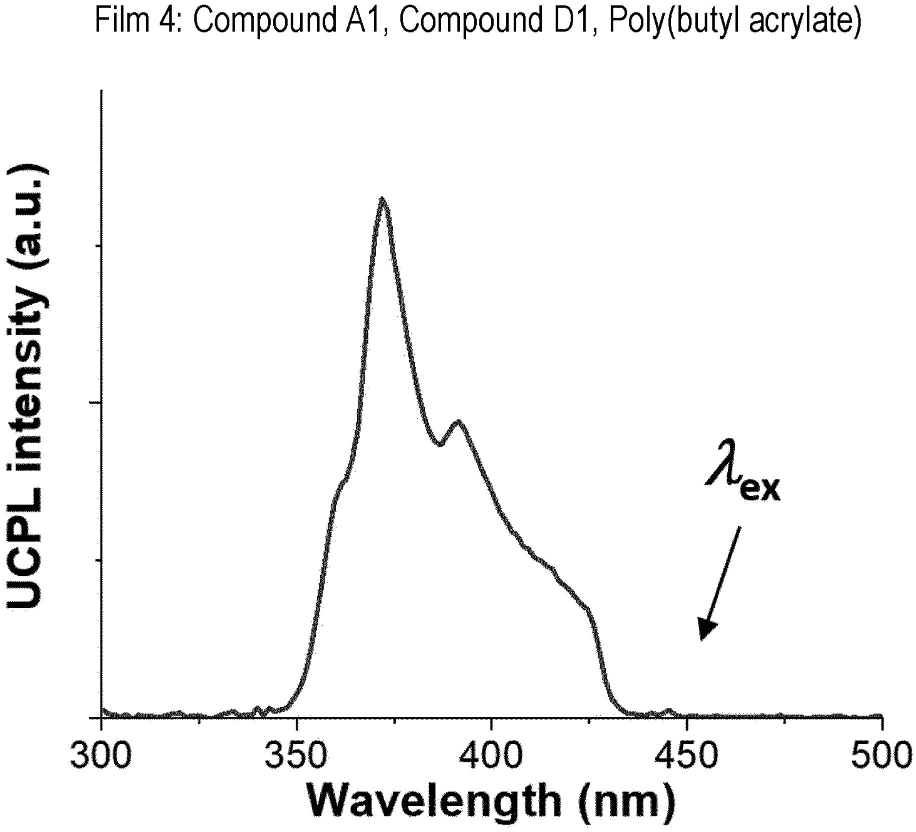
Film 4: Compound A1, Compound D1, Poly(butyl acrylate)
UCPL intensity (a.u.)
λex
300
350
400
450
500
Wavelength (nm)

FIG. 10

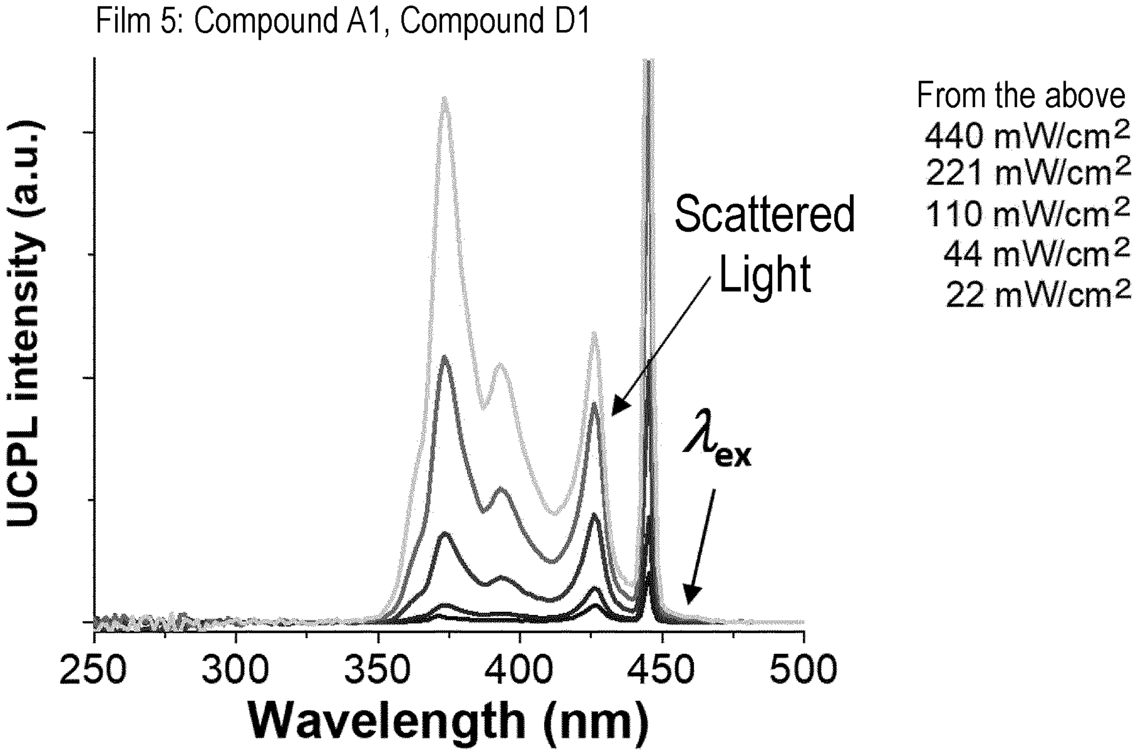
Film 5: Compound A1, Compound D1
UCPL intensity (a.u.)
From the above
440 mW/cm2
221 mW/cm2
110 mW/cm2
44 mW/cm2
22 mW/cm2
Scattered Light
λex
250
300
350
400
450
500
Wavelength (nm)

FIG. 11

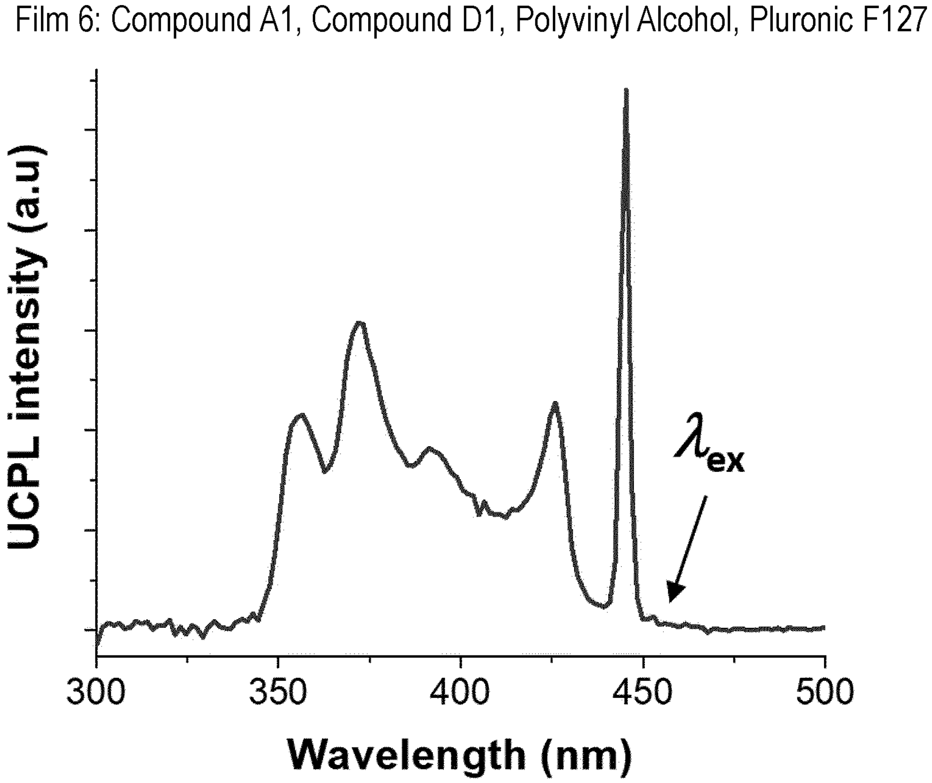
Film 6: Compound A1, Compound D1, Polyvinyl Alcohol, Pluronic F127
UCPL intensity (a.u)
λex
300
350
400
450
500
Wavelength (nm)

FIG. 12

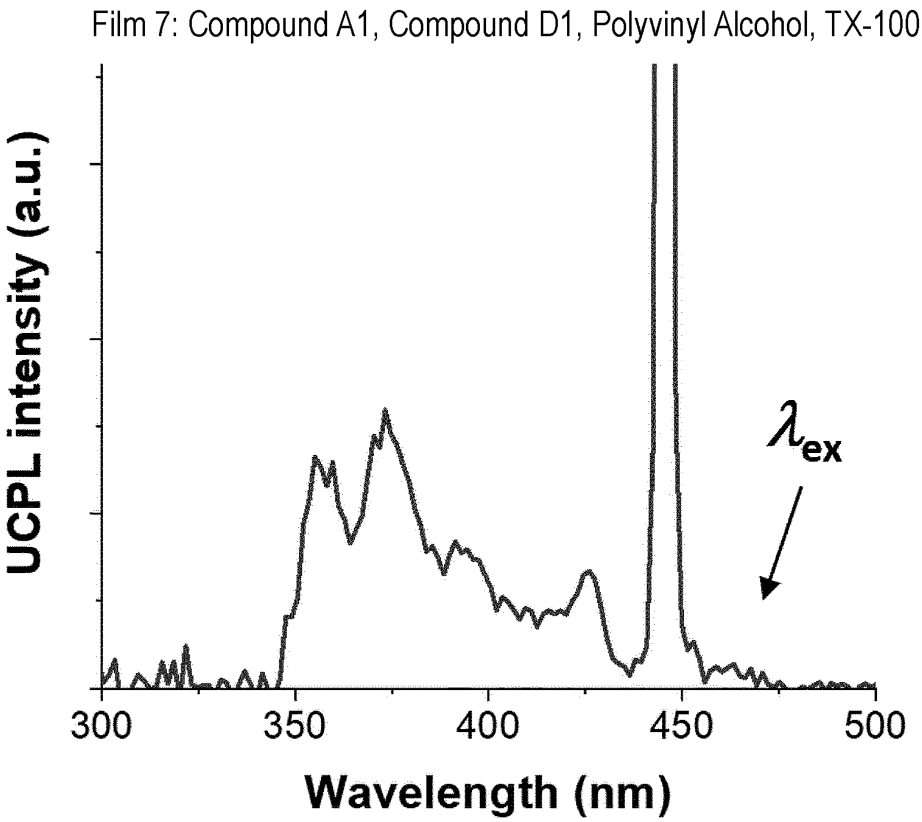
Film 7: Compound A1, Compound D1, Polyvinyl Alcohol, TX-100
UCPL intensity (a.u.)
λex
300
350
400
450
500
Wavelength (nm)

FIG. 13

Film 8: Film impregnated with Solution of Compound A1 and Compound D1 (sealed with EVOH film)

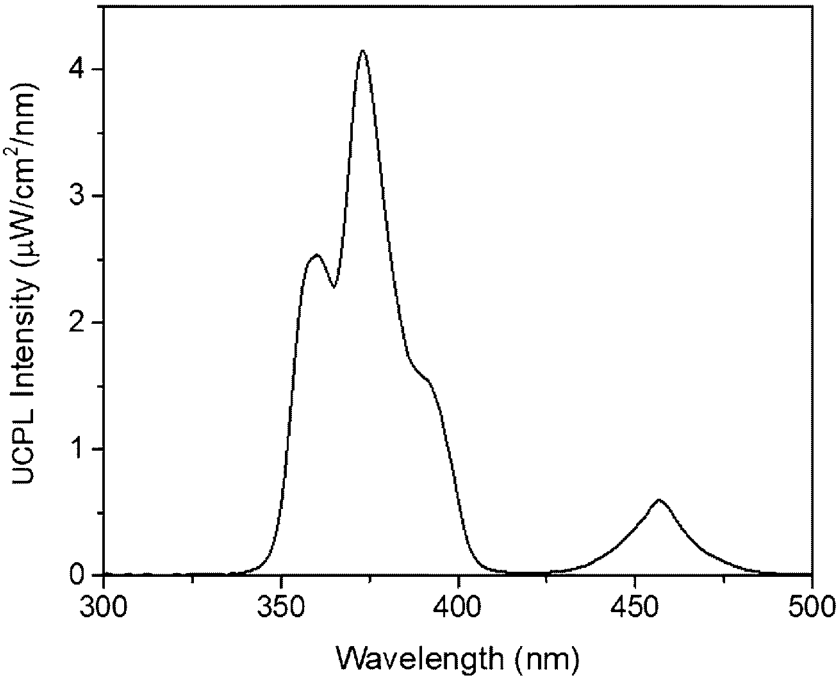


FIG. 14

Film 9: Film impregnated with melt liquid of Compound A1 and Compound D1

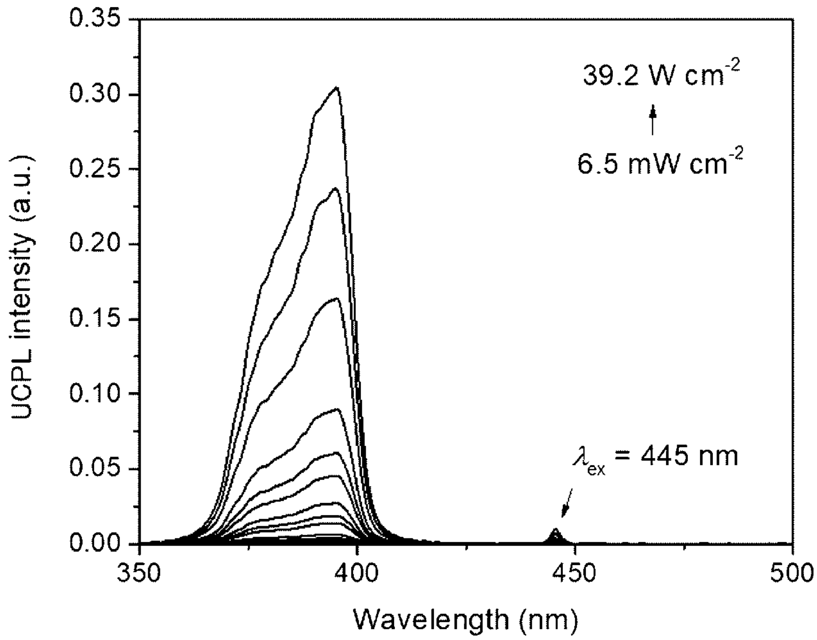

FIG. 15

Composition 4: Tetrahydrofuran solution of Compound A3 and Compound D1

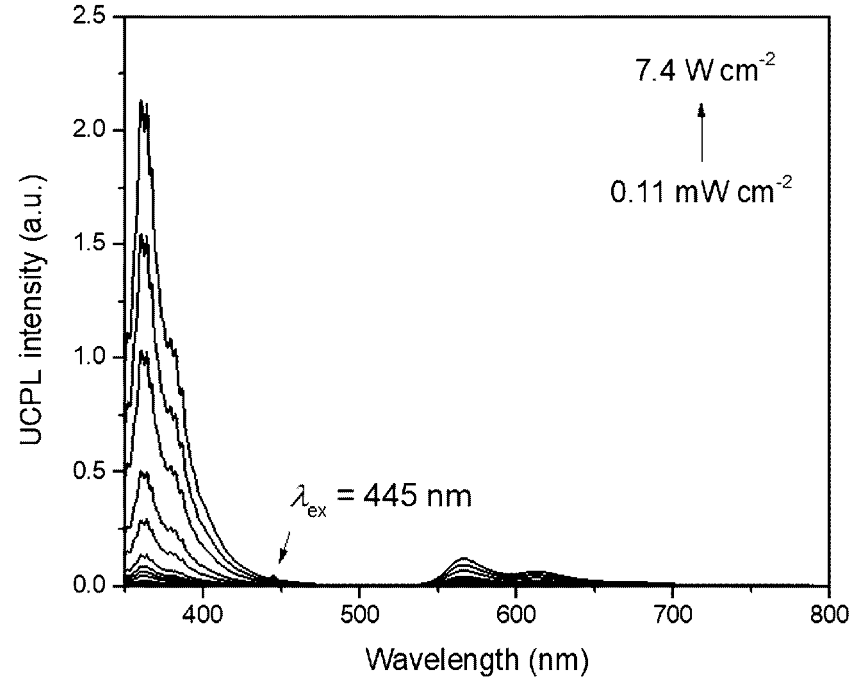


FIG. 16

Composition 4: Tetrahydrofuran solution of Compound A3 and Compound D1

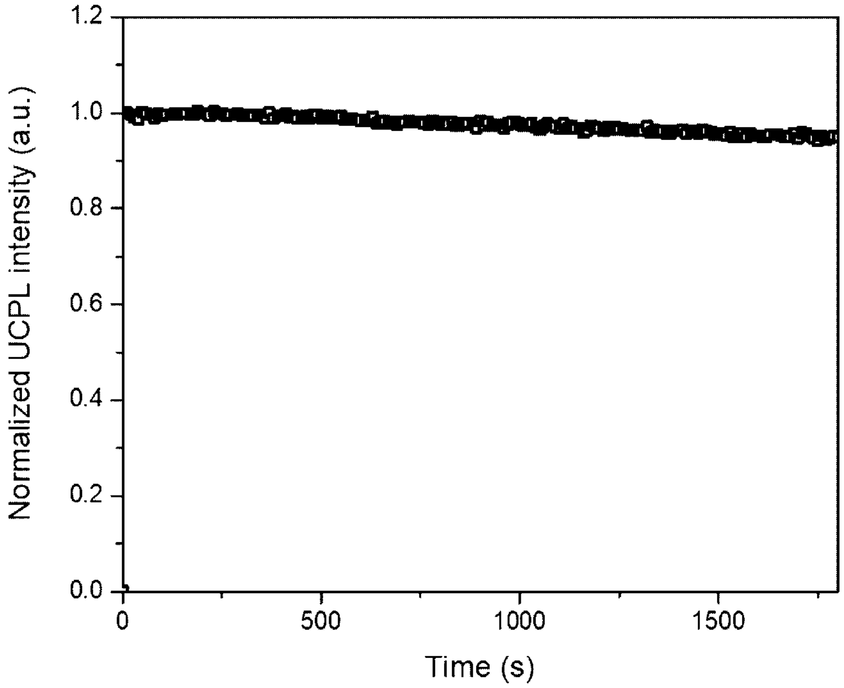

# FIG. 17

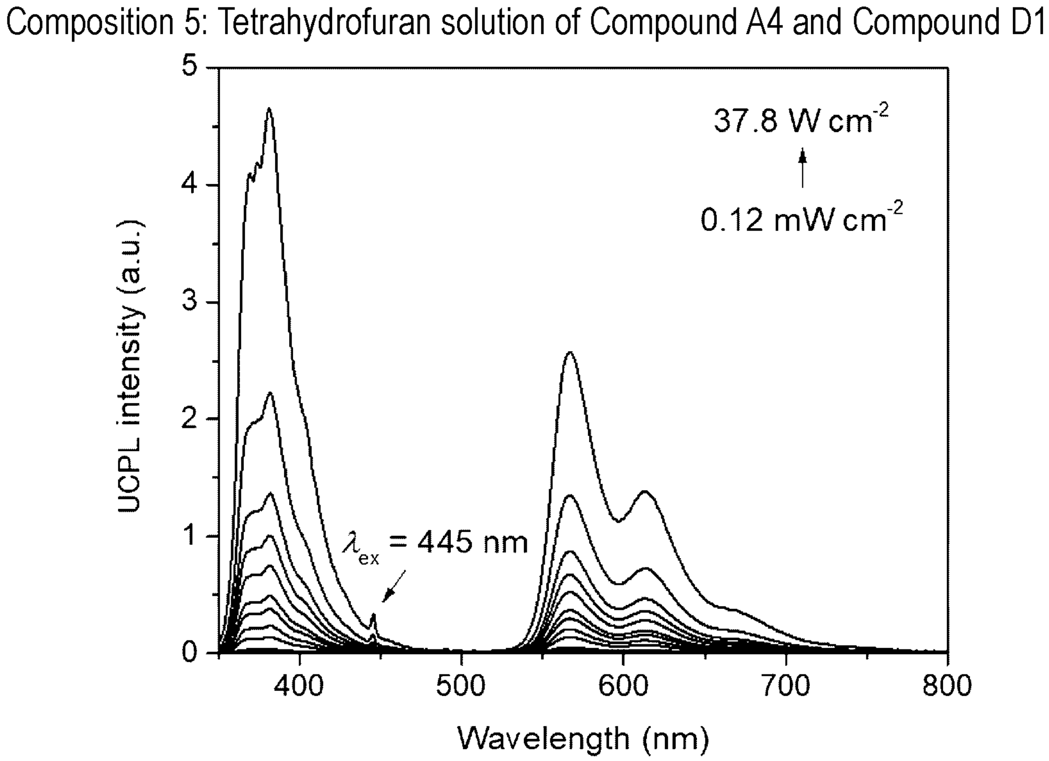
Composition 5: Tetrahydrofuran solution of Compound A4 and Compound D1
37.8 W cm-2
0.12 mW cm-2
λex = 445 nm
UCPL intensity (a.u.)
Wavelength (nm)
0
1
2
3
4
5
400
500
600
700
800

# FIG. 18

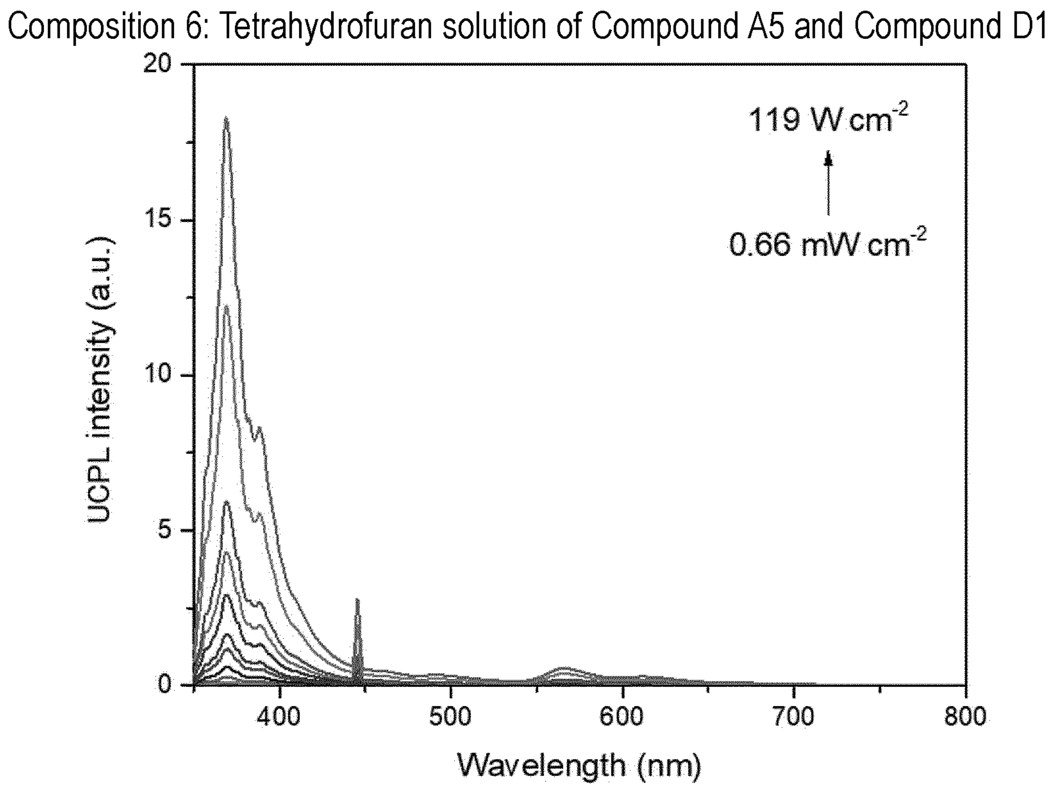
Composition 6: Tetrahydrofuran solution of Compound A5 and Compound D1
119 W cm-2
0.66 mW cm-2
UCPL intensity (a.u.)
Wavelength (nm)
0
5
10
15
20
400
500
600
700
800

FIG. 19

Composition 7: Tetrahydrofuran solution of Compound A6 and Compound D1

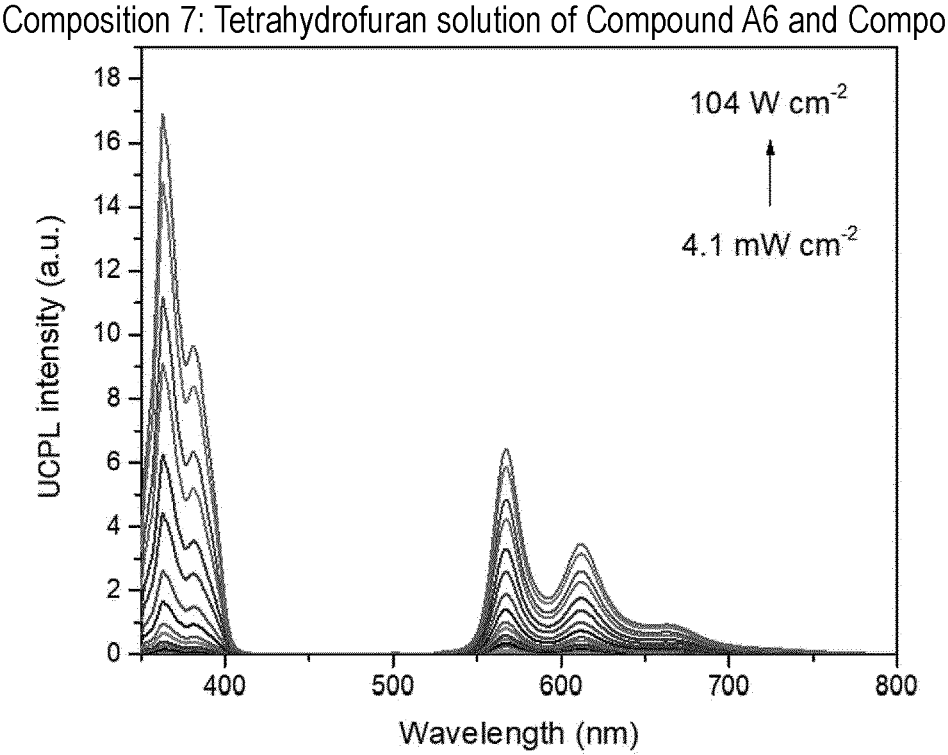

## INTERNATIONAL SEARCH REPORT

International application No.
PCT/JP2021/030649

**A. CLASSIFICATION OF SUBJECT MATTER**

***C09K 11/06***(2006.01)i; ***C07F 7/02***(2006.01)i; ***C07F 7/08***(2006.01)i; ***G02B 5/20***(2006.01)i
FI: C09K11/06; C07F7/02 F; C07F7/08 C; G02B5/20

According to International Patent Classification (IPC) or to both national classification and IPC

**B. FIELDS SEARCHED**

Minimum documentation searched (classification system followed by classification symbols)

C09K11/06; C07F7/02; C07F7/08; G02B5/20

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | BJORN PFUND, Debora et al. J. Am. Chem. Soc., 15 May 2020, 142, 10468-10476, DOI: 10.1021/jacs.0c02835<br>entire text, all drawings | 1, 3, 5-15 |
| A | OKUMURA, Keisuke et al. Chem. Lett., 05 July 2019, 48, 1347-1350, DOI: 10.1246/cl.190473<br>entire text, all drawings | 1, 3, 5-15 |
| A | MOORE, Evan G. et al. J. Phys. Chem. A., 2005, 109, 3788-3796, DOI: 10.1021/jp04422lt<br>entire text, all drawings | 1, 3, 5-15 |
| A | WO 2016/204301 A1 (UNIV KYUSHU NAT UNIV CORP) 22 December 2016 (2016-12-22)<br>compounds Re-3, Pt-25, Pt-27 | 1, 3, 5-15 |
| A | CN 101037392 A (SOOCHOW UNIVERSITY) 19 September 2007 (2007-09-19)<br>entire text, all drawings | 1, 3, 5-15 |
| A | GRAY, Victor et al. Chem. Sci., 2017, 8, 5488-5496, DOI: 10.1039/c7s01610g<br>entire text, all drawings | 1, 3, 5-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 October 2021** | **09 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/030649**

**C. DOCUMENTS CONSIDERED TO BE RELEVANT**

| Category* | Citation of document, with indication, where appropriate, of the relevant passages | Relevant to claim No. |
|---|---|---|
| A | HE, Shan et al. J. Phys. Chem. Lett., 2019, 10, 5036-5040, DOI: 10.1021/acs.jpclett.9b02106<br>entire text, all drawings | 1, 3, 5-15 |
| A | HAN, Yaoyao et al. J. Phys. Chem. Lett., 2019, 10, 1457-1463, DOI: 10.1021/acs.jpclett.9b00597<br>entire text, all drawings | 1, 3, 5-15 |
| P, A | HARADA, Naoyuki. TIPS-Naphthalene for Efficient Visible-to-UV Photon Upconversion under Sunlight and Room Light. ChemRxiv., 31 August 2020, 2020, 1-6<br>entire text, all drawings | 1, 3, 5-15 |
| P, A | HARADA, Naoyuki. Angew. Chem. Int. Ed., 2021, 60, 142-147, DOI: 10.10025/anie.202012419<br>entire text, all drawings | 1, 3, 5-15 |

**INTERNATIONAL SEARCH REPORT**

International application No.
**PCT/JP2021/030649**

**Box No. III Observations where unity of invention is lacking (Continuation of item 3 of first sheet)**

This International Searching Authority found multiple inventions in this international application, as follows:

(Invention 1) Claims 1, 3, and 5-15 (parts relating to a naphthalene compound substituted with a substituent containing an alkynyl group)
(Invention 2) Claims 1-2, 5-6, and 11-14 (parts relating to a naphthalene compound substituted with a substituent containing a substituted silyl group)
(Invention 3) Claims 1, 4-6, and 11-14 (parts relating to a naphthalene compound substituted with a substituent containing a benzene ring)
(Invention 4) Claims 1, 5-6, and 11-14 (parts relating to a naphthalene compound substituted with a substituent containing a heteroaromatic ring)
(Invention 5) Claims 1, 5-6, and 11-14 (parts relating to a naphthalene compound substituted with a substituent containing a cyano group)
(Invention 6) Claims 1, 5-6, and 11-14 (parts relating to a naphthalene compound substituted with a substituent containing a halogen atom)

(Invention 1) Claims 1, 3, and 5-15 (parts relating to a naphthalene compound substituted with a subsituent containing an alkynyl group) have the special technical feature of a photon up-conversion composition including a naphthalene compound substituted with a subsituent containing an alkynyl group, and are thus classified as invention 1.

(Inventions 2-6) Claims 1-2, 4-6, and 11-14 (parts relating to a naphthalene compound substituted with a substituted silyl group, a benzene ring, a heteroaromatic ring, a cyano group, or a halogen atom) share the common technical feature of a “photon up-conversion composition including a naphthalene compound substituted with a subsituent” with invention 1. However, said technical feature does not make a contribution over the prior art in light of the disclosure of documents 1-5 cited in the international search report, and thus cannot be considered a special technical feature. Furthermore, there are no other identical or corresponding special technical features between these inventions.

Therefore, inventions in said claims are classified as inventions 2-6.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **(Invention 1) Claims 1, 3, and 5-15 (parts relating to a naphthalene compound substituted with a substituent containing an alkynyl group)**

**Remark on Protest**

☐ The additional search fees were accompanied by the applicant’s protest and, where applicable, the payment of a protest fee.

☐ The additional search fees were accompanied by the applicant’s protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2021/030649**

| Patent document cited in search report | Publication date (day/month/year) | Patent family member(s) | Publication date (day/month/year) |
|---|---|---|---|
| WO 2016/204301 A1 | 22 December 2016 | US 2016/0367576 A1<br>compounds Re-3, Pt-25, Pt-27 | |
| CN 101037392 A | 19 September 2007 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Chem. Sci.,* 2017, vol. 8, 5488-5496
- *J. Phys. Chem. Lett.,* 2019, vol. 10, 5036-5040
- *ChemistryOpen,* 2020, vol. 9, 14-17